# EUROPEAN PATENT APPLICATION

(11) **EP 4 703 358 A1**
(43) Date of publication of application: **04.03.2026**
(21) Application number: 24796287.1
(22) Date of filing: 26.04.2024
(51) Int. Cl.: C07D 405/14, C07D 401/14, A61K 31/454, A61P 35/00

(54) **BENZO SIX-MEMBERED HETEROCYCLIC GSPT1 PROTEIN DEGRADATION AGENT AND USE THEREOF**

(30) Priority: 28.04.2023 CN 202310478561
(71) Applicant: China Pharmaceutical University, Nanjing, Jiangsu 211198 (CN); Ascentage Pharma (Suzhou) Co., Ltd., Suzhou, Jiangsu 215000 (CN)
(72) Inventor: LI, Zhiyu, Nanjing, Jiangsu 211198 (CN); XU, Xi, Nanjing, Jiangsu 211198 (CN); CHANG, Xiujin, Nanjing, Jiangsu 211198 (CN); BIAN, Jinlei, Nanjing, Jiangsu 211198 (CN); QU, Fangui, Nanjing, Jiangsu 211198 (CN); WANG, Jubo, Nanjing, Jiangsu 211198 (CN); FAN, Zhongpeng, Nanjing, Jiangsu 211198 (CN); QIU, Zhixia, Nanjing, Jiangsu 211198 (CN); WU, Hongxi, Nanjing, Jiangsu 211198 (CN); ZHANG, Di, Nanjing, Jiangsu 211198 (CN); ZHANG, Yanqing, Nanjing, Jiangsu 211198 (CN)
(74) Representative: AWA Denmark A/S
(86) International application number: PCT/CN2024/090223
(87) International publication number: WO 2024/222918

(57) **Abstract**

Disclosed in the present invention are a benzo six-membered heterocyclic compound or a pharmaceutically acceptable salt, tautomer, mesomer, racemate, enantiomer, and diastereomer thereof. The degradation effect of the compound of the present invention on GSPT1 proteins and the anti-proliferation inhibitory activity of the compound of the present invention for AML cells are remarkably improved. Therefore, the compound of the present invention can be used for preparing a drug for treating or preventing diseases related to mutation, expression imbalance, and allosteric and dysfunction of GSPT1, IKZF1, IKZF2, IKZF3, CK1α, N-MYC or C-MYC proteins, and can be used for preparing a GSPT1 degradation agent.

## Description

### TECHNICAL FIELD

The present disclosure pertains to the technical field of pharmaceuticals and particularly relates to a benzo six-membered heterocyclic GSPT1 protein degrader, a preparation method therefor, and use thereof.

### BACKGROUND

Target protein degradation (TPD) technology is an emerging technology that utilizes a protein homeostasis-regulating protein degradation mechanism inherent in eukaryotic cells to interfere with protein functions. The rise of this technology solves, to a certain extent, the challenges confronting small-molecule inhibitors and gene interference technology. At present, TPD degrades target proteins mainly through ubiquitin proteasomes and lysosomes. Currently, the most developed and studied approaches in this area are proteolysis-targeting chimeras (PROTACs) and molecular glues (MGs), which are based on the ubiquitin-proteasome system. Target protein degradation technology has considerable promise for application and potential for development in a number of disease areas, such as malignancies, neurodegenerative diseases, and metabolic diseases.

Molecular glues are a class of proximity-inducing small molecules that enable precise temporal control over various biological processes, such as signal transduction, transcription, chromatin regulation, and protein folding, localization, and degradation. As chemical inducers of proximity, they can induce protein-protein interactions between E3 ubiquitin ligases and target proteins, leading to the degradation of the latter. Through the formation of ternary complexes, molecular glues can promote the dimerization or co-localization of two proteins, thereby producing a variety of biological and pharmacological functions.

G1 to S phase transition 1 (GSPT1, also known as eRF3a) is a translation termination factor in the body. Downregulation of GSPT1 can lead to abnormal expression of key proteins and inhibit proliferation or induce apoptosis in various tumor cells. Since the discovery that GSPT1 is a new substrate of the E3 ubiquitin ligase CRBN, it has been able to be degraded by molecular glue degraders in a targeted manner. Currently, a variety of molecular glue degraders are undergoing clinical and preclinical research. These molecules are very effective in treating hematological tumors and certain solid tumors. GSPT1 is a multifunctional protein. In addition to its role as a class II peptide chain release factor involved in protein translation termination, it is also related to processes such as apoptosis, cell cycle regulation, and tumor development and progression. For example, during apoptosis, GSPT1 is processed proteolytically to form an isoform that contains a conserved N-terminal apoptosis-inhibiting protein conjugate. GSPT1 interacts with inhibitors of apoptosis proteins (IAPs) through this form. This interaction relieves the inhibition of IAPs, leading to the release of caspases and thus the promotion of apoptosis. Moreover, the M domain of eRF3 interacts with polya-binding protein (PABP), coupling translation termination with mRNA degradation.

Acute myeloid leukemia (AML) is a genetically and biologically heterogeneous myeloid malignancy characterized by the abnormal proliferation of immature myeloid progenitor cells, which disrupts normal hematopoiesis, leading to severe infections, anemia, and bleeding. The survival rate of AML is low, and its recurrence rate is high. Therefore, as a relatively rare malignancy, AML has been considered an orphan disease by the U.S. Food and Drug Administration. GSPT1, as a new substrate of CRL4-CRBN-E3 ubiquitin ligase, plays an important role in treating AML. GSPT1-targeting clinical drugs for treating AML, such as CC-90009 and BTX-1188, are currently under development. CC-90009 is administered mainly by intravenous injection to treat AML, and BTX-1188 is a drug that can be orally administered to treat AML and NHL. Further studies show that the degradation of GSPT1 is associated with upregulated expression of the ATF3 and ATF4 genes, which are critical to the integrated stress response pathway. The activation of the integrated stress response pathway is closely linked to the phosphorylation of eRF2. When activated, the integrated stress response pathway can lead to acute apoptosis.

According to the World Health Organization, breast cancer is the leading cause of death from malignancies among women. In 2018, Wang et al. analyzed gene expression in the total RNA extracted from breast cancer samples they had collected, and identified five genes, including GSPT1, as potential therapeutic targets for triple-negative breast cancer. Recently, a study by Malta-Vacas et al. revealed that the longer GSPT1 allele 12-GGC was present in 5.1% of breast cancer patients, and mRNA quantification experiments further indicated that GSPT1 was overexpressed in tumor tissues from patients with the allele 12-GGC compared to adjacent normal tissues. In addition, Miri et al. conducted a study on the link between the GSPT1 gene in breast cancer patients and the susceptibility to breast cancer. The study showed that there were significantly elevated GSPT1 expression levels in breast cancer tissues, and the presence of the longer allele 12-GGC in the GSPT1 exon can increase the risk of developing breast cancer threefold. Therefore, some scholars believe that the link between the allele 12-GGC and cancer progression is achieved by GSPT1 regulating mRNA degradation, translation efficiency, or the like to make proteins undergo functional loss.

In developing countries, gastric cancer is a leading cause of cancer-related deaths. Malta-Vacas et al. found that the expression level of GSPT1 in intestinal-type gastric tumors was significantly higher than that in diffuse gastric tumors. In addition, they evaluated the link between GSPT1 and the potential genetic susceptibility to gastric cancer, and found that patients with the allele 12-GGC had a 20-fold increased risk of developing gastric cancer compared to normal people, regardless of genotype. Tian et al. explored the potential influence of GSPT1 on the development of gastric cancer and found that the expression level of GSPT1 was significantly elevated in gastric cancer tissues. Some scholars believe that the overexpression of GSPT1 promotes tumor progression by enhancing the translation efficiency of particular oncogene mRNAs, while some attribute it to other roles of GSPT1, such as regulating the cell cycle, apoptosis, etc. Since GSPT1 is also involved in cytoskeleton formation and controls chromosomal segregation, the influence of GSPT1 on chromosomal segregation and cytokinesis may be a potential mechanism through which this gene influences the development of gastric cancer.

Liver cancer is a common neoplastic disease. Clinical data indicate that the expression level of GSPT1 is generally higher in liver cancer tissues than in normal cells. Since one of the main functions of GSPT1 is to regulate the transition of cells from the G1 phase to the S phase, some researchers speculate that interfering with GSPT1 mRNA expression may alter the cell cycle, leading to an increase in the percentage of cells in the G1 phase. This, in turn, reduces the viability and proliferative capacity of tumor cells. Although GSPT1 has a cancer-promoting effect in the development and progression of liver cancer, researchers found, by constructing HepG2 cells with overexpression and knockdown of the GSTP1 gene, that GSPT1 may inhibit canceration in the progression of liver cancer.

Colorectal cancer is one of the most common malignancies in the digestive system. A study by Xiao et al. demonstrated that GSPT18 was overexpressed in human colorectal cancer HCT116 cells compared to colorectal cells in the normal control group, and further experiments showed that knocking down GSPT1 in colorectal cancer cells with high GSPT1 expression could inhibit the proliferation and migration of colorectal cancer cells. Research findings show that GSPT1 inhibits cell cycle progression in HCT116 cells via the mTOR pathway. When the mTOR pathway is activated, the translation of a subset of mRNAs is increased. Therefore, GSPT1 may play the role of a proto-oncogene in the development and progression of colorectal cancer.

In summary, GSPT1 plays an important role in the development and progression of related solid tumors. However, due to its functional diversity and complex regulatory mechanisms, it plays different roles in different tumors. Currently, it is widely recognized that GSPT1 is a proto-oncogene of most tumors and, in certain particular types of tumors, plays the role of a tumor suppressor gene. Although there remains a lack of relatively in-depth research on the related mechanisms of action of GSPT1 in tumors, there is no doubt that GSPT1 has become a powerful potential target in tumor treatment.

### SUMMARY

Objectives: One of the objectives of the present disclosure is to provide a benzo six-membered heterocyclic compound of general formula I or a pharmaceutically acceptable salt, tautomer, mesomer, racemate, enantiomer, or diastereomer thereof: 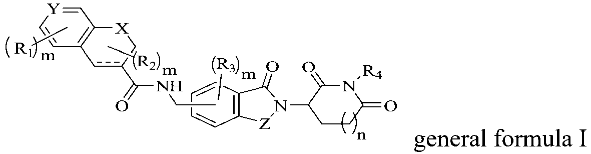 wherein:
- - - - represents a single or double bond;
X is selected from -CH₂-, -NH-, -O-, -S-, and -Se-;
Y is selected from carbon and nitrogen;
Z is selected from -CH₂-, -CD₂-, -C(O)-, and -C(S)-;
each R₁ is independently selected from hydrogen, C₁-C₆ alkyl, halogenated C₁-C₆ alkyl, C₂-C₆ alkenyl, C₂-C₆ alkynyl, halogen, cyano, nitro, -C(O)NRₐR_{b}, -C(O)Rₐ, -C(O)ORₐ, -ORₐ, -OC(O)Rₐ, -OC(O)ORₐ, -OC(O)NRₐR_{b}, -NRₐR_{b}, -SRₐ, -S(O)Rₐ, -S(O)₂Rₐ, and 3- to 10-membered cycloalkyl, heterocyclyl, aryl, and heteroaryl that contain 0-3 heteroatoms, wherein the alkyl, alkenyl, alkynyl, cycloalkyl, heterocyclyl, aryl, and heteroaryl are unsubstituted or optionally substituted with 1-3 Rₐ groups;
each R₂ is independently selected from hydrogen, C₁-C₆ alkyl, halogenated C₁-C₆ alkyl, C₂-C₆ alkenyl, C₂-C₆ alkynyl, halogen, cyano, nitro, -C(O)NRₐR_{b}, -C(O)Rₐ, -C(O)ORₐ, -ORₐ, -OC(O)Rₐ, -OC(O)ORₐ, -OC(O)NRₐR_{b}, -NRₐR_{b}, -SRₐ, -S(O)Rₐ, -S(O)₂Rₐ, and 3- to 10-membered cycloalkyl, heterocyclyl, aryl, and heteroaryl that contain 0-3 heteroatoms, wherein the alkyl, alkenyl, alkynyl, cycloalkyl, heterocyclyl, aryl, and heteroaryl are unsubstituted or optionally substituted with 1-3 Rₐ groups;
each R₃ is independently selected from hydrogen, C₁-C₆ alkyl, halogenated C₁-C₆ alkyl, C₂-C₆ alkenyl, C2-C6 alkynyl, halogen, cyano, nitro, -C(O)NRₐR_{b}, -C(O)Rₐ, -C(O)ORₐ, -ORₐ, -OC(O)Rₐ, -OC(O)ORₐ, -OC(O)NRₐR_{b}, -NRₐR_{b}, -SRₐ, -S(O)Rₐ, -S(O)₂Rₐ, and 3- to 10-membered cycloalkyl, heterocyclyl, aryl, and heteroaryl that contain 0-3 heteroatoms, wherein the alkyl, alkenyl, alkynyl, cycloalkyl, heterocyclyl, aryl, and heteroaryl are unsubstituted or optionally substituted with 1-3 Rₐ groups;
R₄ is selected from hydrogen, deuterium, C₁-C₁₂ alkyl, 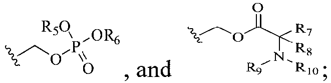
R₅, R₆, R₇, R₈, R₉, and R₁₀ are each independently selected from hydrogen, C₁-C₆ alkyl, halogenated C₁-C₆ alkyl, C₂-C₆ alkenyl, C₂-C₆ alkynyl, halogen, cyano, nitro, -C(O)NRₐR_{b}, -C(O)Rₐ, -C(O)ORₐ, - ORₐ, -OC(O)Rₐ, -OC(O)ORₐ, -OC(O)NRₐR_{b}, -NRₐR_{b}, -SRₐ, -S(O)Rₐ, -S(O)₂Rₐ, and 3- to 10-membered cycloalkyl, heterocyclyl, aryl, and heteroaryl that contain 0-3 heteroatoms, wherein the alkyl, alkenyl, alkynyl, cycloalkyl, heterocyclyl, aryl, and heteroaryl are unsubstituted or optionally substituted with 1-3 Rₐ groups;
Rₐ and R_{b} are each independently selected from hydrogen, C₁-C₆ alkyl, C₂-C₆ alkenyl, C₂-C₆ alkynyl, halogen, hydroxyl, cyano, nitro, benzyl, -C(O)NR_{c}R_{d}, -C(O)R_{c}, -C(O)OR_{c}, -OR_{c}, -OC(O)Rₑ, - OC(O)OR_{c}, -OC(O)NR_{c}R_{d}, -NR_{c}R_{d}, -SR_{c}, -S(O)R_{c}, -S(O)₂R_{c}, and 3- to 10-membered cycloalkyl, heterocyclyl, aryl, and heteroaryl that contain 0-3 heteroatoms, wherein the alkyl, alkenyl, alkynyl, cycloalkyl, heterocyclyl, aryl, heteroaryl, or benzyl is unsubstituted or optionally substituted with 1-3 R_{c} groups;
R_{c} and R_{d} are each independently selected from hydrogen, halogen, carbonyl, hydroxyl, cyano, nitro, phenyl, benzyl, C₁-C₆ alkyl, halogenated C₁-C₆ alkyl, C₁-C₆ alkoxy, halogenated C₁-C₆ alkoxy, C₃-C₆ cycloalkyl, and halogenated C₃-C₆ cycloalkyl;
m and n are each independently selected from 0, 1, 2, and 3.

Another objective of the present disclosure is to provide a compound represented by general formula II or a pharmaceutically acceptable salt, tautomer, mesomer, racemate, enantiomer, or diastereomer thereof: 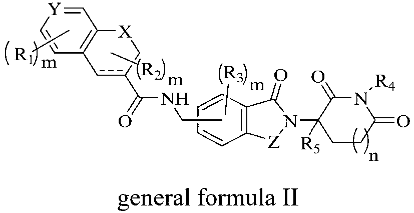 wherein:
- - - - represents a single or double bond;
X is selected from -CH₂-, -NH-, -O-, -S-, and -Se-;
Y is selected from carbon and nitrogen;
Z is selected from -CH₂-, -CD₂-, -C(O)-, and -C(S)-;
each R₁ is independently selected from hydrogen, C₁-C₆ alkyl, halogenated C₁-C₆ alkyl, C₂-C₆ alkenyl, C₂-C₆ alkynyl, halogen, cyano, nitro, -C(O)NRₐR_{b}, -C(O)Rₐ, -C(O)ORₐ, -ORₐ, -OC(O)Rₐ, -OC(O)ORₐ, -OC(O)NRₐR_{b}, -NRₐR_{b}, -SRₐ, -S(O)Rₐ, -S(O)₂Rₐ, and 3- to 10-membered cycloalkyl, heterocyclyl, aryl, and heteroaryl that contain 0-3 heteroatoms, wherein the alkyl, alkenyl, alkynyl, cycloalkyl, heterocyclyl, aryl, and heteroaryl are unsubstituted or optionally substituted with 1-3 Rₐ groups;
each R₂ is independently selected from hydrogen, C₁-C₆ alkyl, halogenated C₁-C₆ alkyl, C₂-C₆ alkenyl, C2-C6 alkynyl, halogen, cyano, nitro, -C(O)NRₐR_{b}, -C(O)Rₐ, -C(O)ORₐ, -ORₐ, -OC(O)Rₐ, -OC(O)ORₐ, -OC(O)NRₐR_{b}, -NRₐR_{b}, -SRₐ, -S(O)Rₐ, -S(O)₂Rₐ, and 3- to 10-membered cycloalkyl, heterocyclyl, aryl, and heteroaryl that contain 0-3 heteroatoms, wherein the alkyl, alkenyl, alkynyl, cycloalkyl, heterocyclyl, aryl, and heteroaryl are unsubstituted or optionally substituted with 1-3 Rₐ groups;
each R₃ is independently selected from hydrogen, C₁-C₆ alkyl, halogenated C₁-C₆ alkyl, C₂-C₆ alkenyl, C2-C6 alkynyl, halogen, cyano, nitro, -C(O)NRₐR_{b}, -C(O)Rₐ, -C(O)ORₐ, -ORₐ, -OC(O)Rₐ, -OC(O)ORₐ, -OC(O)NRₐR_{b}, -NRₐR_{b}, -SRₐ, -S(O)Rₐ, -S(O)₂R₄, and 3- to 10-membered cycloalkyl, heterocyclyl, aryl, and heteroaryl that contain 0-3 heteroatoms, wherein the alkyl, alkenyl, alkynyl, cycloalkyl, heterocyclyl, aryl, and heteroaryl are unsubstituted or optionally substituted with 1-3 Rₐ groups;
R₄ is selected from hydrogen, deuterium, C₁-C₁₂ alkyl, 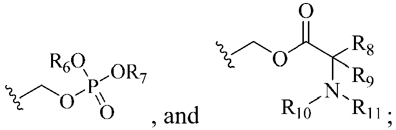
R₅ is selected from hydrogen, deuterium, and C₁-C₁₂ alkyl;
R₆, R₇, R₈, R₉, R₁₀, and R₁₁ are each independently selected from hydrogen, C₁-C₆ alkyl, halogenated C₁-C₆ alkyl, C₂-C₆ alkenyl, C₂-C₆ alkynyl, halogen, cyano, nitro, -C(O)NRₐR_{b}, -C(O)Rₐ, -C(O)ORₐ, - ORₐ, -OC(O)Rₐ, -OC(O)ORₐ, -OC(O)NRₐR_{b}, -NRₐR_{b}, -SRₐ, -S(O)Rₐ, -S(O)₂Rₐ, and 3- to 10-membered cycloalkyl, heterocyclyl, aryl, and heteroaryl that contain 0-3 heteroatoms, wherein the alkyl, alkenyl, alkynyl, cycloalkyl, heterocyclyl, aryl, and heteroaryl are unsubstituted or optionally substituted with 1-3 Rₐ groups;
Rₐ and R_{b} are each independently selected from hydrogen, C₁-C₆ alkyl, C₂-C₆ alkenyl, C₂-C₆ alkynyl, halogen, hydroxyl, cyano, nitro, benzyl, -C(O)NR_{c}R_{d}, -C(O)R_{c}, -C(O)OR_{c}, -OR_{c}, -OC(O)R_{c}, - OC(O)OR_{c}, -OC(O)NR_{c}R_{d}, -NR_{c}R_{d}, -SR_{c}, -S(O)R_{c}, -S(O)₂R_{c}, and 3- to 10-membered cycloalkyl, heterocyclyl, aryl, and heteroaryl that contain 0-3 heteroatoms, wherein the alkyl, alkenyl, alkynyl, cycloalkyl, heterocyclyl, aryl, heteroaryl, or benzyl is unsubstituted or optionally substituted with 1-3 R_{c} groups;
R_{c} and R_{d} are each independently selected from hydrogen, halogen, carbonyl, hydroxyl, cyano, nitro, phenyl, benzyl, C₁-C₆ alkyl, halogenated C₁-C₆ alkyl, C₁-C₆ alkoxy, halogenated C₁-C₆ alkoxy, C₃-C₆ cycloalkyl, and halogenated C₃-C₆ cycloalkyl;
m and n are each independently selected from 0, 1, 2, and 3.

The compound of general formula I or the pharmaceutically acceptable salt, tautomer, mesomer, racemate, enantiomer, or diastereomer thereof of the present disclosure is preferably a compound having a structure of formula Ia, formula Ib, or formula Ic: 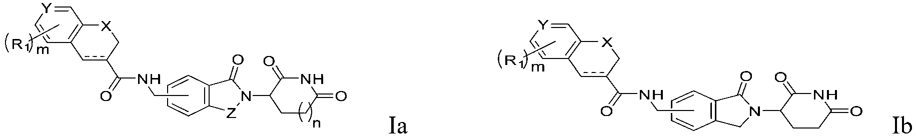 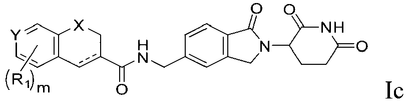 wherein R₁, X, Y, Z, m, n, and - - - - are as defined in general formula I.

In certain preferred embodiments,
X is selected from -NH- and -O-.

In certain preferred embodiments,
each R₁ is independently selected from hydrogen, C₁-C₆ alkyl, halogenated C₁-C₆ alkyl, C₁-C₆ alkoxy, halogenated C₁-C₆ alkoxy, C₂-C₆ alkenyl, C₂-C₆ alkynyl, halogen, cyano, hydroxyl, nitro, C₁-C₁₀ aryl, and -NRₐR_{b};
Rₐ and R_{b} are each independently selected from hydrogen, C₁-C₆ alkyl, and -C(O)R_{c};
R_{c} is selected from hydrogen, halogen, C₁-C₆ alkyl, halogenated C₁-C₆ alkyl, C₁-C₆ alkoxy, halogenated C₁-C₆ alkoxy, and C₃-C₆ cycloalkyl.

In certain more preferred embodiments,
each R₁ is independently selected from hydrogen, methyl, fluorine, chlorine, methoxy, trifluoromethoxy, nitro, cyano, amino, hydroxyl, ethenyl, ethynyl, isobutyl, phenyl,

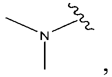

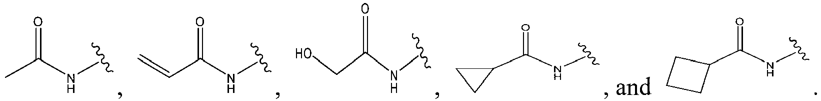

The above compound of general formula I to which the present disclosure relates may also be present in the form of salts thereof, which are converted *in vivo* into the compound of general formula I. For example, within the scope of the present disclosure, according to a process known in the art, the compounds of the present disclosure are converted into pharmaceutically acceptable salt forms and used in the salt forms.

In some preferred embodiments, the pharmaceutically acceptable salt includes, but is not limited to, acid addition salts formed by the compound of general formula I with the following acids: hydrochloric acid, hydrobromic acid, sulfuric acid, phosphoric acid, methanesulfonic acid, benzenesulfonic acid, p-toluenesulfonic acid, naphthalenesulfonic acid, citric acid, tartaric acid, lactic acid, pyruvic acid, acetic acid, maleic acid or succinic acid, fumaric acid, salicylic acid, phenylacetic acid, and mandelic acid; and acid salts formed by the compound of general formula I with inorganic bases.

In some more preferred embodiments, the pharmaceutically acceptable salt includes, but is not limited to, alkali metal cation salts, alkaline earth metal cation salts, and ammonium cation salts.

The compound of general formula I of the present disclosure is preferably the following compounds: 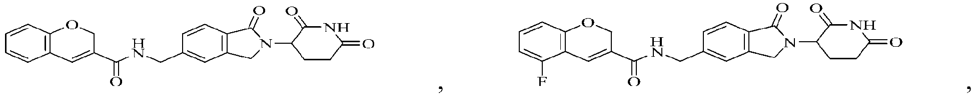 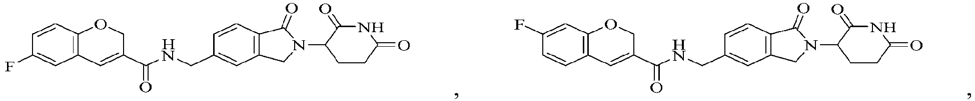 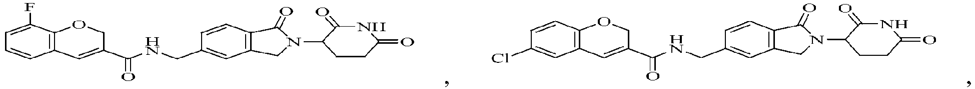 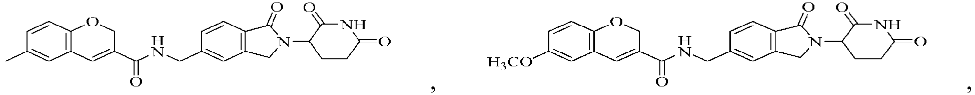 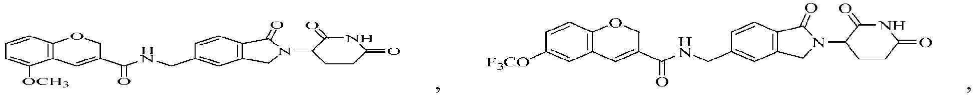 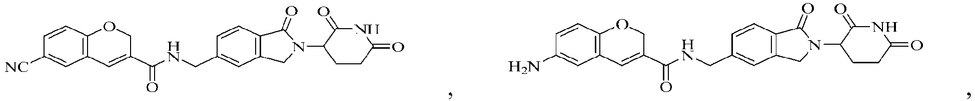 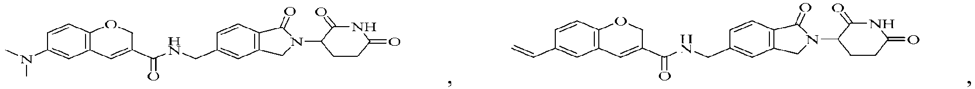 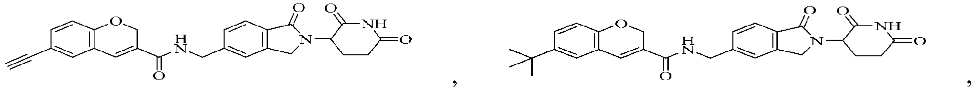 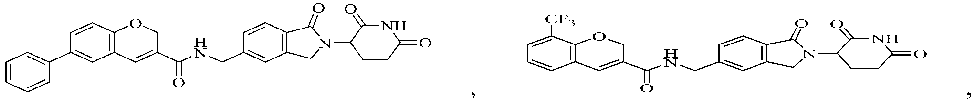 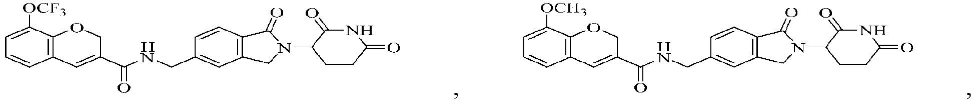 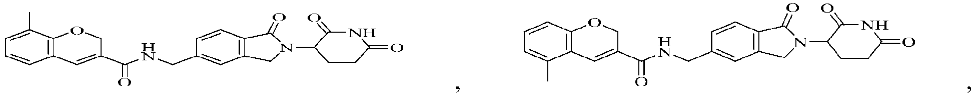 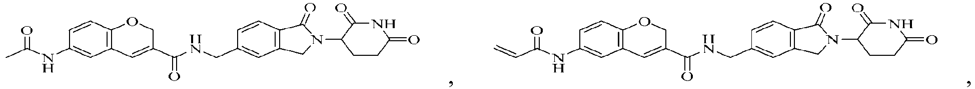 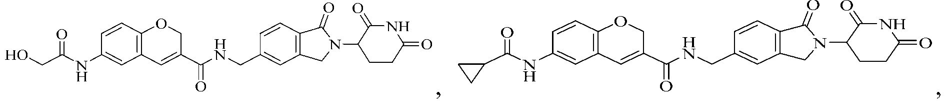 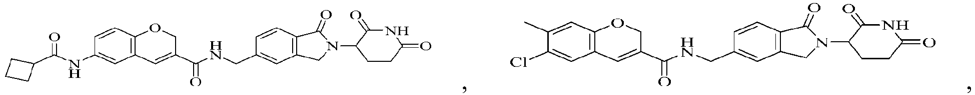 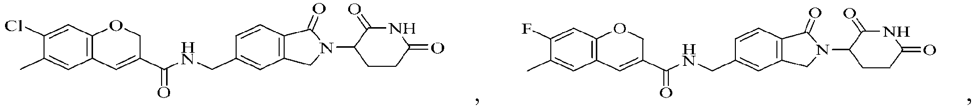 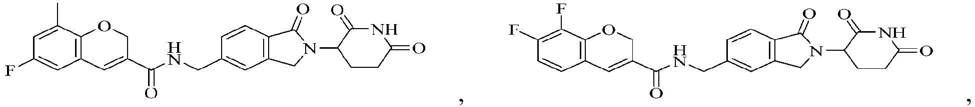 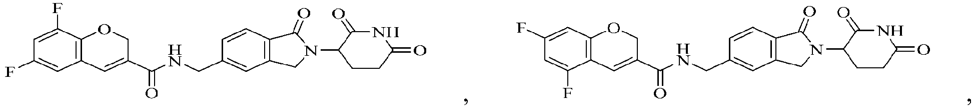 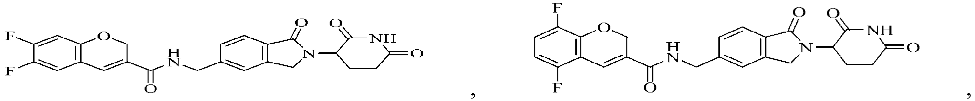 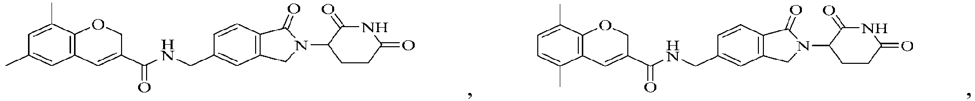 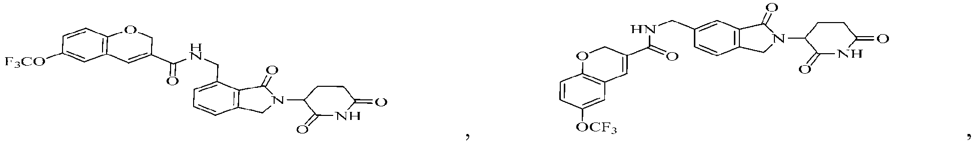 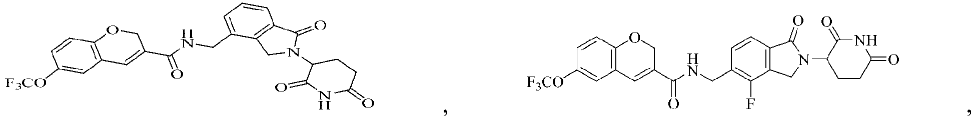 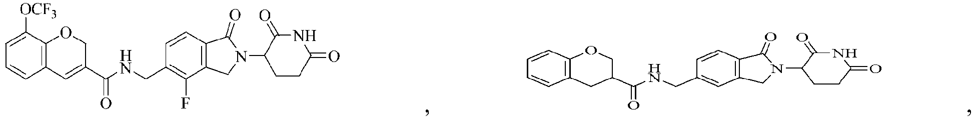 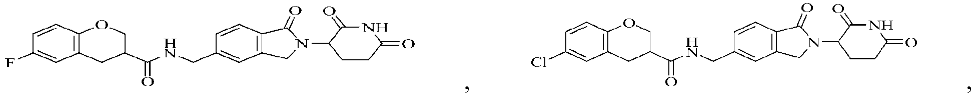 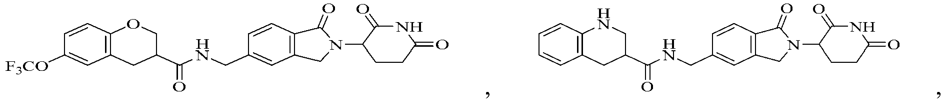 and

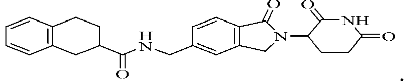

Another objective of the present disclosure is to provide a pharmaceutical composition comprising a therapeutically effective amount of the compound of general formula I or general formula II or the pharmaceutically acceptable salt, tautomer, mesomer, racemate, enantiomer, or diastereomer thereof and a pharmaceutically acceptable carrier or excipient.

The pharmaceutical composition of the present disclosure can be administered in various known ways, e.g., orally, parenterally, by inhalation spray, or via an implanted reservoir. The pharmaceutical composition of the present disclosure can be administered alone or in combination with other drugs. A composition for oral administration may be any orally acceptable dosage form, including but not limited to tablets, capsules, emulsions and suspensions, dispersions, and solutions. Commonly used pharmaceutically acceptable carriers or excipients include stabilizers, diluents, surfactants, lubricants, antioxidants, binders, colorants, fillers, emulsifiers, etc.

A sterile injectable composition can be formulated according to techniques known in the art using suitable dispersing or wetting agents and suspending agents. Pharmaceutically acceptable carriers and solvents that can be used include water, mannitol, sodium chloride solution, etc.

The actual dosage level of the active ingredient in the pharmaceutical composition of the present disclosure may be varied so as to obtain an amount of the active ingredient that is effective to achieve the desired therapeutic response for a particular patient, composition, and mode of administration, without being toxic to the patient. The selected dosage level will depend upon a variety of factors, including the activity of the specific compound or salt thereof of the present disclosure employed, the route of administration, the time of administration, the rate of excretion of the specific composition employed, the duration of the treatment, other drugs, compounds, and/or materials used in combination with the specific composition employed, the age, sex, body weight, general health, and prior medical history of the patient being treated, and similar factors well known in the medical arts.

Another objective of the present disclosure is to provide use of the compound of general formula I or general formula II or the pharmaceutically acceptable salt, tautomer, mesomer, racemate, enantiomer, or diastereomer thereof in the preparation of a medicament for treating or preventing a disease related to mutation, unbalanced expression, allosterism, and dysfunction of GSPT1, IKZF1, IKZF2, IKZF3, CK1α, N-MYC, or C-MYC protein.

The related disease is cancer, aging, an immune disease, or a neurological disease, wherein the cancer is selected from acute myeloid leukemia, liver cancer, acute lymphocytic leukemia, bladder cancer, bone cancer, brain cancer, breast cancer, cervical cancer, choriocarcinoma, chronic lymphocytic leukemia (CLL), chronic granulocytic leukemia (CML), colon cancer, esophageal cancer, gallbladder cancer, gastric cancer, gastrointestinal stromal tumor, head and neck cancer, Hodgkin lymphoma, laryngeal cancer, leukemia, lung cancer, melanoma, mesothelioma, multiple myeloma, ovarian cancer, pancreatic cancer, prostate cancer, rectal cancer, renal cancer, sarcoma, skin cancer, small cell lung cancer, testicular cancer, throat cancer, thyroid cancer, and uterine cancer.

The present disclosure further provides use of the compound of general formula I or general formula II or the pharmaceutically acceptable salt, tautomer, mesomer, racemate, enantiomer, or diastereomer thereof in the preparation of a GSPT1 degrader.

### Beneficial Effects:

Compared to the clinical drug CC-90009, the benzo six-membered heterocyclic compounds of the present disclosure, such as series A, B, and C, exhibited significantly improved degradation effects on GSPT1 protein and anti-proliferation inhibitory activity against AML cells. When 200 nM compound A-10 was administered to KG-1 cells for 4 hours, the percentage of degradation of GSPT1 protein was higher than 80%. Compounds A-1, A-3, and A-10 exhibited *in vitro* rat liver microsome metabolic half-lives that were about 2-5 times that of the clinical drug CC-90009, indicating that the compounds had excellent metabolic stability. In a pharmacokinetic study, compound A-1, when administered by intravenous injection, exhibited an *in vivo* clearance rate less than that of the clinical drug CC-90009, a half-life close to that of CC-90009, and a relatively good metabolic stability *in vivo,* and its initial plasma concentration and plasma drug exposure level were greatly superior to those of CC-90009. Compound A-10, when administered by intravenous injection, exhibited a clearance rate that was one third of that of CC-90009, a half-life longer than that of CC-90009, an initial plasma concentration much higher than that of CC-90009, and a plasma drug exposure level that was nearly four times that of CC-90009. Moreover, the compound, when administered orally, exhibited an exposure level that was nearly three times that of CC-90009, a relatively high peak, and a relatively small time to peak, suggesting that compound A-10 was absorbed faster. Compound A-42 exhibited a lower clearance rate, and higher intravenous and oral plasma drug exposure levels that were fourteen times and four times those of CC-90009, respectively. In addition, a pharmacodynamic study using a xenograft mouse model showed that compounds A-10 and A-42 exhibited relatively good *in vivo* anti-tumor effects, with the *in vivo* anti-tumor effect of A-10 being significantly better than that of the clinical molecule CC-90009 and the *in vivo* anti-tumor effect of A-42 being comparable to that of the clinical molecule MRT-2359. Thus, the compounds of the present disclosure can be used for the preparation of medicaments for treating or preventing diseases related to mutation, unbalanced expression, allosterism, and dysfunction of GSPT1, IKZF1, IKZF2, IKZF3, CK1α, N-MYC, or C-MYC protein, and can be used for the preparation of GSPT1 degraders.

Unless otherwise stated, the terms used in the specification and claims have the following meanings.

The term "isomer" includes enantiomeric, diastereomeric, and geometric (or conformational) isomeric forms of a given structure. For example, the present application includes R and S configurations for each asymmetric center, Z and E double bond isomers, Z and E conformational isomers, single stereochemical isomers, and enantiomeric, diastereomeric, and geometric (or conformational) isomer mixtures.

The term "pharmaceutically acceptable salt" refers to, for example, an acid addition salt thereof and/or a base salt thereof. Suitable acid addition salts are formed from acids that form non-toxic salts, e.g., hydrochlorides/chlorides. Suitable base salts are formed from bases that form non-toxic salts, e.g., calcium salts and sodium salts. Hemisalts of acids and bases, e.g., hemisulphates and hemicalcium salts, may also be formed.

The term "therapeutically effective amount" refers to an amount of a compound of the present application that (i) treats a specific disease, condition, or disorder; (ii) palliates, alleviates, or eliminates one or more symptoms of a specific disease, condition, or disorder; or (iii) prevents or delays the onset of one or more symptoms of the specific disease, condition, or disorder described in the present application.

The term "pharmaceutically acceptable carrier or excipient" refers to a non-toxic carrier, auxiliary material, or vehicle that does not destroy the pharmacological activity of the compound with which it is formulated.

The term "alkyl" refers to a saturated aliphatic hydrocarbon group. Alkyl groups containing 1 to 6 carbon atoms are preferred in the present disclosure. Non-limiting examples of lower alkyl groups containing 1 to 6 carbon atoms include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, etc.

The term "alkenyl" refers to an aliphatic hydrocarbon having at least one carbon-carbon double bond, including linear and branched chains having at least one carbon-carbon double bond. The alkenyl groups of the present disclosure preferably have 2 to 6 carbon atoms. For example, the term "C₂₋₆ alkenyl" includes linear or branched unsaturated groups of 2 to 6 carbon atoms (having at least one carbon-carbon double bond), including but not limited to ethenyl, 1-propenyl, 2-propenyl (allyl), isopropenyl, 2-methyl-1-propenyl, 1-butenyl, 2-butenyl, etc.

The term "alkynyl" refers to an aliphatic hydrocarbon having at least one carbon-carbon triple bond, including linear and branched chains having at least one carbon-carbon triple bond. The alkynyl groups of the present disclosure have 2 to 6 carbon atoms. For example, "C₂₋₆ alkynyl" includes linear or branched hydrocarbon chain alkynyl groups as defined above having 2 to 6 carbon atoms.

The term "alkoxy" refers to -O-(alkyl) and -O-(unsubstituted cycloalkyl), wherein the alkyl is as defined above. Non-limiting examples of alkoxy groups include methoxy, ethoxy, propoxy, butoxy, cyclopropyloxy, cyclobutoxy, cyclopentyloxy, and cyclohexyloxy.

The term "cycloalkyl" refers to a saturated or partially unsaturated monocyclic or polycyclic hydrocarbon substituent. The cycloalkyl groups of the present disclosure preferably contain 3 to 10 carbon atoms. Non-limiting examples of monocyclic cycloalkyl groups include cyclopropyl, cyclobutyl, cyclopentyl, cyclopentenyl, cyclohexyl, cyclohexenyl, cyclohexadienyl, cycloheptyl, cycloheptatrienyl, cyclooctyl, etc. Polycyclic cycloalkyl groups include spiro-ring, fused-ring, and bridged-ring cycloalkyl groups.

The term "spirocycloalkyl" refers to a 5- to 20-membered polycyclic group in which one carbon atom (referred to as a spiro atom) is shared between monocyclic rings; it may contain one or more double bonds, but none of the rings has a fully conjugated π-electron system. It is preferably 6- to 14-membered, and is more preferably 7- to 10-membered (e.g., 7-, 8-, 9-, or 10-membered). According to the number of spiro atoms shared between the rings, spirocycloalkyl may be monospirocycloalkyl, bispirocycloalkyl, or polyspirocycloalkyl, preferably monospirocycloalkyl and bispirocycloalkyl, and more preferably 4-membered/4-membered, 4-membered/5-membered, 4-membered/6-membered, 5-membered/5-membered, or 5-membered/6-membered monospirocycloalkyl.

The term "fused cycloalkyl" refers to a 5- to 20-membered all-carbon polycyclic group in which each ring in the system shares a pair of adjacent carbon atoms with the other rings in the system, wherein one or more rings may contain one or more double bonds, but none of the rings has a fully conjugated π-electron system. It is preferably 6- to 14-membered, and is more preferably 7- to 10-membered. According to the number of constituent rings, it may be bicyclic, tricyclic, tetracyclic, or polycyclic fused cycloalkyl, preferably bicyclic or tricyclic, and more preferably 5-membered/5-membered or 5-membered/6-membered bicycloalkyl.

The term "bridged cycloalkyl" refers to a 5- to 20-membered all-carbon polycyclic group in which any two rings share two carbon atoms that are not directly connected; it may contain one or more double bonds, but none of the rings has a fully conjugated π-electron system. It is preferably 6- to 14-membered, and is more preferably 7- to 10-membered. According to the number of constituent rings, it may be bicyclic, tricyclic, tetracyclic, or polycyclic bridged cycloalkyl, preferably bicyclic, tricyclic, or tetracyclic, and more preferably bicyclic or tricyclic.

The term "heterocyclyl" refers to a saturated or partially unsaturated monocyclic or polycyclic hydrocarbon substituent containing 3 to 20 ring atoms, wherein one or more of the ring atoms are heteroatoms selected from nitrogen, oxygen, and S(O)m (where m is an integer from 0 to 2), but a cyclic portion of -O-O-, -O-S-, or -S-S- is excluded, and the other ring atoms are carbon atoms. Preferably, it contains 3 to 10 ring atoms, of which 1-4 are heteroatoms; most preferably, it contains 3 to 8 ring atoms, of which 1-3 are heteroatoms; most preferably, it contains 5 to 6 ring atoms, of which 1-2 or 1-3 are heteroatoms. Non-limiting examples of monocyclic heterocyclyl groups include pyrrolidinyl, imidazolidinyl, tetrahydrofuranyl, tetrahydropyranyl, tetrahydrothienyl, dihydroimidazolyl, dihydrofuranyl, dihydropyrazolyl, dihydropyrrolyl, piperidinyl, piperazinyl, morpholinyl, thiomorpholinyl, homopiperazinyl, etc., preferably tetrahydropyranyl, piperidinyl, and pyrrolidinyl. Polycyclic heterocyclyl groups include spiroheterocyclyl groups, fused heterocyclyl groups, and bridged heterocyclyl groups.

The term "spiroheterocyclyl" refers to a 5- to 20-membered polycyclic heterocyclyl group in which one atom (referred to as a spiro atom) is shared between monocyclic rings, wherein one or more ring atoms are heteroatoms selected from nitrogen, oxygen, and S(O)m (where m is an integer from 0 to 2), and the other ring atoms are carbon atoms. It may contain one or more double bonds, but none of the rings has a fully conjugated π-electron system. It is preferably 6- to 14-membered, and is more preferably 7- to 10-membered. According to the number of spiro atoms shared between the rings, spiroheterocyclyl may be monospiroheterocyclyl, bispiroheterocyclyl, or polyspiroheterocyclyl, preferably monospiroheterocyclyl and bispiroheterocyclyl, and more preferably 4-membered/4-membered, 4-membered/5-membered, 4-membered/6-membered, 5-membered/5-membered, or 5-membered/6-membered monospiroheterocyclyl.

The term "fused heterocyclyl" refers to a 5- to 20-membered polycyclic heterocyclyl group in which each ring in the system shares a pair of adjacent atoms with the other rings in the system, wherein one or more rings may contain one or more double bonds, but none of the rings has a fully conjugated π-electron system; one or more ring atoms are heteroatoms selected from nitrogen, oxygen, and S(O)m (where m is an integer from 0 to 2), and the other ring atoms are carbon atoms. It is preferably 6- to 14-membered, and is more preferably 7- to 10-membered. According to the number of constituent rings, it may be bicyclic, tricyclic, tetracyclic, or polycyclic fused heterocyclyl, preferably bicyclic or tricyclic, and more preferably 5-membered/5-membered or 5-membered/6-membered bicyclic fused heterocyclyl.

The term "bridged heterocyclyl" refers to a 5- to 14-membered polycyclic heterocyclyl group in which any two rings share two atoms that are not directly connected; it may contain one or more double bonds, but none of the rings has a fully conjugated π-electron system, wherein one or more ring atoms are heteroatoms selected from nitrogen, oxygen, and S(O)m (where m is an integer from 0 to 2), and the other ring atoms are carbon atoms. It is preferably 6- to 14-membered, and is more preferably 7- to 10-membered. According to the number of constituent rings, it may be bicyclic, tricyclic, tetracyclic, or polycyclic bridged heterocyclyl, preferably bicyclic, tricyclic, or tetracyclic, and more preferably bicyclic or tricyclic.

The heterocyclyl includes those in which the heterocyclyl described above (including monocyclic, spiroheterocyclic, fused heterocyclic, and bridged heterocyclic rings) is fused to an aryl, heteroaryl, or cycloalkyl ring, wherein the ring attached to the parent structure is heterocyclyl; its non-limiting examples include:

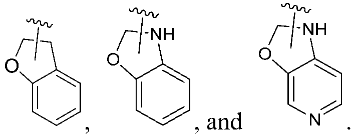

The term "aryl" refers to a 6- to 14-membered, preferably 6- to 10-membered, all-carbon monocyclic or fused polycyclic (i.e., rings sharing a pair of adjacent carbon atoms) group having a conjugated π-electron system, such as phenyl and naphthyl.

The term "heteroaryl" refers to a heteroaromatic system containing 1 to 4 heteroatoms and 5 to 14 ring atoms, wherein the heteroatoms are selected from oxygen, sulfur, and nitrogen. Preferably, heteroaryl groups are 5- to 10-membered and contain 1 to 3 heteroatoms; more preferably, heteroaryl groups are 5- or 6-membered and contain 1 to 2 heteroatoms; preferably, heteroaryl groups are, for example, imidazolyl, furanyl, thienyl, thiazolyl, pyrazolyl, oxazolyl, pyrrolyl, tetrazolyl, pyridinyl, pyrimidinyl, thiadiazole, pyrazinyl, pyridazinyl, etc.

The heteroaryl includes those in which the heteroaryl described above is fused to an aryl, heterocyclyl, or cycloalkyl ring, wherein the ring attached to the parent structure is a heteroaryl ring; its non-limiting examples include:

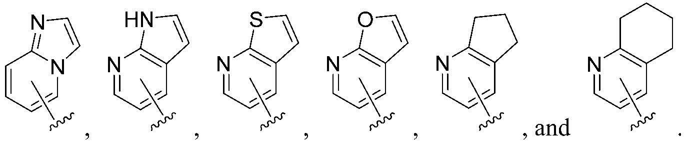

The term "hydroxyalkyl" refers to an alkyl group substituted with a hydroxyl group, wherein the alkyl is as defined above.

The term "haloalkyl" refers to an alkyl group substituted with one or more halogens, wherein the alkyl is as defined above.

The term "haloalkoxy" refers to an alkoxy group substituted with one or more halogens, wherein the alkoxy is as defined above.

### BRIEF DESCRIPTION OF THE DRAWINGS

FIG. 1 shows concentration-dependent degradation of GSPT1 in AML cells by compound A-10 of the present disclosure, wherein FIG. 1A shows the degradation of GSPT1 in KG-1 cells by compound A-10 and the clinical drug CC-90009; FIG. 1B shows the GSPT1 protein quantification results in panel A; FIG. 1C shows the degradation of GSPT1 in MV4-11 cells by compound A-10 and the clinical drug CC-90009; FIG. 1D shows the GSPT1 protein quantification results in panel C.
FIG. 2 shows time-dependent degradation of GSPT1 in AML cells by compound A-10 of the present disclosure.
FIG. 3 shows the results of a study on the metabolic stability of compounds A-1, A-3, A-10, A-42, and B-1 of the present disclosure and the clinical drug CC-90009 in *in vitro* rat liver microsomes.

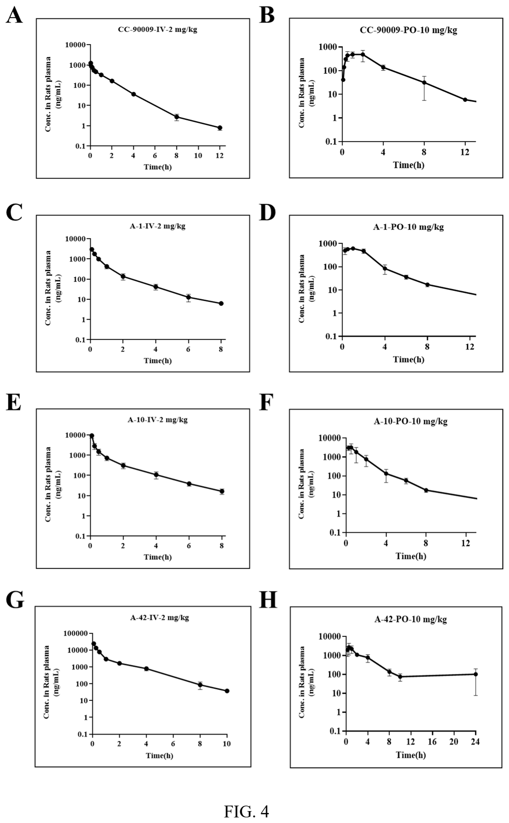

FIG. 4 shows the results of an *in vivo* pharmacokinetic study of compounds A-1, A-10, and A-42 of the present disclosure in rats, wherein FIGs. 4A and 4B show plasma concentration-time curves of intravenous administration and oral administration of CC-90009, respectively; FIGs. 4C and 4D show plasma concentration-time curves of intravenous administration and oral administration of A-1, respectively; FIGs. 4E and 4F show plasma concentration-time curves of intravenous administration and oral administration of A-10, respectively; FIGs. 4G and 4H show plasma concentration-time curves of intravenous administration and oral administration of A-42, respectively.
FIG. 5 shows an *in vivo* pharmacodynamic study of compound A-10 of the present disclosure in an MV411 xenograft mouse model, wherein FIG. 5A shows the body weight of mice in groups; FIG. 5B shows the tumor growth curves of mice in groups; FIG. 5C shows the tumor mass of mice in groups;
FIG. 5D shows the dissection of tumor tissues of mice in groups.
FIG. 6 shows an *in vivo* pharmacodynamic study of compound A-42 of the present disclosure in an MV411 xenograft mouse model, wherein FIG. 6A shows the body weight of mice in groups; FIG. 6B shows the tumor growth curves of mice in groups.
FIG. 7 shows an *in vivo* pharmacodynamic study of compound A-10 of the present disclosure in an 22RV1 xenograft mouse model, wherein FIG. 7A shows the body weight of mice in groups; FIG. 7B shows the tumor growth curves of mice in groups; FIG. 6C shows the tumor mass of mice in groups;
FIG. 6D shows the dissection of tumor tissues of mice in groups.

### DETAILED DESCRIPTION

The technical solutions of the present disclosure are described in detail below using specific examples, but the scope of protection of the present disclosure is not limited to the examples.

### Synthesis of key intermediate-1:

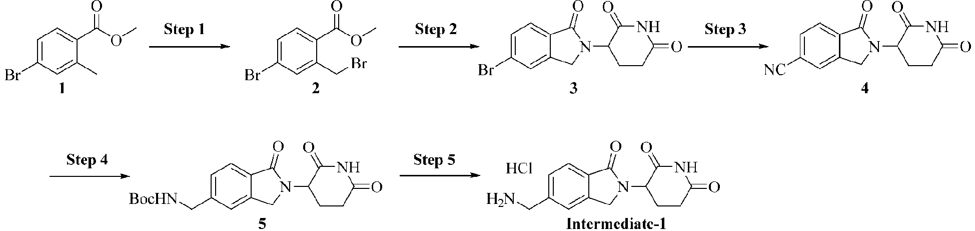

Step 1: Methyl 4-bromo-2-methylbenzoate (10.00 g, 43.86 mmol) was dissolved in 200 mL of carbon tetrachloride. N-Bromosuccinimide (15.61 g, 87.73 mmol) and azobisisobutyronitrile (0.72 g, 4.38 mmol) were added sequentially. After the addition, the mixture was heated at reflux and stirred for 12 hours. TLC monitoring showed that the reaction was complete. The reaction solution was cooled to room temperature and then filtered under vacuum. The filtrate was concentrated under vacuum, and the residue was purified by silica gel column chromatography (DCM:MeOH = 40:1, v/v) to give compound **2,** methyl 4-bromo-2-(bromomethyl)benzoate, as a white solid (8.32 g, yield: 62.0%). HRMS (ESI⁺): calculated C₉H₉Br₂O₂ (M + H)⁺, 306.8964; found 306.8958.

Step 2: Methyl 4-bromo-2-(bromomethyl)benzoate (8.00 g, 26.15 mmol) was dissolved in 60 mL of N,N-dimethylformamide. 3-Aminopiperidine-2,6-dione hydrochloride (5.15 g, 31.38 mmol) and DIPEA (8.45 g, 65.38 mmol) were added sequentially. After the addition, the reaction solution was heated to 90 °C and stirred for 4 hours in a nitrogen atmosphere. TLC monitoring showed that the reaction was complete. The reaction solution was poured into 200 mL of water, and a light blue solid precipitated. The mixture was left to stand until the precipitation was complete, and filtration was performed under vacuum. The filter cake was dried *in vacuo* to give compound **3,** 3-(5-bromo-1-oxoisoindolin-2-yl)piperidine-2,6-dione, as a light blue solid (5.89 g, yield: 70.2%). HRMS (ESI⁺): calculated C₁₃H₁₂BrN₂O₃ (M + H)⁺, 323.0026; found 323.0030.

Step 3: 3-(5-Bromo-1-oxoisoindolin-2-yl)piperidine-2,6-dione (5.00 g, 15.53 mmol) was dissolved in 50 mL of dry N,N-dimethylformamide. 1,1'-Bis(diphenylphosphino)ferrocene (0.40 g, 0.71 mmol), tris(dibenzylideneacetone)dipalladium (0.39 g, 0.43 mmol), and zinc cyanide (1.88 g, 16.00 mmol) were added sequentially. After the addition, the mixture was heated to 90 °C and stirred for 4 hours in a nitrogen atmosphere. TLC monitoring showed that the reaction was complete. The reaction solution was filtered under vacuum. The filtrate was poured into 150 mL of water, and a green solid precipitated. The mixture was left to stand until the precipitation was complete, and filtration was performed under vacuum. The filter cake was dried *in vacuo* to give compound **4,** 2-(2,6-dioxopiperidin-3-yl)-1-oxoisoindoline-5-carbonitrile, as a light green solid (3.84 g, yield: 91.9%). HRMS (ESI⁺): calculated C₁₄H₁₂N₃O₃ (M + H)⁺, 270.0873; found 270.0882.

Step 4: 2-(2,6-Dioxopiperidin-3-yl)-1-oxoisoindoline-5-carbonitrile (3.50 g, 13.00 mmol) was dissolved in 40 mL of N,N-dimethylformamide. Di-tert-butyl dicarbonate (5.67 g, 26.00 mmol) and 15 mL of Raney nickel were added sequentially. After the addition, the mixture was fully purged with hydrogen, heated to 50 °C, and stirred for 5 hours. TLC monitoring showed that the reaction was complete. The reaction solution was filtered under vacuum. The filter cake was washed with ethyl acetate, and the filtrate was poured into 120 mL of water and extracted with ethyl acetate. The organic phases were combined, washed with water and then with saturated brine, dried over anhydrous sodium sulfate, and then concentrated under vacuum to give compound 5, tert-butyl ((2-(2,6-dioxopiperidin-3-yl)-1-oxoisoindolin-5-yl)methyl)carbamate, as a brownish yellow solid (3.20 g, yield: 65.9%). The product could be directly used in the next step without further purification. HRMS (ESI⁺): calculated C₁₉H₂₄N₃O₅ (M + H)⁺, 374.1710; found 374.1721.

Step 5: tert-Butyl ((2-(2,6-dioxopiperidin-3-yl)-1-oxoisoindolin-5-yl)methyl)carbamate (3.00 g, 8.04 mmol) was dissolved in 30 mL of a solution of hydrochloric acid in dioxane (4 M), and the solution was stirred at room temperature for 3 hours. TLC monitoring showed that the reaction was complete. The reaction solution was filtered under vacuum, and the filter cake was dried *in vacuo* to give **intermediate-1,** 3-(5-(aminomethyl)-1-oxoisoindolin-2-yl)piperidine-2,6-dione hydrochloride, as a pale yellow solid (1.80 g, yield: 72.4%). The product could be directly used in the next step without further purification. HRMS (ESI⁺): calculated C₁₄H₁₆N₃O₃ (M + H)⁺, 274.1186; found 274.1184.

### Synthesis of key intermediate-2:

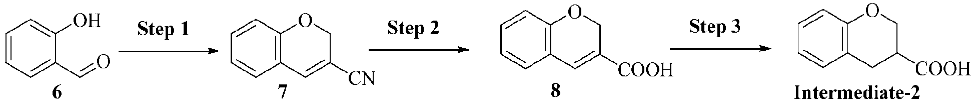

Step 1: 2-Hydroxybenzaldehyde (2.00 g, 16.39 mmol) was dissolved in 15 mL of acrylonitrile. 1,4-Diazidobicyclo[2.2.2]octane (1.84 g, 16.39 mmol) was added. After the addition, the mixture was heated at reflux and stirred for 3 hours in a nitrogen atmosphere. TLC monitoring showed that the reaction was complete. The reaction solution was poured into 30 mL of a 10% aqueous sodium hydroxide solution and extracted with ethyl acetate. The organic phases were combined, washed with saturated brine, dried over anhydrous sodium sulfate, and concentrated under vacuum to give compound **7,** 2H-chromene-3-carbonitrile, as a yellow crystalline solid (2.30 g, yield: 90.2%). The product could be directly used in the next step without further purification. HRMS (ESI⁺): calculated C₁₀H₈NO (M + H)⁺, 158.0600; found 158.0621.

Step 2: 2H-Chromene-3-carbonitrile (2.30 g, 14.73 mmol) was dissolved in 40 mL of a 10% aqueous sodium hydroxide solution, and the solution was heated to 60 °C and stirred for 10 hours. TLC monitoring showed that the reaction was complete. Dilute hydrochloric acid (2 M) was added to adjust the pH to 4-5, and a white solid precipitated. The mixture was left to stand until the precipitation was complete, and filtration was performed under vacuum. The filter cake was dried to give compound **8,** 2H-chromene-3-carboxylic acid, as a yellow solid (1.65 g, yield: 63.7%). HRMS (ESI⁻): calculated C₁₀H₇O₃ (M - H)⁻, 175.0401; found 175.0400.

Step 3: 2H-Chromene-3-carboxylic acid (1.50 g, 8.52 mmol) was dissolved in 20 mL of methanol. 10% Pd/C (1.00 g) was added. After the addition, the mixture was stirred at room temperature for 8 hours. TLC monitoring showed that the reaction was complete. The reaction solution was filtered under vacuum. The filter cake was washed with methanol, and the filtrate was concentrated under vacuum to give **intermediate-2,** chromane-3-carboxylic acid, as a brownish yellow solid (0.76 g, yield: 50.2%). HRMS (ESI⁻): calculated C₁₀H₉O₃ (M - H)⁻, 177.0557; found 177.0545.

### Synthesis of key intermediate-3:

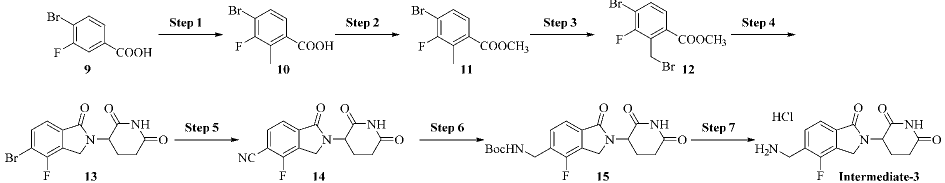

Step 1: 4-Bromo-3-fluorobenzoic acid **(9,** 15.00 g, 68.49 mmol) was dissolved in 150 mL of anhydrous tetrahydrofuran in a nitrogen atmosphere. The solution was cooled to -70 °C, and lithium diisopropylamide (72 mL, 143.8 mmol) was added dropwise. After the addition, the mixture was left to react for another hour, and iodomethane (29.16 g, 205.5 mmol) was added dropwise. The mixture was left to react at room temperature for 12 hours. TLC monitoring showed that the reaction was complete. 150 mL of a saturated ammonium chloride solution was added dropwise to the reaction solution. Extraction was performed with ethyl acetate. The organic phases were combined, washed with saturated brine, dried over anhydrous sodium sulfate, and concentrated under vacuum to give compound **10,** 4-bromo-3-fluoro-2-methylbenzoic acid, as a yellow crystalline solid (9.67 g, yield: 60.6%). The product could be directly used in the next step without further purification. HRMS (ESI⁻): calculated C₈H₅BrFO₂ (M - H)⁻, 230.9462; found 230.9455.

Step 2: 4-Bromo-3-fluoro-2-methylbenzoic acid **(10, 9.67** g, 41.50 mmol) was dissolved in 80 mL of N,N-dimethylformamide. Potassium carbonate (11.47 g, 83.01 mmol) and iodomethane (8.84 g, 62.26 mmol) were added sequentially. After the addition, the mixture was left to react at room temperature for 2 hours. TLC monitoring showed that the reaction was complete. The reaction solution was filtered under vacuum, and the filtrate was poured into 300 mL of water and extracted with ethyl acetate. The organic phases were combined, washed with water and then with saturated brine, dried over anhydrous sodium sulfate, and then concentrated under vacuum to give compound **11,** methyl 4-bromo-3-fluoro-2-methylbenzoate, as a yellow oil (9.29 g, yield: 90.6%). The product could be directly used in the next step without further purification. HRMS (ESI⁺): calculated C₉H₉BrFO₂ (M + H)⁺, 246.9764; found 246.9752.

Step 3: Methyl 4-bromo-3-fluoro-2-methylbenzoate **(11,** 9.29 g, 37.60 mmol) was dissolved in 150 mL of chloroform. N-Bromosuccinimide (16.73 g, 94.00 mmol) and azobisisobutyronitrile (1.85 g, 11.28 mmol) were added sequentially. After the addition, the mixture was heated at reflux and left to react for 10 hours. TLC monitoring showed that the reaction was complete. The reaction solution was filtered under vacuum, and the filtrate was concentrated under vacuum and slurried with petroleum ether to give compound **12,** methyl 4-bromo-2-(bromomethyl)-3-fluorobenzoate, as a white solid (10.36 g, yield: 84.5%). HRMS (ESI⁺): calculated C₉H₈Br₂FO₂ (M + H)⁺, 324.8870; found 324.8812.

Steps 4-7: With reference to the synthesis method for **intermediate-1,** methyl 4-bromo-2-(bromomethyl)-3-fluorobenzoate was used as the starting material to prepare intermediate-3: 3-(5-(aminomethyl)-4-fluoro-1-oxoisoindolin-2-yl)piperidine-2,6-dione hydrochloride (a white solid). HRMS (ESI⁺): calculated C₁₄H₁₅FN₃O₃ (M + H)⁺, 292.1092; found 292.1077.

### Synthesis of clinical drug CC-90009:

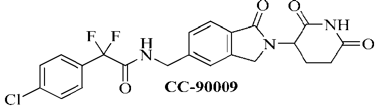

3-(5-Aminomethyl)-1-oxoisoindolin-2-yl)piperidine-2,6-dione hydrochloride (0.10 g, 0.32 mmol) was dissolved in 5 mL of N,N-dimethylformamide. 2-(4-Chlorophenyl)-2,2-difluoroacetic acid (0.08 g, 0.39 mmol) and HATU (0.19 g, 0.49 mmol) were added sequentially. After the addition, the mixture was stirred at room temperature for 1 hour. DIPEA (0.13 g, 0.97 mmol) was added, and the mixture was stirred overnight at room temperature. TLC monitoring showed that the reaction was complete. The reaction solution was poured into 30 mL of iced water, and a brown solid precipitated. The mixture was left to stand until the precipitation was complete, and filtration was performed under vacuum. The filter cake was purified by silica gel column chromatography (dichloromethane:methanol = 50:1, v/v) to give CC-90009 as a white solid (0.10 g, yield: 68.0%, purity: 99.37%). ¹H NMR (300 MHz, DMSO-*d*₆) *δ* (ppm) 11.00 (s, 1H), 9.70 (t, *J =* 6.1 Hz, 1H), 7.68 (d, *J =* 7.8 Hz, 1H), 7.62 (s, 4H), 7.41 (s, 1H), 7.37 (d, *J* = 7.8 Hz, 1H), 5.11 (dd, *J =* 13.3, 5.0 Hz, 1H), 4.53-4.22 (m, 4H), 3.02-2.83 (m, 1H), 2.60 (d, *J* = 17.3 Hz, 1H), 2.47-2.30 (m, 1H), 2.06-1.94 (m, 1H). HRMS (ESI⁺): calculated C₂₂H₁₉ClF₂N₃O₄ (M + H)⁺, 462.1027; found 462.1025.

### Example 1: Synthesis of N-((2-(2,6-Dioxopiperidin-3-yl)-1-oxoisoindolin-5-yl)methyl)-2H-chromene-3-carboxamide (Compound A-1)

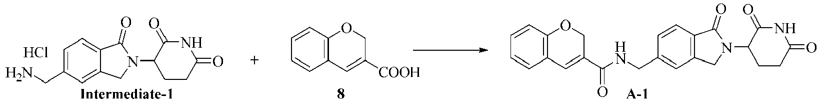

3-(5-Aminomethyl)-1-oxoisoindolin-2-yl)piperidine-2,6-dione hydrochloride (intermediate-1, 0.10 g, 0.32 mmol) was dissolved in 5 mL of N,N-dimethylformamide. 2H-Chromene-3-carboxylic acid (compound 8, 0.07 g, 0.39 mmol) and HATU (0.19 g, 0.49 mmol) were added sequentially. After the addition, the mixture was stirred at room temperature for 1 hour. DIPEA (0.13 g, 0.97 mmol) was added, and the mixture was stirred overnight at room temperature. TLC monitoring showed that the reaction was complete. The reaction solution was poured into 30 mL of iced water, and a brown solid precipitated. The mixture was left to stand until the precipitation was complete, and filtration was performed under vacuum. The filter cake was purified by silica gel column chromatography (dichloromethane:methanol = 50:1, v/v) to give compound A-1 as a white solid (0.10 g, yield: 71.9%, purity: 98.95%). ¹H NMR (300 MHz, DMSO-*d*₆) *δ* (ppm) 11.00 (s, 1H), 8.90 (t, *J =* 6.0 Hz, 1H), 7.70 (d, *J* = 7.8 Hz, 1H), 7.53 (s, 1H), 7.45 (d, *J* = 7.3 Hz, 1H), 7.33 (s, 1H), 7.29-7.18 (m, 2H), 6.96 (t, *J =* 7.4 Hz, 1H), 6.85 (d, *J* = 8.1 Hz, 1H), 5.11 (dd, *J =* 13.3, 5.1 Hz, 1H), 4.94 (s, 2H), 4.56-4.24 (m, 4H), 3.01-2.81 (m, 1H), 2.60 (d, *J =* 17.5 Hz, 1H), 2.49-2.28 (m, 1H), 2.08-1.93 (m, 1H). HRMS (ESI⁺): calculated C₂₄H₂₂N₃O₅ (M + H)⁺, 432.1554; found 432.1539.

### Example 2: Synthesis of N-((2-(2,6-Dioxopiperidin-3-yl)-1-oxoisoindolin-5-yl)methyl)-5-fluoro-2H-chromene-3-carboxamide (Compound A-2)

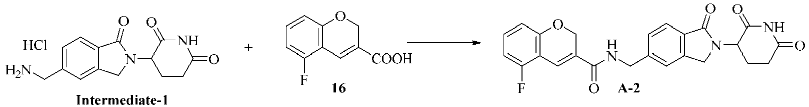

Synthesis of 5-fluoro-2H-chromene-3-carboxylic acid (16): With reference to the synthesis method for intermediate-2, 6-fluorosalicylaldehyde was used as the starting material to prepare the compound, with a two-step yield of 52.4%. HRMS (ESI⁻): calculated C₁₀H₆FO₃ (M - H)⁻, 193.0306; found 193.0318.

Synthesis of compound A-2: With reference to the synthesis method for compound A-1 in Example 1, 5-fluoro-2H-chromene-3-carboxylic acid was used as the starting material to prepare compound A-2 (a white solid, 0.08 g, yield: 55.9%, purity: 95.93%). ¹H NMR (300 MHz, DMSO-*d*₆) *δ (ppm)* 11.00 (s, 1H), 8.96 (t, *J* = 6.0 Hz, 1H), 7.70 (d, *J* = 7.8 Hz, 1H), 7.53 (s, 1H), 7.45 (d, *J* = 7.8 Hz, 1H), 7.37 (s, 1H), 7.26-7.16 (m, 1H), 7.08 (d, *J* = 7.6 Hz, 1H), 7.00-6.88 (m, 1H), 5.11 (dd, *J* = 13.3, 5.0 Hz, 1H), 5.02 (s, 2H), 4.55-4.23 (m, 4H), 3.02-2.83 (m, 1H), 2.60 (d, *J* = 17.3 Hz, 1H), 2.47-2.30 (m, 1H), 2.06-1.93 (m, 1H). HRMS (ESI⁺): calculated C₂₄H₂₁FN₃O₅ (M + H)⁺, 450.1460; found 450.1436.

### Example 3: Synthesis of N-((2-(2,6-Dioxopiperidin-3-yl)-1-oxoisoindolin-5-yl)methyl)-6-fluoro-2H-chromene-3-carboxamide (Compound A-3)

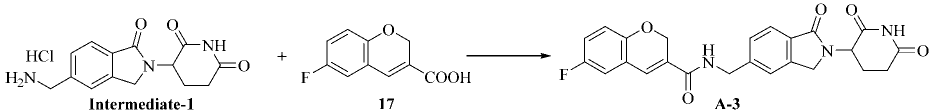

Synthesis of 6-fluoro-2H-chromene-3-carboxylic acid (17): With reference to the synthesis method for intermediate-2, 5-fluorosalicylaldehyde was used as the starting material to prepare the compound, with a two-step yield of 62.1%. HRMS (ESI⁻): calculated C₁₀H₆FO₃ (M - H)⁻, 193.0306; found 193.0310.

Synthesis of compound A-3: With reference to the synthesis method for compound A-1 in Example 1, 6-fluoro-2H-chromene-3-carboxylic acid was used as the starting material to prepare A-3 (a white solid, 0.12 g, yield: 83.9%, purity: 98.72%). ¹H NMR (300 MHz, DMSO-*d*₆) *δ (ppm)* 11.02 (s, 1H), 8.97 (t, *J* = 6.0 Hz, 1H), 7.70 (d, *J* = 7.8 Hz, 1H), 7.52 (s, 1H), 7.45 (d, *J* = 7.2 Hz, 1H), 7.30 (s, 1H), 7.18-7.12 (m, 1H), 7.12-7.03 (m, 1H), 6.89 (dd, *J =* 8.8, 4.6 Hz, 1H), 5.12 (dd, *J =* 13.3, 5.0 Hz, 1H), 4.94 (s, 2H), 4.54-4.24 (m, 4H), 3.00-2.85 (m, 1H), 2.60 (d, *J =* 17.9 Hz, 1H), 2.47-2.29 (m, 1H), 2.06-1.93 (m, 1H). HRMS (ESI⁺): calculated C₂₄H₂₁FN₃O₅ (M + H)⁺, 450.1460; found 450.1428.

### Example 4: Synthesis of N-((2-(2,6-Dioxopiperidin-3-yl)-1-oxoisoindolin-5-yl)methyl)-7-fluoro-2H-chromene-3-carboxamide (Compound A-4)

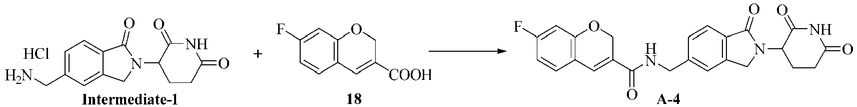

Synthesis of 7-fluoro-2H-chromene-3-carboxylic acid (18): With reference to the synthesis method for intermediate-2, 4-fluorosalicylaldehyde was used as the starting material to prepare the compound, with a two-step yield of 56.8%. HRMS (ESI⁻): calculated C₁₀H₆FO₃ (M - H)⁻, 193.0306; found 193.0321.

Synthesis of compound A-4: With reference to the synthesis method for compound A-1, 7-fluoro-2H-chromene-3-carboxylic acid was used as the starting material to prepare compound A-4 (a white solid, 0.08 g, yield: 55.9%, purity: 95.38%). ¹H NMR (300 MHz, DMSO-*d*₆) *δ (ppm)* 11.02 (s, 1H), 8.92 (t, *J =* 6.1 Hz, 1H), 7.70 (d, *J =* 7.8 Hz, 1H), 7.52 (s, 1H), 7.45 (d, *J =* 7.9 Hz, 1H), 7.34 (s, 1H), 7.29 (t, *J* = 7.4 Hz, 1H), 6.86-6.74 (m, 2H), 5.12 (dd, *J =* 13.2, 5.0 Hz, 1H), 4.98 (s, 2H), 4.55-4.24 (m, 4H), 3.02-2.84 (m, 1H), 2.60 (d, *J =* 15.9 Hz, 1H), 2.47-2.30 (m, 1H), 2.07-1.94 (m, 1H). HRMS (ESI⁺): calculated C₂₄H₂₁FN₃O₅ (M + H)⁺, 450.1460; found 450.1454.

### Example 5: Synthesis of N-((2-(2,6-Dioxopiperidin-3-yl)-1-oxoisoindolin-5-yl)methyl)-8-fluoro-2H-chromene-3-carboxamide (Compound A-5)

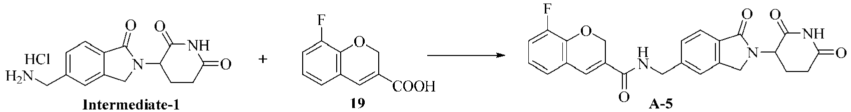

Synthesis of 8-fluoro-2H-chromene-3-carboxylic acid (19): With reference to the synthesis method for intermediate-2, 3-fluorosalicylaldehyde was used as the starting material to prepare the compound, with a two-step yield of 59.2%. HRMS (ESI⁻): calculated C₁₀H₆FO₃ (M - H)⁻, 193.0306; found 193.0311.

Synthesis of compound A-5: With reference to the synthesis method for compound A-1, 8-fluoro-2H-chromene-3-carboxylic acid was used as the starting material to prepare A-5 (a white solid, 0.09 g, yield: 62.9%, purity: 99.89%). ¹H NMR (300 MHz, DMSO-*d*₆) *δ* (ppm) 11.00 (s, 1H), 9.07 (t, *J* = 6.0 Hz, 1H), 7.70 (d, *J =* 7.8 Hz, 1H), 7.51 (d, *J =* 7.3 Hz, 2H), 7.45 (d, *J =* 7.9 Hz, 1H), 7.35-7.21 (m, 1H), 6.84 (t*, J =* 8.8 Hz, 1H), 6.74 (d, *J =* 8.2 Hz, 1H), 5.11 (dd, *J =* 13.3, 5.0 Hz, 1H), 4.97 (s, 2H), 4.57-4.24 (m, 4H), 3.02-2.85 (m, 1H), 2.60 (d, *J* = 17.0 Hz, 1H), 2.47-2.33 (m, 1H), 2.10-1.92 (m, 1H). HRMS (ESI⁺): calculated C₂₄H₂₁FN₃O₅ (M + H)⁺, 450.1460; found 450.1432.

### Example 6: Synthesis of N-((2-(2,6-Dioxopiperidin-3-yl)-1-oxoisoindolin-5-yl)methyl)-6-chloro-2H-chromene-3-carboxamide (Compound A-6)

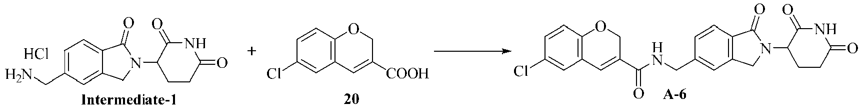

Synthesis of 6-chloro-2H-chromene-3-carboxylic acid (20): With reference to the synthesis method for intermediate-2, 5-chlorosalicylaldehyde was used as the starting material to prepare the compound, with a two-step yield of 62.5%. HRMS (ESI⁻): calculated C₁₀H₆ClO₃ (M - H)⁻, 209.0011; found 209.0023.

Synthesis of compound A-6: With reference to the synthesis method for compound A-1, 6-chloro-2H-chromene-3-carboxylic acid was used as the starting material to prepare A-6 (a white solid, 0.06 g, yield: 40.5%, purity: 97.87%). ¹H NMR (300 MHz, DMSO-*d*₆) *δ* (ppm) 11.00 (s, 1H), 8.93 (t, *J* = 6.1 Hz, 1H), 7.70 (d, *J =* 7.7 Hz, 1H), 7.53 (s, 1H), 7.45 (d, *J =* 7.8 Hz, 1H), 7.32 (s, 1H), 7.28 (s, 1H), 7.25 (s, 1H), 6.88 (d, *J* = 8.6 Hz, 1H), 5.11 (dd, *J* = 13.4, 5.0 Hz, 1H), 4.97 (s, 2H), 4.57-4.26 (m, 4H), 3.04-2.82 (m, 1H), 2.60 (d, *J =* 17.3 Hz, 1H), 2.44-2.29 (m, 1H), 2.08-1.91 (m, 1H). HRMS (ESI⁺): calculated C₂₄H₂₁ClN₃O₅ (M + H)⁺, 466.1164; found 466.1169.

### Example 7: Synthesis of N-((2-(2,6-Dioxopiperidin-3-yl)-1-oxoisoindolin-5-yl)methyl)-6-methyl-2H-chromene-3-carboxamide (Compound A-7)

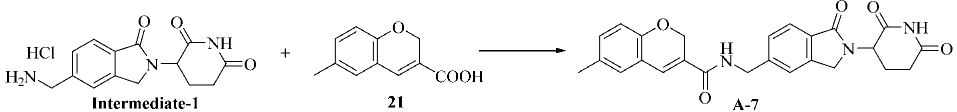

Synthesis of 6-methyl-2H-chromene-3-carboxylic acid (21): With reference to the synthesis method for intermediate-2, 5-methylsalicylaldehyde was used as the starting material to prepare the compound, with a two-step yield of 57.6%. HRMS (ESI⁻): calculated C₁₁H₉O₃ (M - H), 189.0557; found 189.0548.

Synthesis of compound A-7: With reference to the synthesis method for compound A-1, 6-methyl-2H-chromene-3-carboxylic acid was used as the starting material to prepare A-7 (a white solid, 0.07 g, yield: 49.3%, purity: 98.76%). ¹H NMR (300 MHz, DMSO-*d*₆) *δ (ppm)* 11.00 (s, 1H), 8.89 (t, *J =* 6.0 Hz, 1H), 7.70 (d, *J* = 7.8 Hz, 1H), 7.52 (s, 1H), 7.45 (d, *J* = 7.8 Hz, 1H), 7.29 (s, 1H), 7.04 (d, *J* = 8.1 Hz, 2H), 6.75 (d, *J =* 7.9 Hz, 1H), 5.11 (dd, *J* = 13.2, 5.0 Hz, 1H), 4.90 (s, 2H), 4.56-4.24 (m, 4H), 2.99-2.83 (m, 1H), 2.60 (d, *J =* 17.0 Hz, 1H), 2.48-2.31 (m, 1H), 2.23 (s, 3H), 2.07-1.93 (m, 1H). HRMS (ESI⁺): calculated C₂₅H₂₄N₃O₅ (M + H)⁺, 446.1711; found 446.1706.

### Example 8: Synthesis of N-((2-(2,6-Dioxopiperidin-3-yl)-1-oxoisoindolin-5-yl)methyl)-6-methoxy-2H-chromene-3-carboxamide (Compound A-8)

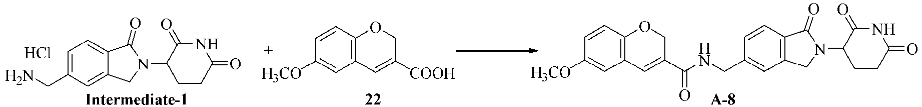

Synthesis of 6-methoxy-2H-chromene-3-carboxylic acid (22): With reference to the synthesis method for intermediate-2, 5-methoxysalicylaldehyde was used as the starting material to prepare the compound, with a two-step yield of 58.6%. HRMS (ESI⁻): calculated C₁₁H₉O₄ (M - H)⁻, 205.0506; found 205.0511.

Synthesis of compound A-8: With reference to the synthesis method for compound A-1, 6-methoxy-2H-chromene-3-carboxylic acid was used as the starting material to prepare A-8 (a white solid, 0.06 g, yield: 40.8%, purity: 99.05%). ¹H NMR (300 MHz, DMSO-*d*₆) *δ* (ppm) 10.99 (s, 1H), 8.88 (t, *J =* 6.0 Hz, 1H), 7.70 (d, *J =* 7.8 Hz, 1H), 7.52 (s, 1H), 7.45 (d, *J =* 7.8 Hz, 1H), 7.30 (s, 1H), 6.88-6.75 (m, 3H), 5.11 (dd, *J* = 13.3, 5.1 Hz, 1H), 4.87 (s, 2H), 4.56-4.25 (m, 4H), 3.71 (s, 3H), 3.00-2.83 (m, 1H), 2.60 (d, *J* = 17.1 Hz, 1H), 2.47-2.30 (m, 1H), 2.07-1.94 (m, 1H). HRMS (ESI⁺): calculated C₂₅H₂₄N₃O₆ (M + H)⁺, 462.1660; found 462.1659.

### Example 9: Synthesis of N-((2-(2,6-Dioxopiperidin-3-yl)-1-oxoisoindolin-5-yl)methyl)-5-methoxy-2H-chromene-3-carboxamide (Compound A-9)

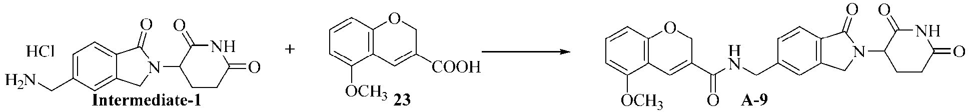

Synthesis of 5-methoxy-2H-chromene-3-carboxylic acid (23): With reference to the synthesis method for intermediate-2, 6-methoxysalicylaldehyde was used as the starting material to prepare the compound, with a two-step yield of 62.5%. HRMS (ESI⁻): calculated C₁₁H₉O₄ (M - H)⁻, 205.0506; found 205.0515.

Synthesis of compound A-9: Through the synthesis method for compound A-1, 5-methoxy-2H-chromene-3-carboxylic acid was used as the starting material to prepare A-9 (a white solid, 0.07 g, yield: 47.6%, purity: 99.23%). ¹H NMR (300 MHz, DMSO-*d*₆) *δ (ppm)* 10.99 (s, 1H), 8.97 (t, *J =* 6.0 Hz, 1H), 7.69 (d, *J* = 7.8 Hz, 1H), 7.53 (d, *J* = 11.1 Hz, 2H), 7.44 (d, *J* = 7.9 Hz, 1H), 7.21 (t, *J* = 8.2 Hz, 1H), 6.63 (d, *J =* 8.4 Hz, 1H), 6.49 (d, *J =* 8.2 Hz, 1H), 5.11 (dd, *J =* 13.3, 5.0 Hz, 1H), 4.86 (s, 2H), 4.53-4.24 (m, 4H), 3.83 (s, 3H), 2.99-2.83 (m, 1H), 2.60 (d*, J =* 17.1 Hz, 1H), 2.49-2.28 (m, 1H), 2.07-1.89 (m, 1H). HRMS (ESI⁺): calculated C₂₅H₂₄N₃O₆ (M + H)⁺, 462.1660; found 462.1639.

### Example 10: Synthesis of N-((2-(2,6-Dioxopiperidin-3-yl)-1-oxoisoindolin-5-yl)methyl)-6-trifluoromethoxy-2H-chromene-3-carboxamide (Compound A-10)

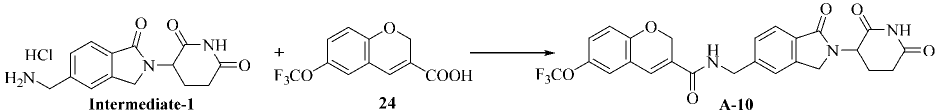

Synthesis of 6-trifluoromethoxy-2H-chromene-3-carboxylic acid (24): With reference to the synthesis method for intermediate-2, 5-trifluoromethoxysalicylaldehyde was used as the starting material to prepare the compound, with a two-step yield of 62.3%. HRMS (ESI⁻): calculated C₁₁H₆F₃O₄ (M - H)⁻, 259.0224; found 259.0228.

Synthesis of compound A-10: Through the synthesis method for compound A-1, 6-trifluoromethoxy-2H-chromene-3-carboxylic acid was used as the starting material to prepare A-10 (a white solid, 0.12 g, yield: 72.7%, purity: 99.89%). ¹H NMR (300 MHz, DMSO-*d*₆) *δ* (ppm) 11.01 (s, 1H), 8.96 (t, *J =* 6.0 Hz, 1H), 7.70 (d, *J* = 7.8 Hz, 1H), 7.53 (s, 1H), 7.45 (d, *J* = 7.8 Hz, 1H), 7.33 (s, 1H), 7.31 (d, *J =* 3.1 Hz, 1H), 7.28-7.20 (m, 1H), 6.96 (d, *J* = 8.8 Hz, 1H), 5.12 (dd, *J* = 13.3, 5.1 Hz, 1H), 5.00 (s, 2H), 4.56-4.25 (m, 4H), 3.00-2.81 (m, 1H), 2.60 (d, *J=* 17.6 Hz, 1H), 2.48-2.30 (m, 1H), 2.07-1.94 (m, 1H). HRMS (ESI⁺): calculated C₂₄H₂₁F₃N₃O₆ (M + H)⁺, 516.1377; found 516.1370.

### Example 11: Synthesis of N-((2-(2,6-Dioxopiperidin-3-yl)-1-oxoisoindolin-5-yl)methyl)-6-cyano-2H-chromene-3-carboxamide (Compound A-11)

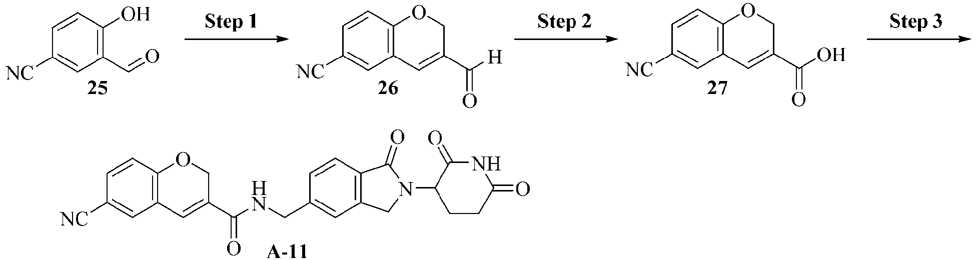

Step 1: 3-Formyl-4-hydroxybenzonitrile (25, 1.00 g, 6.80 mmol) was dissolved in 20 mL of 1,4-dioxane. Potassium carbonate (0.94 g, 6.80 mmol) and acrolein (0.76 g, 13.60 mmol) were added sequentially. After the addition, the mixture was heated at reflux and stirred for 5 hours. TLC monitoring showed that the reaction was complete. The reaction solution was poured into 20 mL of water and extracted with ethyl acetate. The organic phases were combined, washed with a 2 M sodium hydroxide solution and then with saturated brine, dried over anhydrous sodium sulfate, and then concentrated under vacuum. The residue was purified by silica gel column chromatography (petroleum ether:ethyl acetate = 10:1, v/v) to give compound 26, 3-formyl-2H-chromene-6-carbonitrile, as a yellow solid (1.03 g, yield: 82.0%). HRMS (ESI⁺): calculated C₁₁H₈NO₂ (M + H)⁺, 186.0550; found 186.0548.

Step 2: 3-Formyl-2H-chromene-6-carbonitrile (1.03 g, 5.57 mmol) was dissolved in 20 mL of tetrahydrofuran. A solution of sodium chlorite (5.03 g, 55.66 mmol) and sodium dihydrogen phosphate (6.68 g, 55.66 mmol) in 15 mL of water was added dropwise, and 2-methyl-2-butene (4.27 g, 60.81 mmol) was added. After the addition, the mixture was stirred at room temperature for 4 hours. TLC monitoring showed that the reaction was complete. The reaction solution was concentrated under vacuum and then diluted with 30 mL of water, and the aqueous layer was extracted with dichloromethane. The organic phases were combined, washed with saturated brine, dried over anhydrous sodium sulfate, and then concentrated under vacuum to give compound 27, 6-cyano-2H-chromene-3-carboxylic acid, as a yellow oil (0.90 g, yield: 80.0%). The product could be directly used in the next step without further purification. HRMS (ESI⁺): calculated C₁₁H₈NO₃ (M + H)⁺, 202.0499; found 202.0496.

Step 3: With reference to the synthesis method for compound A-1, 6-cyano-2H-chromene-3-carboxylic acid was used as the starting material to prepare A-11 (a white solid, 0.08 g, yield: 54.8%, purity: 96.95%). ¹H NMR (300 MHz, DMSO-*d*₆) *δ (ppm)* 11.01 (s, 1H), 8.92 (t, *J* = 6.0 Hz, 1H), 7.70 (d, *J =* 7.8 Hz, 1H), 7.52 (s, 1H), 7.42 (d, *J =* 7.3 Hz, 1H), 7.30 (s, 1H), 7.23 (t, *J =* 8.2 Hz, 1H), 6.96 (t, *J =* 7.4 Hz, 1H), 6.85 (d, *J* = 8.1 Hz, 1H), 5.12 (dd, *J* = 13.3, 5.1 Hz, 1H), 4.94 (s, 2H), 4.56-4.24 (m, 4H), 2.99-2.80 (m, 1H), 2.59 (d, *J =* 17.5 Hz, 1H), 2.47-2.28 (m, 1H), 2.06-1.95 (m, 1H). HRMS (ESI⁺): calculated C₂₅H₂₁N₄O₅ (M + H)⁺, 457.1507; found 457.1502.

### Example 12: Synthesis of N-((2-(2,6-Dioxopiperidin-3-yl)-1-oxoisoindolin-5-yl)methyl)-6-amino-2H-chromene-3-carboxamide (Compound A-12)

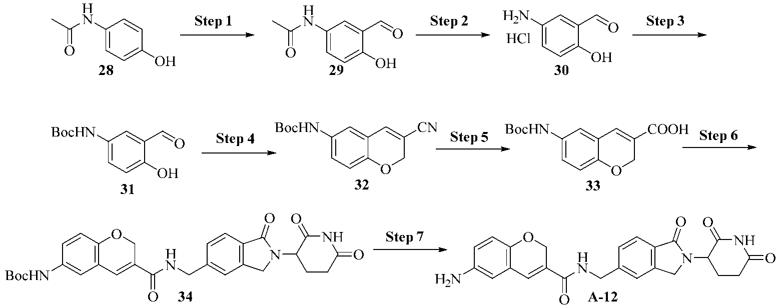

Step 1: N-Acetyl-p-aminophenol (28, 10.00 g, 66.20 mmol) was dissolved in 40 mL of trifluoroacetic acid. Hexamethylenetetramine (36.56 g, 0.26 mol) was added in batches to the reaction solution in an ice bath. After the addition, the mixture was heated to 70 °C and stirred for 5 hours. TLC monitoring showed that the reaction was complete. The reaction solution was cooled to room temperature, then poured into 200 mL of water, and extracted with ethyl acetate. The organic phases were combined, washed with saturated brine, dried over anhydrous sodium sulfate, and then concentrated under vacuum. The residue was purified by silica gel column chromatography (petroleum ether:ethyl acetate = 1:1, v/v) to give compound 29, N-(3-formyl-4-hydroxyphenyl)acetamide, as a white solid (2.03 g, yield: 17.1%). HRMS (ESI⁺): calculated C₉H₁₀NO₃ (M + H)⁺, 180.0655; found 180.0638.

Step 2: N-(3-Formyl-4-hydroxyphenyl)acetamide (2.03 g, 11.34 mmol) was dissolved in ethanol (30 mL) and dilute hydrochloric acid (6 M, 10 mL), and the solution was heated at reflux and stirred for 8 hours. TLC monitoring showed that the reaction was complete. The reaction solution was concentrated under vacuum and slurried with ethanol to give compound 30, 5-amino-2-hydroxybenzaldehyde hydrochloride, as a black solid (1.93 g, yield: 98.5%). HRMS (ESI⁺): calculated C₇H₈NO₂ (M + H)⁺, 138.0550; found 138.0545.

Step 3: 5-Amino-2-hydroxybenzaldehyde hydrochloride (1.93 g, 11.15 mmol) was dissolved in 30 mL of tetrahydrofuran. Triethylamine (1.13 g, 11.15 mmol) and di-tert-butyl dicarbonate (2.92 g, 13.39 mmol) were added sequentially. After the addition, the mixture was stirred at room temperature for 16 hours. TLC monitoring showed that the reaction was complete. The reaction solution was concentrated under vacuum, and the residue was purified by silica gel column chromatography (petroleum ether:ethyl acetate = 10:1, v/v) to give compound 31, tert-butyl (3-formyl-4-hydroxyphenyl)carbamate (2.61 g, yield: 98.9%), as a white solid. HRMS (ESI⁺): calculated C₁₂H₁₆NO₄ (M + H)⁺, 238.1074; found 238.1065.

Steps 4-6: With reference to the synthesis methods for intermediate-2 and compound A-1, tert-butyl (3-formyl-4-hydroxyphenyl)carbamate was used as the starting material to prepare compound 34: tert-butyl ((2-(2,6-dioxopiperidin-3-yl)-1-oxoisoindolin-5-yl)methyl)carbamoyl)-2H-chromen-6-yl)carbamate (a white solid, 1.21 g, yield: 20.1%). HRMS (ESI⁺): calculated C₂₀H₃₁N₂O₇ (M + H)⁺, 547.2187; found 547.2188.

Step 7: tert-Butyl ((2-(2,6-dioxopiperidin-3-yl)-1-oxoisoindolin-5-yl)methyl)carbamoyl)-2H-chromen-6-yl)carbamate (1.21 g, 2.22 mmol) was dissolved in 30 mL of a solution of hydrochloric acid in dioxane (4 M), and the solution was stirred at room temperature for 2 hours. TLC monitoring showed that the reaction was complete. The reaction solution was concentrated under vacuum, and the residue was then adjusted to be weakly alkaline with a saturated sodium bicarbonate solution and filtered under vacuum. The filter cake was washed with a small amount of petroleum ether and dried *in vacuo* to give A-12 as a white solid (0.99 g, yield: 95.0%, purity: 98.70%). ¹H NMR (300 MHz, DMSO-*d*₆) *δ* (ppm) 11.02 (s, 1H), 8.99 (t, *J* = 6.0 Hz, 1H), 7.69 (d, *J* = 7.8 Hz, 1H), 7.52 (s, 1H), 7.44 (d, *J* = 7.3 Hz, 1H), 7.31 (s, 1H), 7.24 (t, *J* = 8.1 Hz, 1H), 6.96 (t, *J* = 7.4 Hz, 1H), 6.85 (d, *J =* 8.1 Hz, 1H), 5.51 (s, 2H), 5.12 (dd, *J* = 13.3, 5.1 Hz, 1H), 4.93 (s, 2H), 4.50-4.24 (m, 4H), 2.99-2.81 (m, 1H), 2.61 (d*, J =* 17.5 Hz, 1H), 2.47-2.28 (m, 1H), 2.06-1.95 (m, 1H). HRMS (ESI⁺): calculated C₂₄H₂₃N₄O₅ (M + H)⁺, 447.1663; found 447.1660.

### Example 13: Synthesis of N-((2-(2,6-Dioxopiperidin-3-yl)-1-oxoisoindolin-5-yl)methyl)-6-(dimethylamino)-2H-chromene-3-carboxamide (Compound A-13)

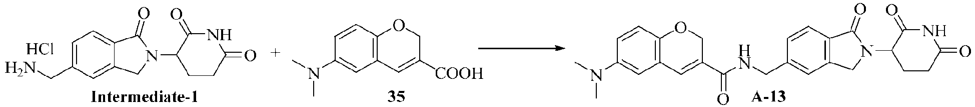

Synthesis of 6-(dimethylamino)-2H-chromene-3-carboxylic acid (35): With reference to the synthesis method for intermediate-2, 5-dimethylaminosalicylaldehyde was used as the starting material to prepare the compound, with a two-step yield of 48.6%. HRMS (ESI⁻): calculated C₁₂H₁₂NO₃ (M - H)⁻, 218.0823; found 218.0821.

Synthesis of compound A-13: With reference to the synthesis method for compound A-1, 6-(dimethylamino)-2H-chromene-3-carboxylic acid was used as the starting material to prepare A-13 (an off-white solid, 0.06 g, yield: 40.0%, purity: 97.13%). ¹H NMR (300 MHz, DMSO-*d*₆) *δ* (ppm) 11.02 (s, 1H), 8.99 (t, *J* = 6.0 Hz, 1H), 7.67 (d, *J =* 7.7 Hz, 1H), 7.56 (s, 1H), 7.45 (d, *J* = 7.3 Hz, 1H), 7.31 (d, *J =* 8.1,1H), 6.67-6.50 (m, 3H), 5.12 (dd, *J =* 13.3, 5.1 Hz, 1H), 4.93 (s, 2H), 4.50-4.24 (m, 4H), 3.12 (s, 6H), 2.98-2.80 (m, 1H), 2.60 (d, *J* = 17.5 Hz, 1H), 2.48-2.30 (m, 1H), 2.06-1.96 (m, 1H). HRMS (ESI⁺): calculated C₂₆H₂₇N₄O₅ (M + H)⁺, 475.1976; found 475.1972.

### Example 14: Synthesis of N-((2-(2,6-Dioxopiperidin-3-yl)-1-oxoisoindolin-5-yl)methyl)-6-vinyl-2H-chromene-3-carboxamide (Compound A-14)

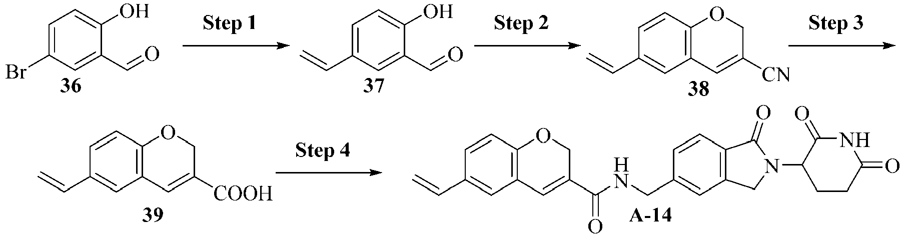

Step 1: 5-Bromo-2-hydroxybenzaldehyde (36, 1.50 g, 7.50 mmol) was dissolved in 20 mL of N,N-dimethylformamide. Lithium chloride (0.95 g, 22.51 mmol), tetrakis(triphenylphosphine)palladium (0.87 g, 0.75 mmol), and tributyl(vinyl)tin (2.85 g, 9.00 mmol) were added sequentially. After the addition, the mixture was heated to 95 °C and stirred for 4 hours. TLC monitoring showed that the reaction was complete. The reaction solution was poured into 100 mL of iced water, and a tan solid precipitated. The mixture was left to stand until the precipitation was complete, and filtration was performed under vacuum. The filter cake was purified by silica gel column chromatography (dichloromethane:methanol = 80:1, v/v) to give compound 37, 2-hydroxy-5-vinylbenzaldehyde, as a yellow solid (0.68 g, yield: 60.9%). HRMS (ESI⁺): calculated C₉H₉O₂ (M + H)⁺, 149.0597; found 149.0588.

Steps 2-4: With reference to the synthesis methods for intermediate-2 and compound A-1, 2-hydroxy-5-vinylbenzaldehyde was used as the starting material to prepare A-14 (a white solid, 0.07 g, yield: 50.5%, purity: 98.49%). ¹H NMR (300 MHz, DMSO-*d*₆) *δ* (ppm) 11.00 (s, 1H), 8.95 (t, *J =* 6.0 Hz, 1H), 7.70 (d, *J* = 7.8 Hz, 1H), 7.53 (s, 1H), 7.46 (d, *J* = 7.3 Hz, 1H), 7.33 (s, 1H), 7.25 (s, 1H), 6.96 (t, *J* = 7.4 Hz, 1H), 6.85 (d*, J =* 8.1 Hz, 1H), 6.61-6.45 (m, 1H), 5.70 (d*, J =* 6.8 Hz,1H), 5.50-5.26 (m,1H), 5.11 (dd, *J* = 13.3, 5.1 Hz, 1H), 4.94 (s, 2H), 4.56-4.24 (m, 4H), 3.01-2.81 (m, 1H), 2.60 (d, *J* = 17.5 Hz, 1H), 2.49-2.28 (m, 1H), 2.08-1.93 (m, 1H). HRMS (ESI⁺): calculated C₂₆H₂₄N₃O₅ (M + H)⁺, 458.1711; found 458.1702.

### Example 15: Synthesis of N-((2-(2,6-Dioxopiperidin-3-yl)-1-oxoisoindolin-5-yl)methyl)-6-ethynyl-2H-chromene-3-carboxamide (Compound A-15)

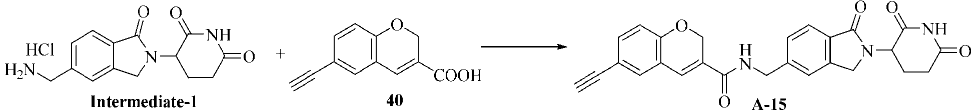

Synthesis of 6-(ethynyl)-2H-chromene-3-carboxylic acid (40): With reference to the synthesis method for intermediate-2, 5-ethynylsalicylaldehyde was used as the starting material to prepare the compound, with a two-step yield of 44.5%. HRMS (ESI⁻): calculated C₁₂H₇O₃ (M - H)⁻, 199.0401; found 199.0411.

Synthesis of compound A-15: With reference to the synthesis method for compound A-1, 6-(ethynyl)-2H-chromene-3-carboxylic acid was used as the starting material to prepare A-15 (a white solid, 0.08 g, yield: 54.7%, purity: 98.90%). ¹H NMR (300 MHz, DMSO-*d*₆) *δ* (ppm) 10.99 (s, 1H), 8.92 (t, *J =* 6.0 Hz, 1H), 7.72 (d, *J* = 7.8 Hz, 1H), 7.53 (s, 1H), 7.46 (d, *J* = 7.3 Hz, 1H), 7.33 (s, 1H), 7.25 (s, 1H), 6.99-6.92 (m, 1H), 6.85 (d, *J =* 8.1 Hz, 1H), 5.11 (dd, *J =* 13.3, 5.1 Hz, 1H), 4.94 (s, 2H), 4.56-4.24 (m, 4H), 4.15 (s, 1H), 2.99-2.84 (m, 1H), 2.60 (d, *J =* 17.5 Hz, 1H), 2.47-2.25 (m, 1H), 2.08-1.95 (m, 1H). HRMS (ESI⁺): calculated C₂₆H₂₂N₃O₅ (M + H)⁺, 456.1554; found 456.1547.

### Example 16: Synthesis of N-((2-(2,6-Dioxopiperidin-3-yl)-1-oxoisoindolin-5-yl)methyl)-6-tert-butyl-2H-chromene-3-carboxamide (Compound A-16)

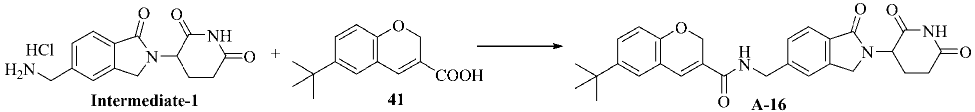

Synthesis of 6-(tert-butyl)-2H-chromene-3-carboxylic acid (41): With reference to the synthesis method for intermediate-2, 5-tert-butylsalicylaldehyde was used as the starting material to prepare the compound, with a two-step yield of 47.6%. HRMS (ESI⁻): calculated C₁₄H₁₅O₃ (M - H)⁻, 231.1027; found 231.1023.

Synthesis of compound A-16: With reference to the synthesis method for compound A-1, 6-(tert-butyl)-2H-chromene-3-carboxylic acid was used as the starting material to prepare A-16 (a white solid, 0.06 g, yield: 38.4%, purity: 97.69%). ¹H NMR (300 MHz, DMSO-*d*₆) *δ (ppm)* 11.03 (s, 1H), 8.90 (t, *J =* 6.0 Hz, 1H), 7.70 (d, *J =* 7.8 Hz, 1H), 7.53 (s, 1H), 7.45 (d, *J =* 7.5 Hz, 1H), 7.36 (s, 1H), 7.30-7.19 (m, 2H), 6.79 (d*, J =* 8.3 Hz, 1H), 5.12 (dd, *J* = 13.3, 5.0 Hz, 1H), 4.90 (s, 2H), 4.56-4.24 (m, 4H), 3.00-2.84 (m, 1H), 2.60 (d, *J* = 16.9 Hz, 1H), 2.48-2.31 (m, 1H), 2.06-1.93 (m, 1H), 1.25 (s, 9H). HRMS (ESI⁺): calculated C₂₈H₃₀N₃O₅ (M + H)⁺, 487.2107; found 487.2103.

### Example 17: Synthesis of N-((2-(2,6-Dioxopiperidin-3-yl)-1-oxoisoindolin-5-yl)methyl)-6-phenyl-2H-chromene-3-carboxamide (Compound A-17)

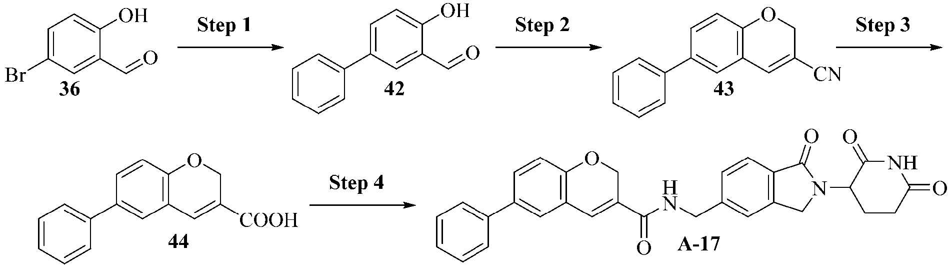

Step 1: 5-Bromo-2-hydroxybenzaldehyde (36, 1.80 g, 9.00 mmol) was dissolved in 30 mL of water. Phenylboronic acid (1.65 g, 13.50 mmol), potassium carbonate (2.49 g, 18.00 mmol), and tetrakis(triphenylphosphine)palladium (0.02 g, 0.02 mmol) were added sequentially. After the addition, the reaction solution was purged with nitrogen, then heated to 80 °C, and stirred for 6 hours. TLC monitoring showed that the reaction was complete. The reaction solution was filtered under vacuum, and the filter cake was washed with water and dried *in vacuo* to give compound 42, 4-hydroxy-[1,1'-biphenyl]-3-carbaldehyde, as an off-white solid (1.60 g, yield: 89.8%). HRMS (ESI⁺): calculated C₁₃H₁₁O₂ (M + H)⁺, 199.0754; found 199.0759.

Steps 2-4: With reference to the synthesis methods for intermediate-2 and compound A-1, 4-hydroxy-[1,1'-biphenyl]-3-carbaldehyde was used as the starting material to prepare A-17 (a white solid, 0.10 g, yield: 60.8%, purity: 98.56%). ¹H NMR (300 MHz, DMSO-*d*₆) *δ* (ppm) 10.99 (s, 1H), 8.90 (t, *J* = 6.0 Hz, 1H), 7.76-7.68 (m, 3H), 7.55-7.40 (m, 5H), 7.33 (s, 1H), 7.29-7.18 (m, 2H), 6.96-6.89 (m, 1H), 5.11 (dd, *J* = 13.3, 5.1 Hz, 1H), 4.85 (s, 2H), 4.55-4.27 (m, 4H), 2.99-2.81 (m, 1H), 2.60 (d, *J* = 17.4 Hz, 1H), 2.49-2.28 (m, 1H), 2.10 -1.95 (m, 1H). HRMS (ESI⁺): calculated C₃₀H₂₆N₃O₅ (M + H)⁺, 508.1867; found 508.1860.

### Example 18: Synthesis of N-((2-(2,6-Dioxopiperidin-3-yl)-1-oxoisoindolin-5-yl)methyl)-8-trifluoromethyl-2H-chromene-3-carboxamide (Compound A-18)

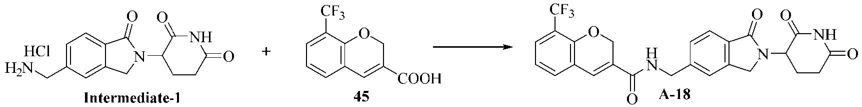

Synthesis of 8-trifluoromethyl-2H-chromene-3-carboxylic acid (45): With reference to the synthesis method for intermediate-2, 2-hydroxy-3-trifluoromethylbenzaldehyde was used as the starting material to prepare the compound, with a two-step yield of 50.6%. HRMS (ESI⁻): calculated C₁₁H₆F₃O₃ (M - H)⁻, 243.0275; found 243.0265.

Synthesis of compound A-18: With reference to the synthesis method for compound A-1, 8-trifluoromethyl-2H-chromene-3-carboxylic acid was used as the starting material to prepare A-18 (a white solid, 0.07 g, yield: 43.5%, purity: 97.20%). ¹H NMR (300 MHz, DMSO-*d*₆) *δ* (ppm) 11.04 (s, 1H), 9.02 (t, *J =* 6.0 Hz, 1H), 7.71 (d, *J =* 7.8 Hz, 1H), 7.61-7.51 (m, 3H), 7.46 (d, *J =* 7.9 Hz, 1H), 7.39 (s, 1H), 7.12 (t, *J* = 7.7 Hz, 1H), 5.17-5.10 (m, 1H), 5.09 (s, 2H), 4.56-4.24 (m, 4H), 3.00-2.84 (m, 1H), 2.60 (d, *J =* 16.9 Hz, 1H), 2.48-2.30 (m, 1H), 2.05-1.94 (m, 1H). HRMS (ESI⁺): calculated C₂₅H₂₁F₃N₃O₅ (M + H)⁺, 500.1428; found 500.1422.

### Example 19: Synthesis of N-((2-(2,6-Dioxopiperidin-3-yl)-1-oxoisoindolin-5-yl)methyl)-8-trifluoromethoxy-2H-chromene-3-carboxamide (Compound A-19)

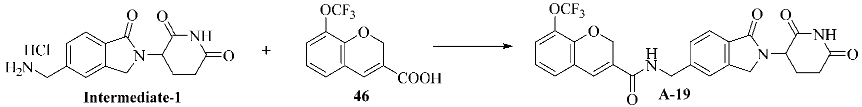

Synthesis of 8-trifluoromethoxy-2H-chromene-3-carboxylic acid (46): With reference to the synthesis method for intermediate-2, 2-hydroxy-3-trifluoromethoxybenzaldehyde was used as the starting material to prepare the compound, with a two-step yield of 48.2%. HRMS (ESI⁻): calculated C₁₁H₆F₃O₄ (M - H)⁻, 259.0224; found 259.0220.

Synthesis of compound A-19: With reference to the synthesis method for compound A-1, 8-trifluoromethoxy-2H-chromene-3-carboxylic acid was used as the starting material to prepare A-19 (a white solid, 0.06 g, yield: 35.9%, purity: 98.56%). ¹H NMR (300 MHz, DMSO-*d*₆) *δ* (ppm) 11.03 (s, 1H), 9.02 (t, *J =* 6.0 Hz, 1H), 7.70 (d, *J =* 7.8 Hz, 1H), 7.53 (s, 1H), 7.46 (d, *J =* 7.9 Hz, 1H), 7.39 (s, 1H), 7.36-7.25 (m, 2H), 7.04 (t, *J =* 7.9 Hz, 1H), 5.12 (dd, *J =* 13.2, 5.0 Hz, 1H), 5.04 (s, 2H), 4.58-4.24 (m, 4H), 3.01-2.84 (m, 1H), 2.60 (d, *J =* 16.9 Hz, 1H), 2.47-2.29 (m, 1H), 2.06-1.94 (m, 1H). HRMS (ESI⁺): calculated C₂₅H₂₁F₃N₃O₆ (M + H)⁺, 515.1406; found 515.1402.

### Example 20: Synthesis of N-((2-(2,6-Dioxopiperidin-3-yl)-1-oxoisoindolin-5-yl)methyl)-8-methoxy-2H-chromene-3-carboxamide (Compound A-20)

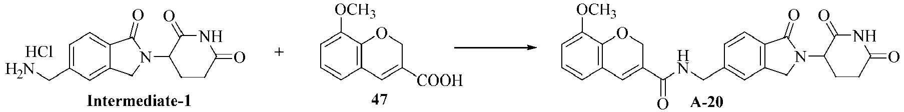

Synthesis of 8-methoxy-2H-chromene-3-carboxylic acid (47): With reference to the synthesis method for intermediate-2, 2-hydroxy-3-methoxybenzaldehyde was used as the starting material to prepare the compound, with a two-step yield of 56.5%. HRMS (ESI⁻): calculated C₁₁H₉O₄ (M - H)⁻, 205.0506; found 205.0500.

Synthesis of compound A-20: With reference to the synthesis method for compound A-1, 8-methoxy-2H-chromene-3-carboxylic acid was used as the starting material to prepare A-20 (a white solid, 0.07 g, yield: 50.0%, purity: 98.56%). ¹H NMR (300 MHz, DMSO-*d*₆) *δ* (ppm) 11.03 (s, 1H), 8.94 (t, *J =* 6.0 Hz, 1H), 7.70 (d, *J =* 7.8 Hz, 1H), 7.52 (s, 1H), 7.45 (d, *J* = 7.8 Hz, 1H), 7.32 (s, 1H), 6.99 (d, *J =* 6.5 Hz, 1H), 6.91 (t, *J* = 7.7 Hz, 1H), 6.83 (d, *J =* 7.5 Hz, 1H), 5.12 (dd, *J =* 13.4, 5.0 Hz, 1H), 4.91 (s, 2H), 4.57-4.23 (m, 4H), 3.76 (s, 3H), 3.02-2.82 (m, 1H), 2.60 (d, *J =* 16.8 Hz, 1H), 2.44-2.29 (m, 1H), 2.06-1.88 (m, 1H). HRMS (ESI⁺): calculated C₂₅H₂₄N₃O₆ (M + H)⁺, 462.5943; found 462.5938.

### Example 21: Synthesis of N-((2-(2,6-Dioxopiperidin-3-yl)-1-oxoisoindolin-5-yl)methyl)-8-methyl-2H-chromene-3-carboxamide (Compound A-21)

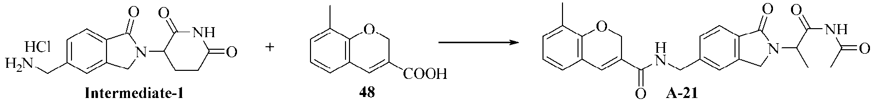

Synthesis of 8-methyl-2H-chromene-3-carboxylic acid (48): With reference to the synthesis method for intermediate-2, 2-hydroxy-3-methylbenzaldehyde was used as the starting material to prepare the compound, with a two-step yield of 60.2%. HRMS (ESI⁻): calculated C₁₁H₉O₃ (M - H)⁻, 189.0557; found 189.0545.

Synthesis of compound A-21: With reference to the synthesis method for compound A-1, 8-methyl-2H-chromene-3-carboxylic acid was used as the starting material to prepare A-21 (a white solid, 0.07 g, yield: 48.6%, purity: 98.26%). ¹H NMR (300 MHz, DMSO-*d*₆) *δ* (ppm) 1.00 (s, 1H), 8.89 (t, *J* = 6.0 Hz, 1H), 7.70 (d, *J =* 7.8 Hz, 1H), 7.49 (s, 1H), 7.44 (d, *J =* 7.8 Hz, 1H), 7.29 (s, 1H), 7.04 (d, *J =* 8.1 Hz, 2H), 6.75 (d, *J =* 7.9 Hz, 1H), 5.11 (dd, *J =* 13.2, 5.0 Hz, 1H), 4.90 (s, 2H), 4.56-4.24 (m, 4H), 2.99-2.83 (m, 1H), 2.60 (d, *J =* 17.0 Hz, 1H), 2.48-2.31 (m, 1H), 2.19 (s, 3H), 2.07-1.93 (m, 1H). HRMS (ESI⁺): calculated C₂₅H₂₄N₃O₅ (M + H)⁺, 446.1711; found 446.1707.

### Example 22: Synthesis of N-((2-(2,6-Dioxopiperidin-3-yl)-1-oxoisoindolin-5-yl)methyl)-5-methyl-2H-chromene-3-carboxamide (Compound A-22)

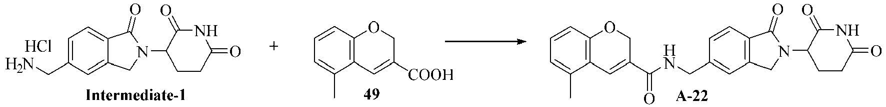

Synthesis of 5-methyl-2H-chromene-3-carboxylic acid (49): With reference to the synthesis method for intermediate-2, 2-hydroxy-6-methylbenzaldehyde was used as the starting material to prepare the compound, with a two-step yield of 60.6%. HRMS (ESI⁻): calculated C₁₁H₉O₃ (M - H)⁻, 189.0557; found 189.0546.

Synthesis of compound A-22: Through the synthesis method for compound A-1, 5-methyl-2H-chromene-3-carboxylic acid was used as the starting material to prepare A-22 (a white solid, 0.09 g, yield: 62.5%, purity: 97.88%). ¹H NMR (300 MHz, DMSO-*d*₆) *δ (ppm)* 11.00 (s, 1H), 8.89 (t, *J =* 6.0 Hz, 1H), 7.70 (d, *J* = 7.8 Hz, 1H), 7.50 (s, 1H), 7.35 (d, *J =* 7.8 Hz, 1H), 7.16 (s, 1H), 7.04 (d, *J =* 8.1 Hz, 2H), 6.75 (d, *J =* 7.9 Hz, 1H), 5.13 (dd, *J =* 13.2, 5.0 Hz, 1H), 4.92 (s, 2H), 4.56-4.24 (m, 4H), 2.99-2.84 (m, 1H), 2.59 (d, *J =* 17.0 Hz, 1H), 2.47-2.30 (m, 1H), 2.12 (s, 3H), 2.05-1.90 (m, 1H). HRMS (ESI⁺): calculated C₂₅H₂₄N₃O₅ (M + H)⁺, 446.1711; found 446.1709.

### Example 23: Synthesis of N-((2-(2,6-Dioxopiperidin-3-yl)-1-oxoisoindolin-5-yl)methyl)-6-(acetamido)-2H-chromene-3-carboxamide (Compound A-23)

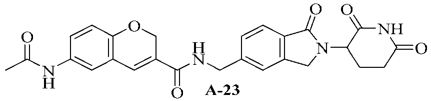

N-((2-(2,6-Dioxopiperidin-3-yl)-1-oxoisoindolin-5-yl)methyl)-6-amino-2H-chromene-3-carboxamide (compound 12, 0.10 g, 0.22 mmol) was dissolved in 5 mL of acetonitrile. Triethylamine (0.03 g, 0.34 mmol) and acetyl chloride (0.02 g, 0.25 mmol) were added sequentially. After the addition, the mixture was stirred at room temperature for 1 hour. TLC monitoring showed that the reaction was complete. The reaction solution was concentrated under vacuum, and the residue was purified by silica gel column chromatography (dichloromethane:methanol = 50:1, v/v) to give A-23 as a white solid (0.04 g, yield: 36.7%, purity: 98.20%). ¹H NMR (300 MHz, DMSO-*d*₆) *δ* (ppm) 11.02 (s, 1H), 10.22 (s, 1H), 8.98 (t, *J* = 6.0 Hz, 1H), 7.67 (d, *J =* 7.8 Hz, 1H), 7.56 (s, 1H), 7.45 (d, *J =* 7.3 Hz, 1H), 7.31 (d, *J* = 8.1,1H), 7.10-6.89 (m, 3H), 5.11 (dd, *J =* 13.3, 5.1 Hz, 1H), 4.88 (s, 2H), 4.48-4.24 (m, 4H), 2.97-2.80 (m, 1H), 2.58 (d, *J =* 17.5 Hz, 1H), 2.48-2.32 (m, 1H), 2.13 (s, 3H), 2.07-1.96 (m, 1H). HRMS (ESI⁺): calculated C₂₆H₂₅N₄O₆ (M + H)⁺, 489.1769; found 489.1760.

### Example 24: Synthesis of N-((2-(2,6-Dioxopiperidin-3-yl)-1-oxoisoindolin-5-yl)methyl)-6-(acrylamido)-2H-chromene-3-carboxamide (Compound A-24)

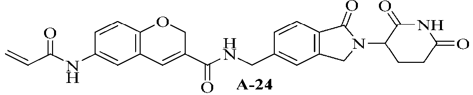

With reference to the synthesis method for compound A-15, acryloyl chloride was used as the starting material to prepare A-24 (a white solid, 0.05 g, yield: 44.6%, purity: 95.96%). ¹H NMR (300 MHz, DMSO-*d*₆) *δ* (ppm) 11.00 (s, 1H), 9.54 (s, 1H), 8.90 (t, *J* = 6.0 Hz, 1H), 7.67 (d, *J =* 7.8 Hz, 1H), 7.56 (s, 1H), 7.45 (d, *J =* 7.3 Hz, 1H), 7.33 (s, 1H), 7.30-7.22 (m, 2H), 6.99 (d, *J =* 8.1 Hz, 1H), 6.50-6.44 (m, 1H), 6.08-6.00 (m, 2H), 5.11 (dd, *J* = 13.3, 5.1 Hz, 1H), 4.81 (s, 2H), 4.48-4.24 (m, 4H), 2.97-2.80 (m, 1H), 2.58 (d*, J =* 17.5 Hz, 1H), 2.48-2.32 (m, 1H), 2.07-1.96 (m, 1H).

HRMS (ESI⁺): calculated C₂₇H₂₅N₄O₆ (M + H)⁺, 501.1769; found 501.1757.

### Example 25: Synthesis of N-((2-(2,6-Dioxopiperidin-3-yl)-1-oxoisoindolin-5-yl)methyl)-6-(2-hydroxyacetamido)-2H-chromene-3-carboxamide (Compound A-25)

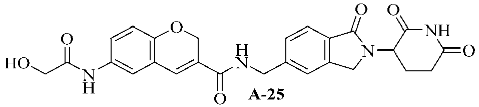

N-((2-(2,6-Dioxopiperidin-3-yl)-1-oxoisoindolin-5-yl)methyl)-6-amino-2H-chromene-3-carboxamide (compound 12, 0.07 g, 0.16 mmol) was dissolved in 5 mL of N,N-dimethylformamide. Hydroxyacetic acid (0.01 g, 0.19 mmol) and HATU (0.09 g, 0.24 mmol) were added sequentially. After the addition, the mixture was stirred at room temperature for 1 hour. DIPEA (0.06 g, 0.47 mmol) was added, and the mixture was stirred overnight at room temperature. TLC monitoring showed that the reaction was complete. The reaction solution was poured into 30 mL of iced water and extracted with ethyl acetate. The organic phases were combined, washed with water and then with saturated brine, dried over anhydrous sodium sulfate, and then concentrated under vacuum. The residue was purified by silica gel column chromatography (dichloromethane:methanol = 50:1, v/v) to give A-25 as a pale yellow solid (0.03 g, yield: 43.0%, purity: 97.48%). ¹H NMR (300 MHz, DMSO-*d*₆) *δ* (ppm) 11.00 (s, 1H), 9.54 (s, 1H), 8.90 (t, *J =* 6.0 Hz, 1H), 7.67 (d, *J* = 7.8 Hz, 1H), 7.57 (s, 1H), 7.45 (d, *J =* 7.3 Hz, 1H), 7.33 (s, 1H), 7.28 (d, *J* = 8.0 Hz, 1H), 7.00 (d, *J* = 8.1 Hz, 1H), 6.88 (d, *J* = 7.9 Hz, 1H), 5.15 (s, 1H), 5.07 (dd, *J* = 13.3, 5.1 Hz, 1H), 4.79 (s, 2H), 4.55-4.29 (m, 6H), 2.97-2.80 (m, 1H), 2.61 (d, *J* = 17.5 Hz, 1H), 2.47-2.30 (m, 1H), 2.07-1.96 (m, 1H). HRMS (ESI⁺): calculated C₂₆H₂₅N₄O₇ (M + H)⁺, 505.1718; found 505.1712.

### Example 26: Synthesis of N-((2-(2,6-Dioxopiperidin-3-yl)-1-oxoisoindolin-5-yl)methyl)-6-(cyclopropanecarboxamido)-2H-chromene-3-carboxamide (Compound A-26)

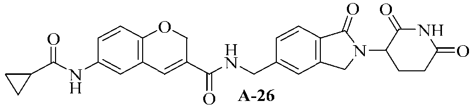

With reference to the synthesis method for compound A-15, cyclopropanecarbonyl chloride was used as the starting material to prepare A-26 (a white solid, 0.05 g, yield: 43.5%, purity: 96.40%). ¹H NMR (300 MHz, DMSO-*d*₆) *δ (ppm)* 11.00 (s, 1H), 9.99 (s, 1H), 8.99 (t, *J =* 6.0 Hz, 1H), 7.70 (d, *J =* 7.8 Hz, 1H), 7.59 (s, 1H), 7.47 (d, *J =* 7.3 Hz, 1H), 7.35 (s, 1H), 7.25 (d, *J =* 8.0 Hz, 1H), 6.99-6.88 (m, 2H), 5.02 (dd, *J =* 13.3, 5.1 Hz, 1H), 4.80 (s, 2H), 4.55-4.30 (m, 4H), 2.97-2.81 (m, 1H), 2.59 (d, *J =* 17.6 Hz, 1H), 2.47-2.31 (m, 1H), 2.07-1.99 (m, 1H), 1.49-1.45 (m, 1H), 0.80-0.61 (m, 4H). HRMS (ESI⁺): calculated C₂₈H₂₇N₄O₆ (M + H)⁺, 515.1925; found 515.1918.

### Example 27: Synthesis of N-((2-(2,6-Dioxopiperidin-3-yl)-1-oxoisoindolin-5-yl)methyl)-6-(cyclobutanecarboxamido)-2H-chromene-3-carboxamide (Compound A-27)

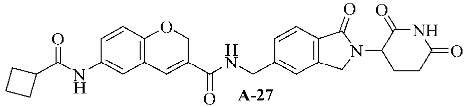

With reference to the synthesis method for compound A-15, cyclobutanecarbonyl chloride was used as the starting material to prepare A-27 (a white solid, 0.06 g, yield: 50.8%, purity: 97.68%). ¹H NMR (300 MHz, DMSO-*d*₆) *δ (ppm)* 11.01 (s, 1H), 9.89 (s, 1H), 8.98 (t, *J =* 6.0 Hz, 1H), 7.68 (d, *J =* 7.8 Hz, 1H), 7.55 (s, 1H), 7.44 (d, *J =* 7.3 Hz, 1H), 7.35 (s, 1H), 7.25 (d, *J =* 8.0 Hz, 1H), 7.01 (d, *J* = 8.2 Hz, 1H), 6.90 (d, *J =* 7.9 Hz, 1H), 5.04 (dd, *J =* 13.3, 5.1 Hz, 1H), 4.80 (s, 2H), 4.52-4.29 (m, 4H), 2.97-2.82 (m, 1H), 2.59 (d*, J =* 17.6 Hz, 1H), 2.47-2.31 (m, 1H), 2.07-1.85 (m, 4H), 1.78-1.53 (m, 4H). HRMS (ESI⁺): calculated C₂₉H₂₉N₄O₆ (M + H)⁺, 529.2082; found 529.2077.

### Example 28: Synthesis of N-(2-(2,6-Dioxopiperidin-3-yl)-1-oxoisoindolin-5-yl)methyl)-6-chloro-7-methyl-2H-chromene-3-carboxamide (Compound A-28)

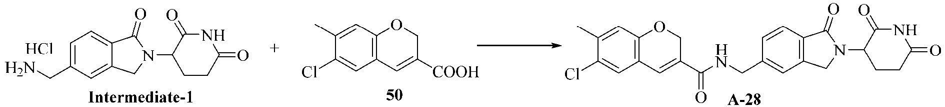

Synthesis of 6-chloro-7-methyl-2H-chromene-3-carboxylic acid (50): With reference to the synthesis method for intermediate-2, 5-chloro-4-methylsalicylaldehyde was used as the starting material to prepare the compound, with a two-step yield of 54.6%. HRMS (ESI⁻): calculated C₁₁H₈ClO₃ (M - H)⁻, 223.0167; found 223.0158.

Synthesis of compound A-28: With reference to the synthesis method for compound A-1, 6-chloro-7-methyl-2H-chromene-3-carboxylic acid was used as the starting material to prepare A-28 (a white solid, 0.08 g, yield: 52.2%, purity: 97.43%). ¹H NMR (300 MHz, DMSO-*d*₆) *δ* (ppm) 11.00 (s, 1H), 8.89 (t, *J =* 6.0 Hz, 1H), 7.69 (d, *J =* 7.8 Hz, 1H), 7.52 (s, 1H), 7.44 (d, *J =* 7.9 Hz, 1H), 7.28 (d, *J =* 5.9 Hz, 2H), 6.88 (s, 1H), 5.11 (dd, *J =* 13.2, 5.0 Hz, 1H), 4.94 (s, 2H), 4.55-4.25 (m, 4H), 3.03-2.85 (m, 1H), 2.60 (d, *J =* 17.8 Hz, 1H), 2.46-2.33 (m, 1H), 2.27 (s, 3H), 2.07-1.96 (m, 1H). HRMS (ESI⁺): calculated C₂₅H₂₃ClN₃O₅ (M + H)⁺, 480.1321; found 480.1309.

### Example 29: Synthesis of N-((2-(2,6-Dioxopiperidin-3-yl)-1-oxoisoindolin-5-yl)methyl)-7-chloro-6-methyl-2H-chromene-3-carboxamide (Compound A-29)

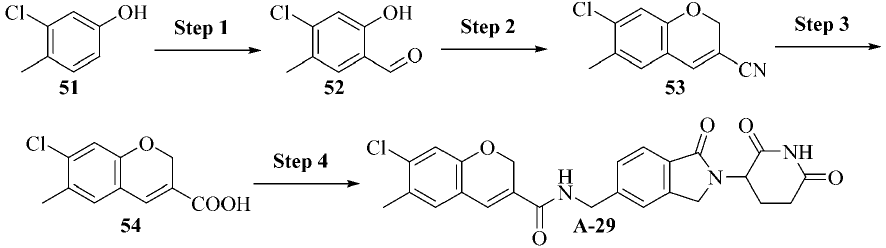

Step 1: 3-Chloro-4-methyl-phenol (51, 1.00 g, 7.04 mmol) was dissolved in 20 mL of acetonitrile. Formaldehyde (1.11 g, 36.99 mmol), triethylamine (3.74 g, 36.99 mmol), and magnesium chloride (2.68 g, 28.16 mmol) were added sequentially. After the addition, the mixture was heated to 80 °C and stirred for 15 hours. TLC monitoring showed that the reaction was complete. 50 mL of hydrochloric acid (1 M) was added to quench the reaction, and extraction was performed with ethyl acetate. The organic phases were combined, washed with saturated brine, dried over anhydrous sodium sulfate, and then concentrated under vacuum to give compound 52, 4-chloro-2-hydroxy-5-methylbenzaldehyde, as a brown oil (1.02 g, yield: 84.9%). The product could be directly used in the next step without further purification. HRMS (ESI⁺): calculated C₈H₈ClO₂ (M + H)⁺, 171.0207; found 171.0217.

Steps 2-4: With reference to the synthesis methods for intermediate-2 and compound A-1, 4-chloro-2-hydroxy-5-methylbenzaldehyde was used as the starting material to prepare A-29 (a white solid, 0.03 g, yield: 19.4%, purity: 98.23%). ¹H NMR (300 MHz, DMSO-*d*₆) *δ* (ppm) 11.00 (s, 1H), 8.89 (t, *J =* 6.0 Hz, 1H), 7.69 (d, *J =* 7.8 Hz, 1H), 7.52 (s, 1H), 7.44 (d, *J =* 7.9 Hz, 1H), 7.23 (d, *J =* 5.9 Hz, 2H), 6.94 (s, 1H), 5.11 (dd, *J* = 13.2, 5.0 Hz, 1H), 4.94 (s, 2H), 4.53-4.25 (m, 4H), 3.00-2.85 (m, 1H), 2.60 (d, *J* = 17.8 Hz, 1H), 2.47-2.35 (m, 1H), 2.22 (s, 3H), 2.07-1.96 (m, 1H). HRMS (ESI⁺): calculated C₂₅H₂₃ClN₃O₅ (M + H)⁺, 480.1321; found 480.1318.

### Example 30: Synthesis of N-((2-(2,6-Dioxopiperidin-3-yl)-1-oxoisoindolin-5-yl)methyl)-7-fluoro-6-methyl-2H-chromene-3-carboxamide (Compound A-30)

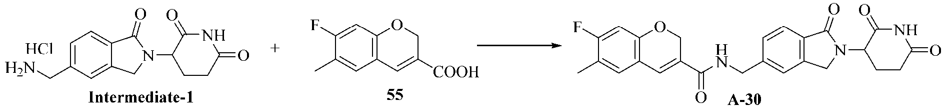

Synthesis of 7-fluoro-6-methyl-2H-chromene-3-carboxylic acid (55): With reference to the synthesis method for intermediate-2, 4-fluoro-5-methylsalicylaldehyde was used as the starting material to prepare the compound, with a two-step yield of 51.2%. HRMS (ESI⁻): calculated C₁₁H₈FO₃ (M - H)⁻, 207.0463; found 207.0458.

Synthesis of compound A-30: Through the synthesis method for compound A-1, 7-fluoro-6-methyl-2H-chromene-3-carboxylic acid was used as the starting material to prepare A-30 (a white solid, 0.07 g, yield: 47.3%, purity: 98.32%). ¹H NMR (300 MHz, DMSO-*d*₆) *δ (ppm)* 11.01 (s, 1H), 8.92 (t, *J =* 6.0 Hz, 1H), 7.69 (d, *J* = 7.8 Hz, 1H), 7.50 (s, 1H), 7.40 (d, *J* = 7.5 Hz, 1H), 7.31 (d, *J=* 5.9 Hz, 2H), 6.96 (s, 1H), 5.11 (dd, *J* = 13.2, 5.0 Hz, 1H), 4.94 (s, 2H), 4.55-4.25 (m, 4H), 3.03-2.85 (m, 1H), 2.59 (d, *J* = 17.5 Hz, 1H), 2.48-2.35 (m, 1H), 2.16 (s, 3H), 2.08-1.95 (m, 1H). HRMS (ESI⁺): calculated C₂₅H₂₃FN₃O₅ (M + H)⁺, 464.1616; found 464.1612.

### Example 31: Synthesis of N-((2-(2,6-Dioxopiperidin-3-yl)-1-oxoisoindolin-5-yl)methyl)-6-fluoro-8-methyl-2H-chromene-3-carboxamide (Compound A-31)

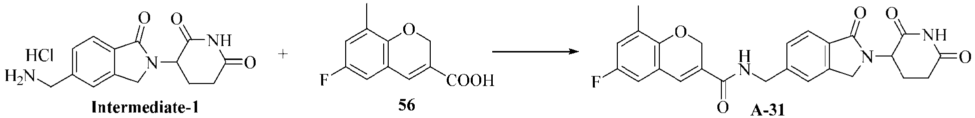

Synthesis of 6-fluoro-8-methyl-2H-chromene-3-carboxylic acid (56): With reference to the synthesis method for intermediate-2, 5-fluoro-3-methylsalicylaldehyde was used as the starting material to prepare the compound, with a two-step yield of 57.3%. HRMS (ESI⁻): calculated C₁₁H₈FO₃ (M - H)⁻, 207.0463; found 207.0465.

Synthesis of compound A-31: Through the synthesis method for compound A-1, 5-fluoro-3-methyl-2H-chromene-3-carboxylic acid was used as the starting material to prepare A-31 (a white solid, 0.08 g, yield: 54.0%, purity: 99.82%). ¹H NMR (300 MHz, DMSO-*d*₆) *δ (ppm)* 11.00 (s, 1H), 8.89 (t, *J*= 6.0 Hz, 1H), 7.69 (d, *J =* 7.8 Hz, 1H), 7.55 (s, 1H), 7.46 (d, *J =* 7.9 Hz, 1H), 7.32 (d, *J =* 5.9 Hz, 2H), 6.99 (s, 1H), 5.12 (dd, *J* = 13.2, 5.0 Hz, 1H), 4.94 (s, 2H), 4.55-4.25 (m, 4H), 3.00-2.85 (m, 1H), 2.60 (d, *J =* 17.8 Hz, 1H), 2.45-2.30 (m, 1H), 2.20 (s, 3H), 2.07-1.95 (m, 1H). HRMS (ESI⁺): calculated C₂₅H₂₃FN₃O₅ (M + H)⁺, 464.1616; found 464.1604.

### Example 32: Synthesis of N-((2-(2,6-Dioxopiperidin-3-yl)-1-oxoisoindolin-5-yl)methyl)-7,8-difluoro-2H-chromene-3-carboxamide (Compound A-32)

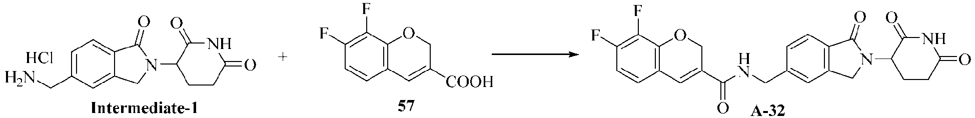

Synthesis of 7,8-difluoro-2H-chromene-3-carboxylic acid (57): With reference to the synthesis method for intermediate-2, 3,4-difluorosalicylaldehyde was used as the starting material to prepare the compound, with a two-step yield of 52.4%. HRMS (ESI⁻): calculated C₁₀H₅F₂O₃ (M - H)⁻, 211.0212; found 211.0215.

Synthesis of compound A-32: Through the synthesis method for compound A-1, 7,8-difluoro-2H-chromene-3-carboxylic acid was used as the starting material to prepare A-32 (a white solid, 0.06 g, yield: 40.2%, purity: 98.32%). ¹H NMR (300 MHz, DMSO-*d*₆) *δ* (ppm) 11.00 (s, 1H), 8.95 (s, 1H), 7.70 (d, *J =* 7.8 Hz, 1H), 7.53 (s, 1H), 7.40 (d, *J =* 8.1 Hz, 1H), 7.29 (d, *J* = 8.9 Hz, 2H), 7.15 (d, *J =* 8.4 Hz, 1H), 5.11 (dd, *J* = 13.2, 5.1 Hz, 1H), 5.00 (s, 2H), 4.56-4.24 (m, 4H), 3.04-2.79 (m, 1H), 2.60 (d, *J* = 16.9 Hz, 1H), 2.47-2.33 (m, 1H), 2.07-1.93 (m, 1H). HRMS (ESI⁺): calculated C₂₄H₂₀F₂N₃O₅ (M + H)⁺, 468.1366; found 468.1361.

### Example 33: Synthesis of N-((2-(2,6-Dioxopiperidin-3-yl)-1-oxoisoindolin-5-yl)methyl)-6,8-difluoro-2H-chromene-3-carboxamide (Compound A-33)

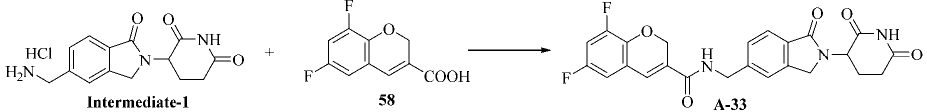

Synthesis of 6,8-difluoro-2H-chromene-3-carboxylic acid (58): With reference to the synthesis method for intermediate-2, 3,5-difluorosalicylaldehyde was used as the starting material to prepare the compound, with a two-step yield of 52.4%. HRMS (ESI⁻): calculated C₁₀H₅F₂O₃ (M - H)⁻, 211.0212; found 211.0220.

Synthesis of compound A-33: With reference to the synthesis method for compound A-1, 6,8-difluoro-2H-chromene-3-carboxylic acid was used as the starting material to prepare A-33 (a white solid, 0.07 g, yield: 46.9%, purity: 97.14%). ¹H NMR (300 MHz, DMSO-*d*₆) *δ* (ppm) 11.01 (s, 1H), 9.02 (s, 1H), 7.70 (d*, J =* 7.8 Hz, 1H), 7.53 (s, 1H), 7.45 (d, *J =* 8.1 Hz, 1H), 7.30 (d*, J =* 8.9 Hz, 2H), 7.05 (d*, J =* 8.4 Hz, 1H), 5.11 (dd, *J* = 13.2, 5.1 Hz, 1H), 5.01 (s, 2H), 4.56-4.23 (m, 4H), 3.04-2.79 (m, 1H), 2.60 (d, *J* = 16.9 Hz, 1H), 2.44-2.33 (m, 1H), 2.07-1.90 (m, 1H). HRMS (ESI⁺): calculated C₂₄H₂₀F₂N₃O₅ (M + H)⁺, 468.1366; found 468.1362.

### Example 34: Synthesis of N-((2-(2,6-Dioxopiperidin-3-yl)-1-oxoisoindolin-5-yl)methyl)-5,7-difluoro-2H-chromene-3-carboxamide (Compound A-34)

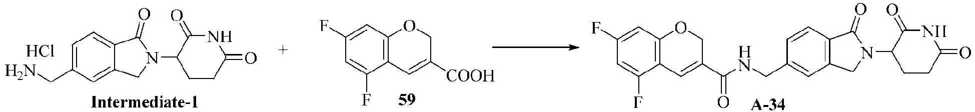

Synthesis of 5,7-difluoro-2H-chromene-3-carboxylic acid (59): With reference to the synthesis method for intermediate-2, 2,4-difluorosalicylaldehyde was used as the starting material to prepare the compound, with a two-step yield of 54.8%. HRMS (ESI⁻): calculated C₁₀H₅F₂O₃ (M - H)⁻, 211.0212; found 211.0217.

Synthesis of compound A-34: Through the synthesis method for compound A-1, 5,7-difluoro-2H-chromene-3-carboxylic acid was used as the starting material to prepare A-34 (a white solid, 0.08 g, yield: 53.6%, purity: 97.92%). ¹H NMR (300 MHz, DMSO-*d*₆) *δ* (ppm) 11.00 (s, 1H), 8.96 (t, *J =* 6.0 Hz, 1H), 7.70 (d, *J* = 7.8 Hz, 1H), 7.55 (s, 1H), 7.40-7.31 (m, 2H), 7.22 (s, 1H), 6.98 (dd, *J* = 11.5, 6.8 Hz, 1H), 5.11 (dd, *J =* 13.3, 5.0 Hz, 1H), 4.97 (s, 2H), 4.57-4.26 (m, 4H), 3.00-2.83 (m, 1H), 2.59 (d, *J =* 17.2 Hz, 1H), 2.47-2.32 (m, 1H), 2.07-1.93 (m, 1H). HRMS (ESI⁺): calculated C₂₄H₂₀F₂N₃O₅ (M + H)⁺, 468.1366; found 468.1357.

### Example 35: Synthesis of N-((2-(2,6-Dioxopiperidin-3-yl)-1-oxoisoindolin-5-yl)methyl)-6,7-difluoro-2H-chromene-3-carboxamide (Compound A-35)

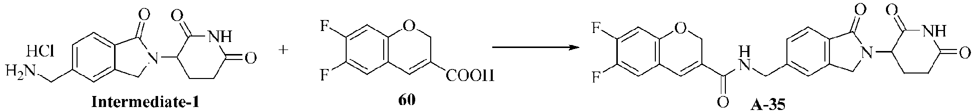

Synthesis of 6,7-difluoro-2H-chromene-3-carboxylic acid (60): With reference to the synthesis method for intermediate-2, 4,5-difluorosalicylaldehyde was used as the starting material to prepare the compound, with a two-step yield of 54.1%. HRMS (ESI⁻): calculated C₁₀H₅F₂O₃ (M - H)⁻, 211.0212; found 211.0223.

Synthesis of compound A-35: Through the synthesis method for compound A-1, 6,7-difluoro-2H-chromene-3-carboxylic acid was used as the starting material to prepare A-35 (a white solid, 0.05 g, yield: 33.5%, purity: 97.09%). ¹H NMR (300 MHz, DMSO-*d*₆) *δ* (ppm) 11.00 (s, 1H), 8.93 (t, *J =* 6.0 Hz, 1H), 7.70 (d, *J* = 7.8 Hz, 1H), 7.52 (s, 1H), 7.47-7.35 (m, 2H), 7.27 (s, 1H), 7.04 (dd, *J =* 11.7, 6.9 Hz, 1H), 5.11 (dd, *J =* 13.3, 5.0 Hz, 1H), 4.97 (s, 2H), 4.57-4.24 (m, 4H), 3.00-2.81 (m, 1H), 2.60 (d, *J* = 17.2 Hz, 1H), 2.47-2.30 (m, 1H), 2.07-1.91 (m, 1H). HRMS (ESI⁺): calculated C₂₄H₂₀F₂N₃O₅ (M + H)⁺, 468.1366; found 468.1359.

### Example 36: Synthesis of N-((2-(2,6-Dioxopiperidin-3-yl)-1-oxoisoindolin-5-yl)methyl)-5,8-difluoro-2H-chromene-3-carboxamide (Compound A-36)

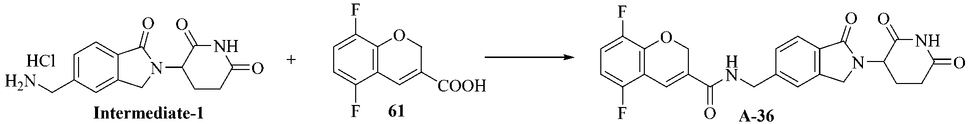

Synthesis of 5,8-difluoro-2H-chromene-3-carboxylic acid (61): With reference to the synthesis method for intermediate-2, 3,6-difluorosalicylaldehyde was used as the starting material to prepare the compound, with a two-step yield of 49.8%. HRMS (ESI⁻): calculated C₁₀H₅F₂O₃ (M-H)⁻, 211.0212; found 211.0219.

Synthesis of compound A-36: With reference to the synthesis method for compound A-1, 5,8-difluoro-2H-chromene-3-carboxylic acid was used as the starting material to prepare A-36 (a white solid, 0.07 g, yield: 46.9%, purity: 98.62%). ¹H NMR (300 MHz, DMSO-*d*₆) *δ* (ppm) 11.00 (s, 1H), 8.93 (t, *J =* 6.0 Hz, 1H), 7.70 (d, *J* = 7.8 Hz, 1H), 7.53 (s, 1H), 7.45 (d, *J* = 8.1 Hz, 1H), 7.32 (s, 1H), 7.20 (s, 1H), 7.04 (dd, *J* = 11.7, 6.9 Hz, 1H), 5.11 (dd, *J* = 13.3, 5.0 Hz, 1H), 4.98 (s, 2H), 4.55-4.24 (m, 4H), 2.99-2.80 (m, 1H), 2.60 (d, *J* = 17.2 Hz, 1H), 2.47-2.31 (m, 1H), 2.07-1.91 (m, 1H). HRMS (ESI⁺): calculated C₂₄H₂₀F₂N₃O₅ (M + H)⁺, 468.1366; found 468.1360.

### Example 37: Synthesis of N-((2-(2,6-Dioxopiperidin-3-yl)-1-oxoisoindolin-5-yl)methyl)-6,7-dimethyl-2H-chromene-3-carboxamide (Compound A-37)

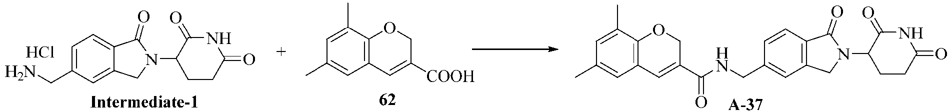

Synthesis of 6,8-dimethyl-2H-chromene-3-carboxylic acid (62): With reference to the synthesis method for intermediate-2, 3,5-dimethylsalicylaldehyde was used as the starting material to prepare the compound, with a two-step yield of 54.1%. HRMS (ESI⁻): calculated C₁₂H₁₁O₃ (M - H)⁻, 203.0714; found 203.0716.

Synthesis of compound A-37: With reference to the synthesis method for compound A-1, 6,8-dimethyl-2H-chromene-3-carboxylic acid was used as the starting material to prepare A-37 (a white solid, 0.06 g, yield: 40.8%, purity: 97.09%). ¹H NMR (300 MHz, DMSO-*d*₆) *δ* (ppm) 11.00 (s, 1H), 8.89 (t, *J =* 6.0 Hz, 1H), 7.70 (d, *J* = 7.8 Hz, 1H), 7.50 (s, 1H), 7.42 (d, *J =* 7.7 Hz, 1H), 7.30 (s, 1H), 7.02 (d, *J =* 8.1 Hz, 2H), 5.11 (dd, *J =* 13.2, 5.0 Hz, 1H), 4.91 (s, 2H), 4.56-4.23 (m, 4H), 2.99-2.83 (m, 1H), 2.60 (d, *J* = 17.5 Hz, 1H), 2.48-2.30 (m, 1H), 2.24 (s, 3H), 2.12 (s, 3H), 2.07-1.93 (m, 1H). HRMS (ESI⁺): calculated C₂₆H₂₆N₃O₅ (M + H)⁺, 460.1867; found 460.1860.

### Example 38: Synthesis of N-((2-(2,6-Dioxopiperidin-3-yl)-1-oxoisoindolin-5-yl)methyl)-5,8-dimethyl-2H-chromene-3-carboxamide (Compound A-38)

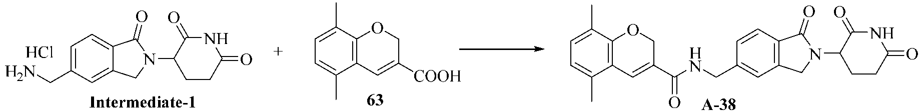

Synthesis of 5,8-dimethyl-2H-chromene-3-carboxylic acid (63): With reference to the synthesis method for intermediate-2, 3,6-dimethylsalicylaldehyde was used as the starting material to prepare the compound, with a two-step yield of 52.6%. HRMS (ESI⁻): calculated C₁₂H₁₁O₃ (M - H)⁻, 203.0714; found 203.0720.

Synthesis of compound A-38: With reference to the synthesis method for compound A-1, 5,8-dimethyl-2H-chromene-3-carboxylic acid was used as the starting material to prepare A-38 (a white solid, 0.07 g, yield: 47.6%, purity: 96.34%). ¹H NMR (300 MHz, DMSO-*d*₆) *δ* (ppm) 10.98 (s, 1H), 8.99 (t, *J* = 6.0 Hz, 1H), 7.75 (d, *J =* 7.6 Hz, 1H), 7.50 (s, 1H), 7.42 (d, *J* = 7.7 Hz, 1H), 7.30 (s, 1H), 7.00 (d, *J =* 8.5 Hz, 2H), 5.11 (dd, *J =* 13.2, 5.0 Hz, 1H), 4.91 (s, 2H), 4.55-4.24 (m, 4H), 2.99-2.83 (m, 1H), 2.60 (d, *J =* 17.6 Hz, 1H), 2.47 (s, 3H), 2.45-2.33 (m, 1H), 2.15 (s, 3H)2.05-1.93 (m, 1H). HRMS (ESI⁺): calculated C₂₆H₂₆N₃O₅ (M + H)⁺, 460.1867; found 460.1853.

### Example 39: Synthesis of N-((2-(2,6-Dioxopiperidin-3-yl)-3-oxoisoindolin-4-yl)methyl)-6-(trifluoromethoxy)-2H-chromene-3-carboxamide (Compound A-39)

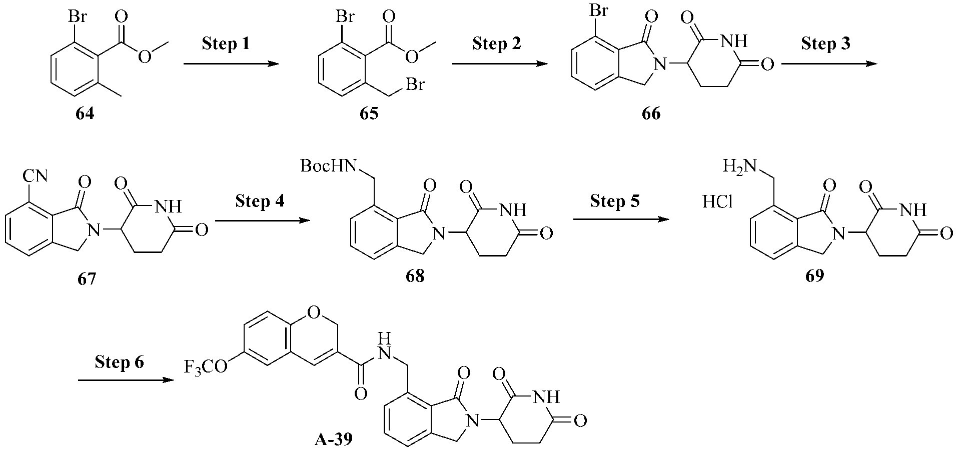

Steps 1-6: With reference to the synthesis methods for intermediate-1 and compound A-1, methyl 2-bromo-6-methylbenzoate (64) was used as the starting material to prepare A-39 (a white solid, 0.10 g, yield: 57.1%, purity: 98.16%). ¹H NMR (300 MHz, DMSO-*d*₆) *δ* (ppm) 11.01 (s, 1H), 8.96 (t, *J =* 6.0 Hz, 1H), 7.78-7.70 (m, 2H), 7.45 (d, *J =* 7.8 Hz, 1H), 7.33 (s, 1H), 6.96-6.79 (m, 3H), 5.12 (dd, *J =* 13.3, 5.1 Hz, 1H), 5.00 (s, 2H), 4.56-4.25 (m, 4H), 3.00-2.81 (m, 1H), 2.62 (d, *J =* 17.6 Hz, 1H), 2.48-2.30 (m, 1H), 2.07-1.94 (m, 1H). HRMS (ESI⁺): calculated C₂₄H₂₁F₃N₃O₆ (M + H)⁺, 516.1377; found 516.1368.

### Example 40: Synthesis of N-((2-(2,6-Dioxopiperidin-3-yl)-3-oxoisoindolin-5-yl)methyl)-6-(trifluoromethoxy)-2H-chromene-3-carboxamide (Compound A-40)

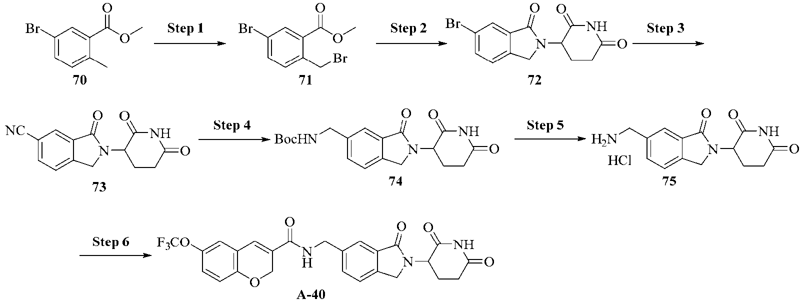

Steps 1-6: With reference to the synthesis methods for intermediate-1 and compound A-1, methyl 5-bromo-2-methylbenzoate (70) was used as the starting material to prepare A-40 (a white solid, 0.10 g, yield: 62.7%, purity: 99.20%). ¹H NMR (300 MHz, DMSO-*d*₆) *δ (ppm)* 11.01 (s, 1H), 8.96 (t, *J =* 6.0 Hz, 1H), 7.84 (s, 1H), 7.75 (d, *J* = 7.6 Hz, 1H), 7.50-7.38 (m, 2H), 6.90-6.75 (m, 3H), 5.12 (dd, *J* = 13.3, 5.1 Hz, 1H), 5.00 (s, 2H), 4.56-4.29 (m, 4H), 3.00-2.81 (m, 1H), 2.60 (d, *J* = 17.1 Hz, 1H), 2.49-2.35 (m, 1H), 2.07-1.93 (m, 1H). HRMS (ESI⁺): calculated C₂₄H₂₁F₃N₃O₆ (M + H)⁺, 516.1377; found, 516.1366.

### Example 41: Synthesis of N-((2-(2,6-Dioxopiperidin-3-yl)-1-oxoisoindolin-4-yl)methyl)-6-(trifluoromethoxy)-2H-chromene-3-carboxamide (Compound A-41)

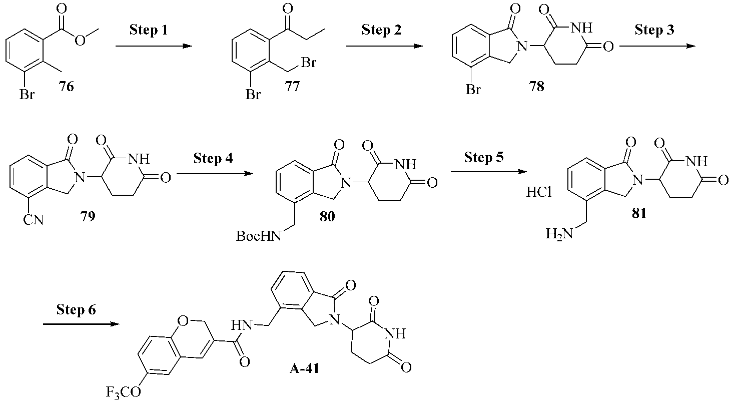

Steps 1-6: With reference to the synthesis methods for intermediate-1 and compound A-1, methyl 3-bromo-2-methylbenzoate (76) was used as the starting material to prepare A-41 (a white solid, 0.09 g, yield: 54.2%, purity: 97.59%). ¹H NMR (300 MHz, DMSO-*d*₆) *δ (ppm)* 10.99 (s, 1H), 8.99 (t, *J =* 6.0 Hz, 1H), 7.85 (s, 1H), 7.75 (d, *J* = 7.6 Hz, 1H), 7.55-7.39 (m, 2H), 6.92-6.74 (m, 3H), 5.12 (dd, *J* = 13.3, 5.1 Hz, 1H), 4.95 (s, 2H), 4.55-4.27 (m, 4H), 3.00-2.82 (m, 1H), 2.62 (d, *J =* 17.6 Hz, 1H), 2.49-2.34 (m, 1H), 2.07-1.94 (m, 1H). HRMS (ESI⁺): calculated C₂₄H₂₁F₃N₃O₆ (M + H)⁺, 516.1377; found, 516.1374.

### Example 42: Synthesis of N-((2-(2,6-Dioxopiperidin-3-yl)-4-fluoro-1-oxoisoindolin-5-yl)methyl)-6-(trifluoromethoxy)-2H-chromene-3-carboxamide (Compound A-42)

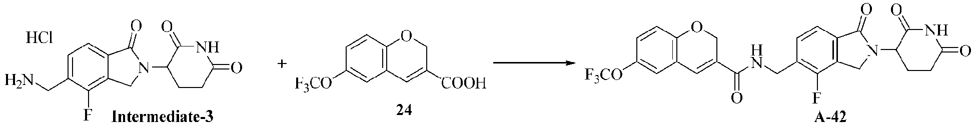

Synthesis of compound **A-42:** With reference to the synthesis method for compound **A-1, intermediate-3** and 6-trifluoromethoxy-2H-chromene-3-carboxylic acid **(24)** were used as the starting materials to prepare **A-42** (a white solid, 0.09 g, yield: 56.3%, purity: 97.98%). ¹H NMR (300 MHz, DMSO-*d*₆) *δ (ppm)* 11.02 (s, 1H), 8.93 (t*, J =* 5.4 Hz, 1H), 7.70-7.51 (m, 2H), 7.33 (d, *J* = 9.1 Hz, 2H), 7.25 (d, *J* = 8.7 Hz, 1H), 6.97 (d, *J =* 8.8 Hz, 1H), 5.14 (dd, *J =* 13.2, 4.8 Hz, 1H), 4.98 (s, 2H), 4.60-4.36 (m, 4H), 2.97-2.86 (m, 1H), 2.63 (d, *J* = 17.2 Hz, 1H), 2.46-2.38 (m, 1H), 2.03-1.99 (m, 1H) ppm. HRMS (ESI⁺): calculated C₂₅H₂₀F₄N₃O₆ (M + H)⁺, 534.1283; found 534.1291.

### Example 43: Synthesis of N-((2-(2,6-Dioxopiperidin-3-yl)-4-fluoro-1-oxoisoindolin-5-yl)methyl)-8-(trifluoromethoxy)-2H-chromene-3-carboxamide (Compound A-43)

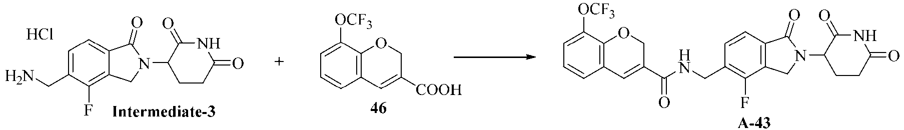

Synthesis of compound **A-43:** With reference to the synthesis method for compound **A-1, intermediate-3** and 8-trifluoromethoxy-2H-chromene-3-carboxylic acid **(46)** were used as the starting materials to prepare **A-43** (a white solid, 0.09 g, yield: 56.5%, purity: 95.82%). ¹H NMR (300 MHz, DMSO-*d*₆) *δ (ppm)* 11.03 (s, 1H), 8.97 (t, *J =* 6.0 Hz, 1H), 7.60-7.52 (m, 2H), 7.39 (s, 1H), 7.30 (t, *J* = 9.9 Hz, 2H), 7.03 (t, *J =* 7.9 Hz, 1H), 5.15 (dd, *J =* 13.0, 4.8 Hz, 1H), 5.02 (s, 2H), 4.60-4.36 (m, 4H), 2.97-2.86 (m, 1H), 2.63 (d, *J =* 17.7 Hz, 1H), 2.46-2.36 (m, 1H), 2.03-1.90 (m, 1H) ppm. HRMS (ESI⁺): calculated C₂₅H₂₀F₄N₃O₆ (M + H)⁺, 534.1283; found 534.1263.

### Example 44: Synthesis of N-((2-(2,6-Dioxopiperidin-3-yl)-1-oxoisoindolin-5-yl)methyl)chromane-3-carboxamide (Compound B-1)

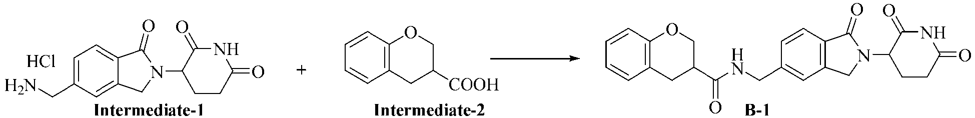

With reference to the synthesis method for compound A-1, chromane-3-carboxylic acid was used as the starting material to prepare B-1 (a white solid, 0.08 g, yield: 57.9%, purity: 98.03%). ¹H NMR (300 MHz, DMSO-*d*₆) *δ* (ppm) 11.03 (s, 1H), 8.78 (t, *J =* 6.0 Hz, 1H), 7.71 (d, *J* = 7.8 Hz, 1H), 7.48 (s, 1H), 7.43 (d, *J =* 7.9 Hz, 1H), 7.18-7.05 (m, 2H), 6.86 (t, *J =* 7.3 Hz, 1H), 6.79 (d, *J =* 8.1 Hz, 1H), 5.14 (dd, *J =* 13.3, 5.0 Hz, 1H), 4.50-4.28 (m, 5H), 3.99 (t, *J* = 9.7 Hz, 1H), 3.04-2.86 (m, 4H), 2.62 (d, *J =* 17.7 Hz, 1H), 2.47-2.33 (m, 1H), 2.09-1.97 (m, 1H). HRMS (ESI⁺): calculated C₂₄H₂₄N₃O₅ (M + H)⁺, 434.1711; found 434.1702.

### Example 45: Synthesis of N-((2-(2,6-Dioxopiperidin-3-yl)-1-oxoisoindolin-5-yl)methyl)-6-fluorochromane-3-carboxamide (Compound B-2)

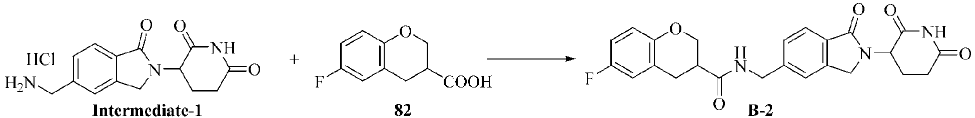

Synthesis of 6-fluorochromane-3-carboxylic acid (82): With reference to the synthesis method for intermediate-2, 5-fluorosalicylaldehyde was used as the starting material to prepare the compound, with a three-step yield of 51.2%. HRMS (ESI⁻): calculated C₁₀H₈FO₃ (M - H)⁻, 195.0463; found 195.0468.

Synthesis of compound B-2: With reference to the synthesis method for compound A-1, 6-fluorochromane-3-carboxylic acid was used as the starting material to prepare B-2 (a white solid, 0.06 g, yield: 41.6%, purity: 97.09%). ¹H NMR (300 MHz, DMSO-*d*₆) *δ (ppm)* 11.00 (s, 1H), 8.86 (t, *J* = 6.0 Hz, 1H), 7.75 (d, *J =* 7.8 Hz, 1H), 7.50 (s, 1H), 7.40 (d, *J =* 7.9 Hz, 1H), 7.15-7.03 (m, 2H), 6.79 (d, *J =* 8.1 Hz, 1H), 5.14 (dd, *J =* 13.3, 5.0 Hz, 1H), 4.48-4.25 (m, 5H), 3.99 (t, *J =* 9.7 Hz, 1H), 3.02-2.86 (m, 4H), 2.61 (d, *J* = 17.5 Hz, 1H), 2.48-2.33 (m, 1H), 2.07-1.95 (m, 1H). HRMS (ESI⁺): calculated C₂₄H₂₃FN₃O₅ (M + H)⁺, 452.1616; found 452.1613.

### Example 46: Synthesis of N-((2-(2,6-Dioxopiperidin-3-yl)-1-oxoisoindolin-5-yl)methyl)-6-chlorochromane-3-carboxamide (Compound B-3)

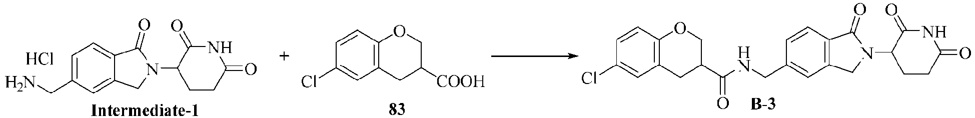

Synthesis of 6-chlorochromane-3-carboxylic acid (83): With reference to the synthesis method for intermediate-2, 5-chlorosalicylaldehyde was used as the starting material to prepare the compound, with a three-step yield of 49.6%. HRMS (ESI⁻): calculated C₁₀H₈ClO₃ (M - H)⁻, 211.0167; found 211.0159.

Synthesis of compound B-3: With reference to the synthesis method for compound A-1, 6-chlorochromane-3-carboxylic acid was used as the starting material to prepare B-3 (a white solid, 0.04 g, yield: 26.8%, purity: 98.73%). ¹H NMR (300 MHz, DMSO-*d*₆) *δ* (ppm) 11.00 (s, 1H), 8.87 (t, *J =* 6.0 Hz, 1H), 7.70 (d, *J =* 7.8 Hz, 1H), 7.55 (s, 1H), 7.40 (d, *J =* 7.9 Hz, 1H), 7.14-7.00 (m, 2H), 6.79 (d, *J* = 8.1 Hz, 1H), 5.10 (dd, *J =* 13.3, 5.0 Hz, 1H), 4.48-4.25 (m, 5H), 4.01 (t, *J =* 9.6 Hz, 1H), 3.02-2.85 (m, 4H), 2.60 (d, *J* = 17.0 Hz, 1H), 2.48-2.32 (m, 1H), 2.07-1.95 (m, 1H). HRMS (ESI⁺): calculated C₂₄H₂₃ClN₃O₅ (M + H)⁺, 468.1231; found 468.1225.

### Example 47: Synthesis of N-((2-(2,6-Dioxopiperidin-3-yl)-1-oxoisoindolin-5-yl)methyl)-6-(trifluoromethoxy)chromane-3-carboxamide (Compound B-4)

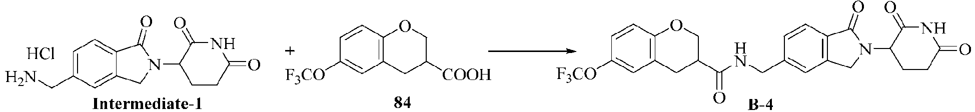

Synthesis of 6-trifluoromethoxychromane-3-carboxylic acid (84): With reference to the synthesis method for intermediate-2, 5-trifluoromethoxysalicylaldehyde was used as the starting material to prepare the compound, with a three-step yield of 50.8%. HRMS (ESI⁻): calculated C₁₁H₈F₃O₄ (M - H)⁻, 261.0380; found 261.0386.

Synthesis of compound B-4: With reference to the synthesis method for compound A-1, 6-trifluoromethoxychromane-3-carboxylic acid was used as the starting material to prepare B-4 (a white solid, 0.06 g, yield: 36.3%, purity: 99.50%). ¹H NMR (300 MHz, DMSO-*d*₆) *δ* (ppm) 11.00 (s, 1H), 8.77 (t, *J =* 5.9 Hz, 1H), 7.68 (d, *J =* 7.8 Hz, 1H), 7.47 (s, 1H), 7.40 (d, *J =* 7.9 Hz, 1H), 7.17 (s, 1H), 7.09 (d, *J =* 8.9 Hz, 1H), 6.87 (d, *J =* 8.9 Hz, 1H), 5.12 (dd, *J* = 13.3, 5.0 Hz, 1H), 4.48-4.26 (m, 5H), 4.04 (t, *J =* 9.7 Hz, 1H), 3.09-2.85 (m, 4H), 2.60 (d, *J =* 17.4 Hz, 1H), 2.48-2.32 (m, 1H), 2.07-1.93 (m, 1H). HRMS (ESI⁺): calculated C₂₅H₂₃F₃N₃O₆ (M + H)⁺, 518.1534; found 518.1540.

### Example 48: Synthesis of N-((2-(2,6-Dioxopiperidin-3-yl)-1-oxoisoindolin-5-yl)methyl)-1,2,3,4-tetrahydroquinoline-3-carboxamide (Compound C-1)

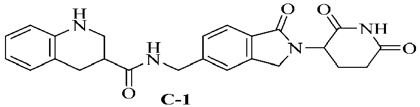

With reference to the synthesis method for compound A-1, 1,2,3,4-tetrahydroquinoline-3-carboxylic acid was used as the starting material to prepare C-1 (a white solid, 0.09 g, yield: 63.3%, purity: 98.67%). ¹H NMR (300 MHz, DMSO-*d*₆) *δ* (ppm) 11.01 (s, 1H), 8.64 (t, *J =* 5.9Hz, 1H), 7.68 (d, *J*=7.8Hz, 1H), 7.45 (s,1H), 7.39 (d, *J* =7.8 Hz, 1H), 6.87 (t, *J =* 8.1Hz, 2H), 6.49-6.38 (m,2H), 5.80 (s,1H), 5.12 (dd, *J =* 13.3, 5.0Hz,1H), 4.49-4.26 (m,4H), 3.20-2.71 (m,5H), 2.68-2.55 (m,2H), 2.48-2.32 (m,1H), 2.07-1.93 (m,1H). HRMS (ESI⁺): calculated C₂₄H₂₅N₄O₄ (M+H)⁺, 433.1871; found 433.1866.

### Example 49: Synthesis of N-((2-(2,6-Dioxopiperidin-3-yl)-1-oxoisoindolin-5-yl)methyl)-1,2,3,4-tetrahydronaphthalene-2-carboxamide (Compound D-1)

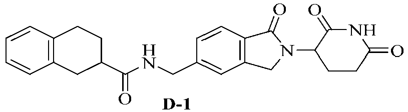

With reference to the synthesis method for compound A-1, 1,2,3,4-tetrahydronaphthalene-2-carboxylic acid was used as the starting material to prepare D-1 (a white solid, 0.12 g, 86.9%, purity: 97.13%). ¹H NMR (300 MHz, DMSO-*d*₆) *δ* (ppm) 10.99 (s, 1H), 8.54 (t, *J =* 6.0 Hz, 1H), 7.69 (d, *J =* 7.8 Hz, 1H), 7.48 (s, 1H), 7.41 (d, *J* = 7.2 Hz, 1H), 7.08 (s, 4H), 5.11 (dd, *J =* 13.3, 5.1 Hz, 1H), 4.52-4.24 (m, 4H), 2.96-2.83 (m, 3H), 2.83-2.72 (m, 2H), 2.66-2.54 (m, 2H), 2.47-2.32 (m, 1H), 2.07-1.96 (m, 2H), 1.82-1.64 (m, 1H). HRMS (ESI⁺): calculated C₂₅H₂₆N₃O₄ (M + H)⁺, 432.1918; found 432.1910.

### Biological Test Example 1: Study on Degradation of GSPT1 in KG-1 Cells

KG-1 cells (ATCC) were grown in an IMDM culture medium (containing 20% FBS). After centrifugation and counting, the cell concentration was adjusted. The cells were plated onto 6-well plates at 10⁶ cells/well, with 1350 µL per well. 150 µL of DMSO and the test compounds of the present disclosure (compound concentration: 200 nM) were added. The plates were incubated in an incubator at 37 °C with 5% CO₂ for 4 hours. The cells were centrifuged, and the culture medium was discarded. PBS was added to wash the cells and then discarded. Whole cell lysates were prepared using a protease inhibitor cocktail (100×, Fude Biological), a protein phosphatase inhibitor cocktail (100×, Fude Biological), SuperNuclease (Beyotime), and a high-intensity RIPA lysis buffer (Thermo Fisher) and placed on ice for 30 minutes. After centrifugation, the cell debris precipitates were discarded, and the whole cell lysate supernatants were collected and transferred to new centrifuge tubes. After a BCA protein assay, samples were prepared using a 5× loading buffer (Thermo Fisher). The samples were subjected to protein electrophoresis separation in a 4-20% precast gel (SDS-PAGE gel), transferred to a PVDF membrane, then blocked with 5% NFDM/TBST at room temperature for 1 hour, incubated overnight with primary antibodies at 4 °C, and incubated with a secondary antibody at room temperature for 2 hours the next day. Signals were detected using a MINICHEMITM imaging system.

Primary antibodies:
Anti-GSPT1: Abcam ab234433
Anti-beta actin: Fude Biological FD0060

Secondary antibody:
Anti-rabbit peroxidase-linked secondary antibody: Fude Biological FDG007

The degradation effects of the compounds on GSPT1 protein are shown in Table 1, where A represents a GSPT1 protein degradation percentage of no less than 80%, B represents a degradation percentage of less than 80% but no less than 50%, C represents a degradation percentage of less than 50% but no less than 25%, and D represents a degradation percentage of less than 25%.

**Table 1. The degradation of GSPT1 protein by compounds**

| Compound | GSPT1 degradation | Compound | GSPT1 degradation |
|---|---|---|---|
| A-1 | A | A-26 | D |
| A-2 | A | A-27 | D |
| A-3 | A | A-28 | A |
| A-4 | B | A-29 | A |
| A-5 | A | A-30 | A |
| A-6 | A | A-31 | A |
| A-7 | A | A-32 | A |
| A-8 | B | A-33 | A |
| A-9 | A | A-34 | A |
| A-10 | A | A-35 | A |
| A-11 | A | A-36 | A |
| A-12 | A | A-37 | A |
| A-13 | B | A-38 | A |
| A-14 | B | A-39 | B |
| A-15 | B | A-40 | B |
| A-16 | B | A-41 | B |
| A-17 | B | A-42 | A |
| A-18 | A | A-43 | A |
| A-19 | A | B-1 | A |
| A-20 | A | B-2 | A |
| A-21 | A | B-3 | A |
| A-22 | A | B-4 | A |
| A-23 | C | C-1 | A |
| A-24 | C | D-1 | D |
| A-25 | C | CC-90009 | B |

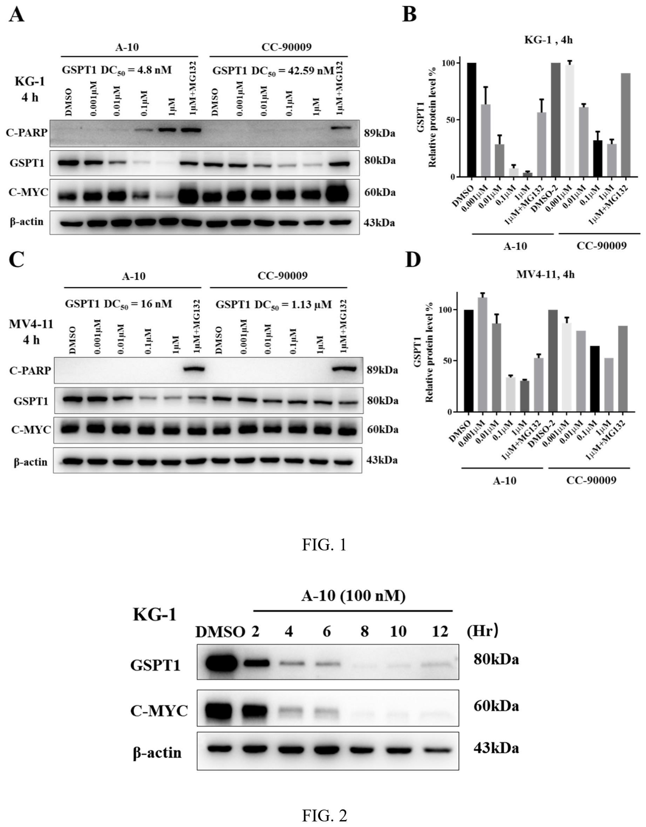

As can be seen from the table, compared to the clinical drug CC-90009, the benzo six-membered heterocyclic compounds of the present disclosure, such as series A, B, and C, exhibited significantly improved degradation effects on GSPT1 protein, while the benzo six-membered aliphatic rings lost the degradation effect on GSPT1 protein. In addition, as can be seen from FIGs. 1 and 2, the degradation of GSPT1 in KG-1 and MV4-11 cells by compound A-10 of the present disclosure was concentration-dependent and time-dependent, and its effect was more significant than that of CC-90009.

### Biological Test Example 2: Antiproliferative Activity Against KG-1 Cells

KG-1 cells in the logarithmic growth phase were diluted in a culture medium (RPMI + 10% FBS) and plated onto 96-well plates at 5000 cells/well, and the plates were incubated in an incubator at 37 °C with 5% CO₂ for 24 hours. 10 mM stock solutions of the test compounds of the present disclosure in DMSO were prepared and diluted with the culture medium to set 9 concentration gradients. Three replicate wells were set for each concentration. After compound addition, the plates were incubated in an incubator at 37 °C with 5% CO₂ for 72 hours. The cell proliferation was assessed using the CCK8 method. The cell growth inhibition IC₅₀ values of the compounds were determined using an enzyme labeling method. The IC₅₀ values were calculated using GraphPad Prism 7 software.

The antiproliferative activity of the compounds against KG-1 cells is shown in Table 2, where A represents IC₅₀ < 1 nM, B represents 1 ≤ IC₅₀ < 10 nM, C represents 10 nM ≤ IC₅₀ < 100 nM, D represents 100 nM ≤ IC₅₀ < 1000 nM, and E represents IC₅₀ ≥ 1000 nM.

**Table 2. The antiproliferative activity of compounds against KG-1 cells**

| Compound | IC₅₀ | Compound | IC₅₀ |
|---|---|---|---|
| A-1 | C | A-26 | D |
| A-2 | C | A-27 | D |
| A-3 | C | A-28 | C |
| A-4 | C | A-29 | C |
| A-5 | B | A-30 | C |
| A-6 | C | A-31 | B |
| A-7 | B | A-32 | B |
| A-8 | C | A-33 | B |
| A-9 | B | A-34 | B |
| A-10 | C | A-35 | C |
| A-11 | C | A-36 | B |
| A-12 | C | A-37 | C |
| A-13 | C | A-38 | C |
| A-14 | C | A-39 | C |
| A-15 | D | A-40 | C |
| A-16 | C | A-41 | C |
| A-17 | D | A-42 | B |
| A-18 | A | A-43 | B |
| A-19 | B | B-1 | C |
| A-20 | B | B-2 | B |
| A-21 | B | B-3 | B |
| A-22 | B | B-4 | A |
| A-23 | E | C-1 | B |
| A-24 | E | D-1 | E |
| A-25 | E | CC-90009 | C |

As can be seen from the table, compared to the clinical drug CC-90009, the benzo six-membered heterocyclic compounds of the present disclosure, such as series A, B, and C, exhibited significantly improved antiproliferative inhibitory activity against KG-1 cells, while the benzo six-membered aliphatic rings lost the antiproliferative inhibitory effect on KG-1 cells.

### Biological Test Example 3: Antiproliferative Activity of Compounds of Present Disclosure Against Various Tumor Cells

U937, MOLM-13, and MV4-11 cells (ATCC) in the logarithmic growth phase were separately diluted in a culture medium and plated onto 96-well plates at 5000 cells/well, and the plates were incubated in an incubator at 37 °C with 5% CO₂ for 24 hours. 10 mM stock solutions of the compounds in DMSO were prepared and diluted with the culture medium to set 9 concentration gradients. Three replicate wells were set for each concentration. After compound addition, the plates were incubated in an incubator at 37 °C with 5% CO₂ for 72 hours. The cell proliferation was assessed using the CCK8 method. The cell growth inhibition IC₅₀ values of the compounds were determined using an enzyme labeling method. The IC₅₀ values were calculated using GraphPad Prism 7 software.

The antiproliferative activity of the compounds against the tumor cells is shown in Table 3, where A represents IC₅₀ < 1 nM, B represents 1 ≤ IC₅₀ < 10 nM, C represents 10 nM ≤ IC₅₀ < 100 nM, D represents 100 nM ≤ IC₅₀ < 1000 nM, and E represents IC₅₀ ≥ 1000 nM.

**Table 3. The antiproliferative activity of compounds against tumor cells**

| Compound | U937 | MOLM-13 | MV4-11 |
|---|---|---|---|
| A-1 | B | C | C |
| A-3 | C | C | C |
| A-10 | B | B | C |
| B-1 | B | B | B |
| B-4 | B | B | A |
| C-1 | C | C | C |
| D-1 | E | E | E |
| CC-90009 | C | D | C |

As can be seen from the table, compared to the clinical drug CC-90009, the benzo six-membered heterocyclic compounds of the present disclosure, such as series A, B, and C, exhibited significantly improved antiproliferative inhibitory activity against various AML cells, while the benzo six-membered aliphatic rings lost the antiproliferative inhibitory effect on AML cells.

### Biological Test Example 4: Study on Metabolic Stability of Compounds of Present Disclosure in In Vitro Rat Liver Microsomes

10 mM stock solutions of the test compounds in DMSO were prepared, diluted with acetonitrile and then with PBS, and vortexed, with the DMSO content controlled at 0.1%. The dilute working solutions (10 µL) were incubated with 65 µL of a liver microsome system (30 µL of 100 mM PBS, 25 µL of 20 mM MgCl₂, and 10 µL of 5 mg/mL RLMs) and preheated at 37 °C for 5 min. 25 µL of 4 mM NADPH was added to start a reaction. Samples were taken at different time points (0 min, 5 min, 15 min, 30 min, 60 min, and 120 min), and an acetonitrile solution containing an internal standard was added to stop the reaction. After vortexing and centrifugation, the supernatants were collected and analyzed by LC-MS/MS, and the half-lives and clearance rates were calculated. RLMs were purchased from RILD Liver.

The results of the study on the metabolic stability of the compounds of the present disclosure in *in vitro* rat liver microsomes are shown in Table 4:

**Table 4. A study on the metabolic stability of compounds in in vitro rat liver microsomes**

| Compound | RLM T_{1/2}(min) | RLM CL(µL/min/mg) |
|---|---|---|
| A-1 | 369.94 | 3.75 |
| A-3 | 280.04 | 4.95 |
| A-10 | 166.05 | 8.35 |
| A-42 | 186.60 | 7.43 |
| B-1 | 44.11 | 31.42 |
| CC-90009 | 77.49 | 17.89 |

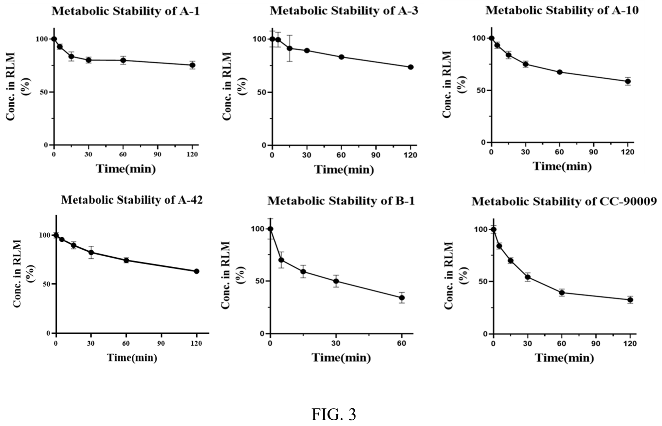

FIG. 3 shows the results of a study on the metabolic stability of compounds A-1, A-3, A-10, and A-42 of the present disclosure in *in vitro* rat liver microsomes.

As can be seen from the figure and table, compounds A-1, A-3, A-10, and A-42 exhibited half-lives that were more than 2 times that of the clinical drug CC-90009, indicating that the compounds had excellent metabolic stability in *in vitro* rat liver microsomes.

### Biological Test Example 5: In Vivo Pharmacokinetic Study of Compounds of Present Disclosure in Rats

Sample collection and preparation: After SPF SD rats (male, 210-230 g, 42-48 days, RILD Liver) in a good growth state were dosed intravenously at 2 mg/kg and orally at 10 mg/kg, blood samples were collected via the orbit at different blood collection time points and transferred to centrifuge tubes containing an anticoagulant; the intravenous blood collection time points were 0.08 hours, 0.25 hours, 0.5 hours, 1 hour, 2 hours, 4 hours, 6 hours, 8 hours, and 24 hours, and the oral blood collection time points were 0.25 hours, 0.5 hours, 1 hour, 2 hours, 4 hours, 6 hours, 8 hours, and 24 hours. The blood samples were immediately centrifuged at 4 °C and 8000 rpm for 10 minutes, and the plasma was collected and stored in an ultra-low-temperature freezer at -80 °C until it was analyzed by LC-MS/MS. Pharmacokinetic data analysis: Pharmacokinetic parameters of the compounds were calculated using pharmacokinetic software in a non-compartmental model. The peak plasma concentration (Cₘₐₓ) and the time to reach the peak plasma concentration (Tₘₐₓ) were taken as observation values. Parameters such as the half-life T_{1/2}, the areas under the plasma concentration-time curve AUCₗₐₛₜ and AUC_{inf}, the apparent volume of distribution Vₛₛ, and the clearance rate CL were determined through regression analysis of the linear terminal portion of the logarithmic plasma concentration-time curve and the linear trapezoidal rule.

The results of the *in vivo* pharmacokinetic study of the compounds of the present disclosure in rats are shown in Table 5:

**Table 5. An in vivo pharmacokinetic study of compounds in rats**

| Pharmacokinetic parameter | | A-1 | A-10 | A-42 | CC-90009 |
|---|---|---|---|---|---|
| IV 2 mg/kg | CL (mL/min/kg) | 17.6±2.6 | 8.1±2.5 | 1.9±0.1 | 28.79±6.40 |
| | T_{1/2} (h) | 1.38±0.14 | 1.53±0.29 | 1.37±0.04 | 1.44±0.26 |
| | C₀ (ng/mL) | 3840±226 | 16744±4330 | 32100±1550 | 1869±492 |
| | AUCₗₐₛₜ (h*ng/mL) | 1903±270 | 4313±1196 | 17200±1500 | 1196+296 |
| | AUC_{inf}(h*ng/mL) | 1916±267 | 4349±1200 | 17300±1470 | 1203±304 |
| | Vₛₛ (L/kg) | 0.92±0.07 | 0.42±0.13 | 0.14 | 2.85±0.63 |
| PO 10mg/kg | T_{1/2} (h) | 2.69±1.13 | 3.03±0.63 | 1.71 | 2.91±0.28 |
| | Cₘₐₓ (ng/mL) | 619±42 | 3460±1544 | 2810±1527 | 616±53 |
| | Tₘₐₓ (h) | 0.75±0.43 | 0.33±0.14 | 0.66±0.28 | 1.50±0.71 |
| | AUCₗₐₛₜ (h*ng/mL) | 1813±343 | 4952±2766 | 8380±1279 | 1892±12 |
| | AUC_{inf}(h*ng/mL) | 1847±330 | 4978±2742 | 7190 | 1896±14 |
| | F (%) | 19.3±3.4 | 22.9±12.6 | 9.8±1.5 | 31.2±9.1 |

FIG. 4 shows the results of an *in vivo* pharmacokinetic study of compounds A-1, A-10, and A-42 and the clinical drug CC-90009 in rats.

As can be seen from the figure and table, compound A-1, when administered by intravenous injection, exhibited an *in vivo* clearance rate less than that of the clinical drug CC-90009, a half-life close to that of CC-90009, and a relatively good metabolic stability *in vivo,* and its initial plasma concentration and plasma drug exposure level were greatly superior to those of CC-90009.

Compound A-10, when administered by intravenous injection, exhibited a clearance rate that was one third of that of CC-90009, a half-life longer than that of CC-90009, an initial plasma concentration much higher than that of CC-90009, and a plasma drug exposure level that was nearly four times that of CC-90009. Moreover, the compound, when administered orally, exhibited an exposure level that was nearly three times that of CC-90009, a relatively high peak, and a relatively small time to peak, suggesting that compound A-10 was absorbed faster.

Compound A-42 exhibited a lower clearance rate, and higher intravenous and oral plasma drug exposure levels that were fourteen times and four times those of CC-90009, respectively.

### Biological Test Example 6: In Vivo Pharmacodynamic Study of Compounds of Present Disclosure in Acute Myeloid Leukemia Xenograft Mouse Model

The human leukemia cells MV411 (Cell Bank, Chinese Academy of Sciences) used in this experiment were cultured in an IMDM culture medium supplemented with 10% FBS in an incubator containing CO₂ at 37 °C. Before the cells were continuously sub-cultured to the tenth generation, MV411 cells in the logarithmic growth phase were used, and the cell concentration was adjusted to 1 × 10⁸ cells/mL with a serum-free culture medium. Each BABL/c Nude mouse (Jiangsu GemPharmatech Co., Ltd., female, 4-6 weeks old, 14-16 g) was inoculated with 100 µL of the cell suspension by subcutaneous injection on the right dorsal side (near the axilla) under sterile conditions. When the mean tumor volume of the tumor-bearing mice reached approximately 100 mm³, the mice were randomly divided into groups, with the tumor volume in each group being roughly the same. The day of grouping and administration was defined as day 0. The animals in each treatment group were given a compound once daily by oral gavage at 15 mg/kg, 30 mg/kg, or 60 mg/kg. The body weight and tumor volume of the animals were measured every other day.

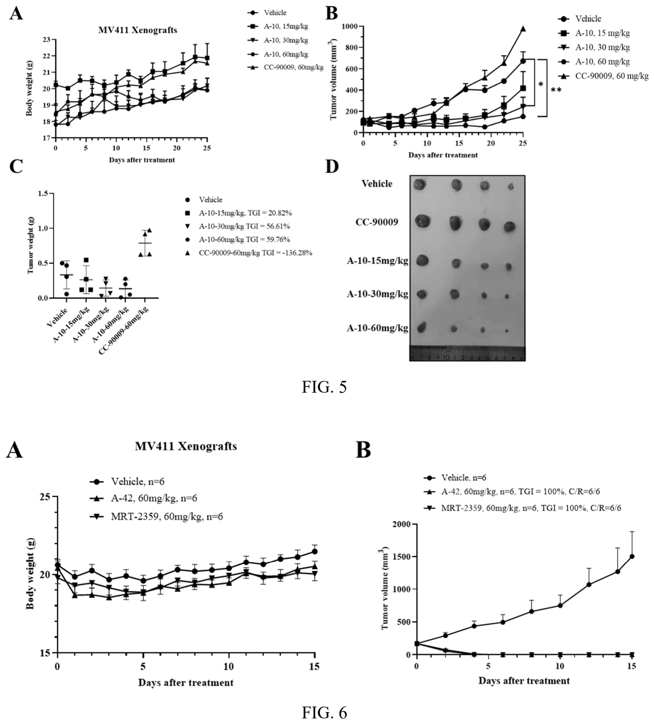

Experimental indicator: The anti-tumor efficacy of the compounds was evaluated using the relative tumor growth inhibition rate (TGI). Tumor weight TGI (%) = {1 - [(final average tumor weight of experimental group)/(final average tumor weight of control group of tumor-bearing mice without administration)]} × 100%. Tumor volume TGI (%) = [1 - relative tumor volume of experimental group/relative tumor volume of control group of tumor-bearing mice without administration] × 100%. Results: FIG. 5 shows an *in vivo* pharmacodynamic study of compound A-10 in an MV411 xenograft mouse model. In the case of no significant weight loss in mice, when compound A-10 was administered at doses of 15 mg/kg, 30 mg/kg, and 60 mg/kg, the tumor weight TGI reached 20.82%, 56.61%, and 59.76%, respectively; these results were significantly better than that of the clinical molecule CC-90009. FIG. 6 shows an *in vivo* pharmacodynamic study of compound A-42 in an MV411 xenograft mouse model. In the case of no significant weight loss in mice, when compound A-42 was administered at a dose of 60 mg/kg, the tumor volume TGI reached 100%; this result was comparable to that of the clinical molecule MRT-2359.

### Biological Test Example 7: In Vivo Pharmacodynamic Study of Compounds of Present Disclosure in Prostate Cancer Xenograft Mouse Model

The 22RV1 cells (Cell Bank, Chinese Academy of Sciences) used in this experiment were cultured in a 1640 culture medium supplemented with 10% FBS in an incubator containing CO₂ at 37 °C. Before the cells were continuously sub-cultured to the tenth generation, 22RV1 cells in the logarithmic growth phase were used, and the cell concentration was adjusted to 5 × 10⁷ cells/mL with a serum-free culture medium. Each BABL/c Nude mouse was inoculated with 100 µL of the cell suspension by subcutaneous injection on the right dorsal side (near the axilla) under sterile conditions. When the mean tumor volume of the tumor-bearing mice reached approximately 50-100 mm³, the mice were randomly divided into 4 groups of 8, with the tumor volume in each group being roughly the same. The day of grouping and administration was defined as day 0. The animals in each treatment group were given a compound once daily by oral gavage at 60 mg/kg. The body weight and tumor volume of the animals were measured every other day.

Experimental indicator: The anti-tumor efficacy of the compounds was evaluated using the relative tumor growth inhibition rate (TGI). Tumor weight TGI (%) = {1 - [(final average tumor weight of experimental group)/(final average tumor weight of control group of tumor-bearing mice without administration)]} × 100%.

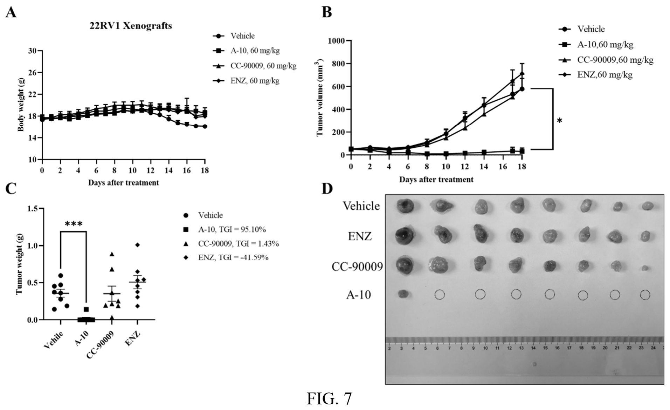

Results: FIG. 7 shows an *in vivo* pharmacodynamic study of compound A-10 in an 22RV1 xenograft mouse model. When compound A-10 was administered at a dose of 60 mg/kg, the TGI reached 95.10%; this result was significantly better than those of the clinical molecule CC-90009 and the commercially available drug enzalutamide.

As described above, while the present disclosure has been shown and described with reference to particular preferred examples, they are not to be construed as limitations on the present disclosure per se. Various changes can be made in its form and details without departing from the spirit and scope of the present disclosure defined by the appended claims.

## Claims

1. A compound represented by general formula I or a pharmaceutically acceptable salt, tautomer, mesomer, racemate, enantiomer, or diastereomer thereof: 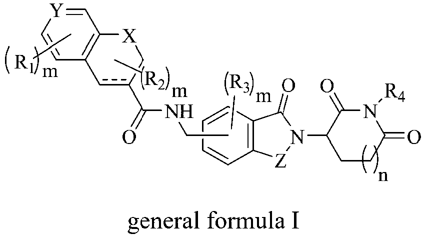 wherein:
- - - - represents a single or double bond;
X is selected from -CH₂-, -NH-, -O-, -S-, and -Se-;
Y is selected from carbon and nitrogen;
Z is selected from -CH₂-, -CD₂-, -C(O)-, and -C(S)-;
each R₁ is independently selected from hydrogen, C₁-C₆ alkyl, halogenated C₁-C₆ alkyl, C₂-C₆ alkenyl, C₂-C₆ alkynyl, halogen, cyano, nitro, -C(O)NRₐR_{b}, -C(O)Rₐ, -C(O)ORₐ, -ORₐ, -OC(O)Rₐ, -OC(O)ORₐ, -OC(O)NRₐR_{b}, -NRₐR_{b}, -SRₐ, -S(O)Rₐ, -S(O)₂Rₐ, and 3- to 10-membered cycloalkyl, heterocyclyl, aryl, and heteroaryl that contain 0-3 heteroatoms, wherein the alkyl, alkenyl, alkynyl, cycloalkyl, heterocyclyl, aryl, and heteroaryl are unsubstituted or optionally substituted with 1-3 Rₐ groups;
each R₂ is independently selected from hydrogen, C₁-C₆ alkyl, halogenated C₁-C₆ alkyl, C₂-C₆ alkenyl, C₂-C₆ alkynyl, halogen, cyano, nitro, -C(O)NRₐR_{b}, -C(O)Rₐ, -C(O)ORₐ, -ORₐ, -OC(O)Rₐ, -OC(O)ORₐ, -OC(O)NRₐR_{b}, -NRₐR_{b}, -SRₐ, -S(O)Rₐ, -S(O)₂Rₐ, and 3- to 10-membered cycloalkyl, heterocyclyl, aryl, and heteroaryl that contain 0-3 heteroatoms, wherein the alkyl, alkenyl, alkynyl, cycloalkyl, heterocyclyl, aryl, and heteroaryl are unsubstituted or optionally substituted with 1-3 Rₐ groups;
each R₃ is independently selected from hydrogen, C₁-C₆ alkyl, halogenated C₁-C₆ alkyl, C₂-C₆ alkenyl, C₂-C₆ alkynyl, halogen, cyano, nitro, -C(O)NRₐR_{b}, -C(O)Rₐ, -C(O)ORₐ, -ORₐ, -OC(O)Rₐ, -OC(O)ORₐ, -OC(O)NRₐR_{b}, -NRₐR_{b}, -SRₐ, -S(O)Rₐ, -S(O)₂Rₐ, and 3- to 10-membered cycloalkyl, heterocyclyl, aryl, and heteroaryl that contain 0-3 heteroatoms, wherein the alkyl, alkenyl, alkynyl, cycloalkyl, heterocyclyl, aryl, and heteroaryl are unsubstituted or optionally substituted with 1-3 Rₐ groups;
R₄ is selected from hydrogen, deuterium, C₁-C₁₂ alkyl, 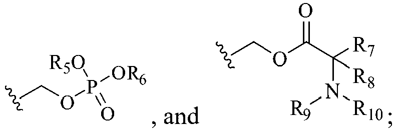
R₅, R₆, R₇, R₈, R₉, and R₁₀ are each independently selected from hydrogen, C₁-C₆ alkyl, halogenated C₁-C₆ alkyl, C₂-C₆ alkenyl, C₂-C₆ alkynyl, halogen, cyano, nitro, -C(O)NRₐR_{b}, -C(O)Rₐ, -C(O)ORₐ, - ORₐ, -OC(O)Rₐ, -OC(O)ORₐ, -OC(O)NRₐR_{b}, -NRₐR_{b}, -SRₐ, -S(O)Rₐ, -S(O)₂Rₐ, and 3- to 10-membered cycloalkyl, heterocyclyl, aryl, and heteroaryl that contain 0-3 heteroatoms, wherein the alkyl, alkenyl, alkynyl, cycloalkyl, heterocyclyl, aryl, and heteroaryl are unsubstituted or optionally substituted with 1-3 Rₐ groups;
Rₐ and R_{b} are each independently selected from hydrogen, C₁-C₆ alkyl, C₂-C₆ alkenyl, C₂-C₆ alkynyl, halogen, hydroxyl, cyano, nitro, benzyl, -C(O)NR_{c}R_{d}, -C(O)R_{c}, -C(O)OR_{c}, -OR_{c}, -OC(O)R_{c}, - OC(O)OR_{c}, -OC(O)NR_{c}R_{d}, -NR_{c}R_{d}, -SR_{c}, -S(O)R_{c}, -S(O)₂R_{c}, and 3- to 10-membered cycloalkyl, heterocyclyl, aryl, and heteroaryl that contain 0-3 heteroatoms, wherein the alkyl, alkenyl, alkynyl, cycloalkyl, heterocyclyl, aryl, heteroaryl, or benzyl is unsubstituted or optionally substituted with 1-3 R_{c} groups;
R_{c} and R_{d} are each independently selected from hydrogen, halogen, carbonyl, hydroxyl, cyano, nitro, phenyl, benzyl, C₁-C₆ alkyl, halogenated C₁-C₆ alkyl, C₁-C₆ alkoxy, halogenated C₁-C₆ alkoxy, C₃-C₆ cycloalkyl, and halogenated C₃-C₆ cycloalkyl;
m and n are each independently selected from 0, 1, 2, and 3.

2. A compound represented by general formula II or a pharmaceutically acceptable salt, tautomer, mesomer, racemate, enantiomer, or diastereomer thereof: 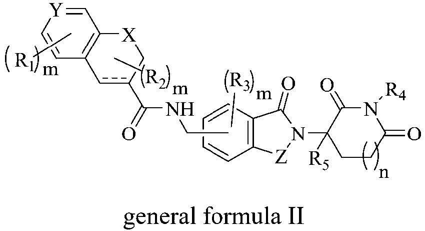 wherein:
- - - - represents a single or double bond;
X is selected from -CH₂-, -NH-, -O-, -S-, and -Se-;
Y is selected from carbon and nitrogen;
Z is selected from -CH₂-, -CD₂-, -C(O)-, and -C(S)-;
each R₁ is independently selected from hydrogen, C₁-C₆ alkyl, halogenated C₁-C₆ alkyl, C₂-C₆ alkenyl, C₂-C₆ alkynyl, halogen, cyano, nitro, -C(O)NRₐR_{b}, -C(O)Rₐ, -C(O)ORₐ, -ORₐ, -OC(O)Rₐ, -OC(O)ORₐ, -OC(O)NRₐR_{b}, -NRₐR_{b}, -SRₐ, -S(O)Rₐ, -S(O)₂Rₐ, and 3- to 10-membered cycloalkyl, heterocyclyl, aryl, and heteroaryl that contain 0-3 heteroatoms, wherein the alkyl, alkenyl, alkynyl, cycloalkyl, heterocyclyl, aryl, and heteroaryl are unsubstituted or optionally substituted with 1-3 Rₐ groups;
each R₂ is independently selected from hydrogen, C₁-C₆ alkyl, halogenated C₁-C₆ alkyl, C₂-C₆ alkenyl,
C₂-C₆ alkynyl, halogen, cyano, nitro, -C(O)NRₐR_{b}, -C(O)Rₐ, -C(O)ORₐ, -ORₐ, -OC(O)Rₐ, -OC(O)ORₐ, -OC(O)NRₐR_{b}, -NRₐR_{b}, -SRₐ, -S(O)Rₐ, -S(O)₂Rₐ, and 3- to 10-membered cycloalkyl, heterocyclyl, aryl, and heteroaryl that contain 0-3 heteroatoms, wherein the alkyl, alkenyl, alkynyl, cycloalkyl, heterocyclyl, aryl, and heteroaryl are unsubstituted or optionally substituted with 1-3 Rₐ groups;
each R₃ is independently selected from hydrogen, C₁-C₆ alkyl, halogenated C₁-C₆ alkyl, C₂-C₆ alkenyl, C₂-C₆ alkynyl, halogen, cyano, nitro, -C(O)NRₐR_{b}, -C(O)Rₐ, -C(O)ORₐ, -ORₐ, -OC(O)Rₐ, -OC(O)ORₐ, -OC(O)NRₐR_{b}, -NRₐR_{b}, -SRₐ, -S(O)Rₐ, -S(O)₂Rₐ, and 3- to 10-membered cycloalkyl, heterocyclyl, aryl, and heteroaryl that contain 0-3 heteroatoms, wherein the alkyl, alkenyl, alkynyl, cycloalkyl, heterocyclyl, aryl, and heteroaryl are unsubstituted or optionally substituted with 1-3 Rₐ groups;
R₄ is selected from hydrogen, deuterium, C₁-C₁₂ alkyl, 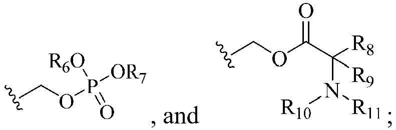
R₅ is selected from hydrogen, deuterium, and C₁-C₁₂ alkyl;
R₆, R₇, R₈, R₉, R₁₀, and R₁₁ are each independently selected from hydrogen, C₁-C₆ alkyl, halogenated C₁-C₆ alkyl, C₂-C₆ alkenyl, C₂-C₆ alkynyl, halogen, cyano, nitro, -C(O)NRₐR_{b}, -C(O)Rₐ, -C(O)ORₐ, - ORₐ, -OC(O)Rₐ, -OC(O)ORₐ, -OC(O)NRₐR_{b}, -NRₐR_{b}, -SRₐ, -S(O)Rₐ, -S(O)₂Rₐ, and 3- to 10-membered cycloalkyl, heterocyclyl, aryl, and heteroaryl that contain 0-3 heteroatoms, wherein the alkyl, alkenyl, alkynyl, cycloalkyl, heterocyclyl, aryl, and heteroaryl are unsubstituted or optionally substituted with 1-3 Rₐ groups;
Rₐ and R_{b} are each independently selected from hydrogen, C₁-C₆ alkyl, C₂-C₆ alkenyl, C₂-C₆ alkynyl, halogen, hydroxyl, cyano, nitro, benzyl, -C(O)NR_{c}R_{d}, -C(O)R_{c}, -C(O)OR_{c}, -OR_{c}, -OC(O)R_{c}, - OC(O)OR_{c}, -OC(O)NR_{c}R_{d}, -NR_{c}R_{d}, -SR_{c}, -S(O)R_{c}, -S(O)₂R_{c}, and 3- to 10-membered cycloalkyl, heterocyclyl, aryl, and heteroaryl that contain 0-3 heteroatoms, wherein the alkyl, alkenyl, alkynyl, cycloalkyl, heterocyclyl, aryl, heteroaryl, or benzyl is unsubstituted or optionally substituted with 1-3 R_{c} groups;
R_{c} and R_{d} are each independently selected from hydrogen, halogen, carbonyl, hydroxyl, cyano, nitro, phenyl, benzyl, C₁-C₆ alkyl, halogenated C₁-C₆ alkyl, C₁-C₆ alkoxy, halogenated C₁-C₆ alkoxy, C₃-C₆ cycloalkyl, and halogenated C₃-C₆ cycloalkyl;
m and n are each independently selected from 0, 1, 2, and 3.

3. The compound according to claim 1, wherein the compound has a structure of formula Ia, formula Ib, or formula Ic: 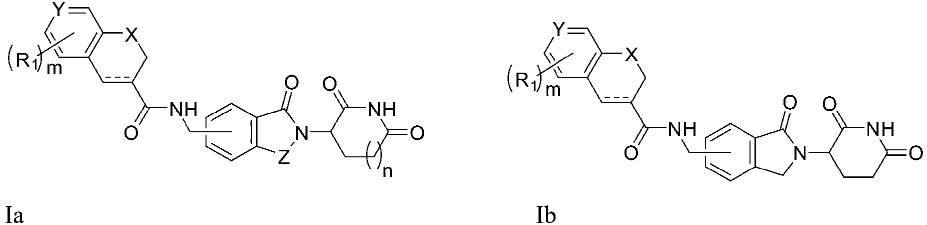 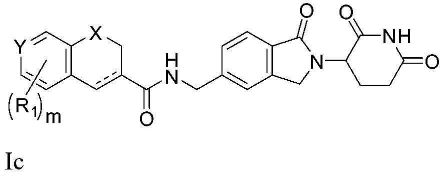 wherein R₁, X, Y, Z, m, n, and - - - - are as defined in general formula I.

4. The compound according to claim 1 or 2, wherein:
X is selected from -NH- and -O-.

5. The compound according to claim 1 or 2, wherein:
each R₁ is independently selected from hydrogen, C₁-C₆ alkyl, halogenated C₁-C₆ alkyl, C₁-C₆ alkoxy, halogenated C₁-C₆ alkoxy, C₂-C₆ alkenyl, C₂-C₆ alkynyl, halogen, cyano, hydroxyl, nitro, C₁-C₁₀ aryl, and -NRₐR_{b};
Rₐ and R_{b} are each independently selected from hydrogen, C₁-C₆ alkyl, and -C(O)R_{c};
R_{c} is selected from hydrogen, halogen, C₁-C₆ alkyl, halogenated C₁-C₆ alkyl, C₁-C₆ alkoxy, halogenated C₁-C₆ alkoxy, and C₃-C₆ cycloalkyl.

6. The compound according to claim 4, wherein:
each R₁ is independently selected from hydrogen, methyl, fluorine, chlorine, methoxy, trifluoromethoxy, nitro, cyano, amino, hydroxyl, ethenyl, ethynyl, isobutyl, phenyl,

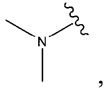

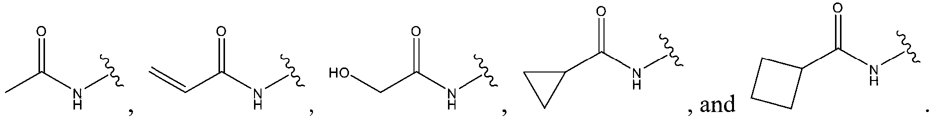

7. The compound according to claim 1, wherein the compound is selected from: 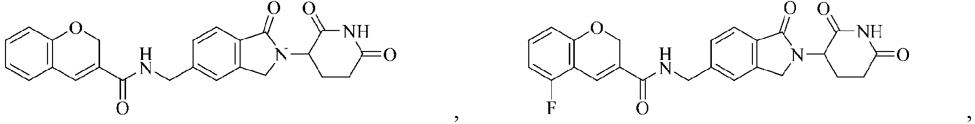 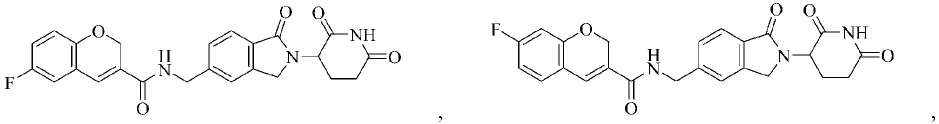 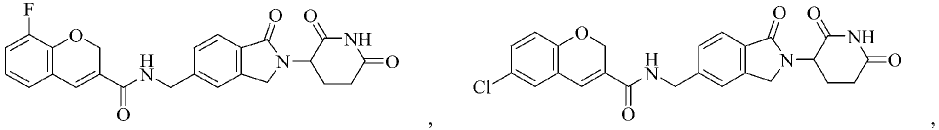 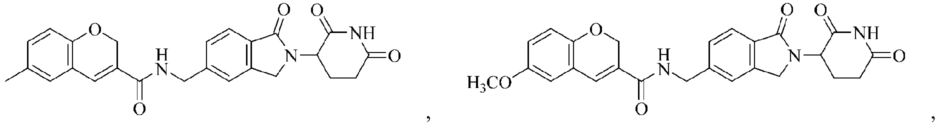 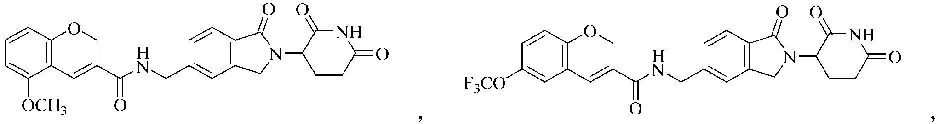 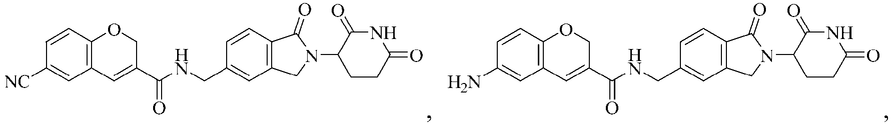 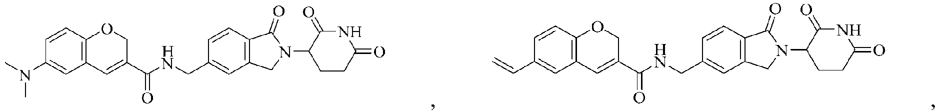 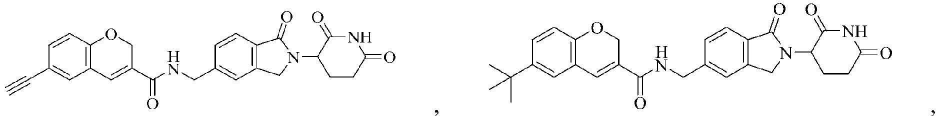 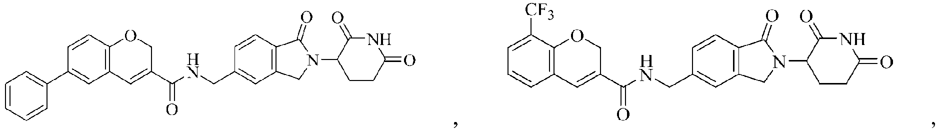 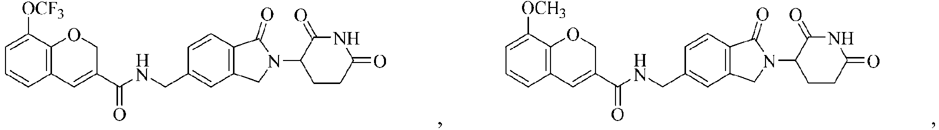 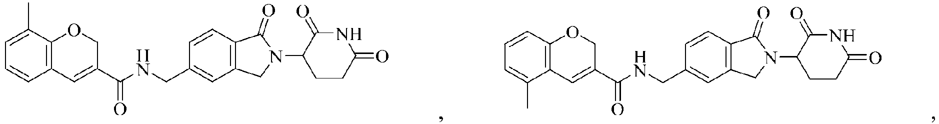 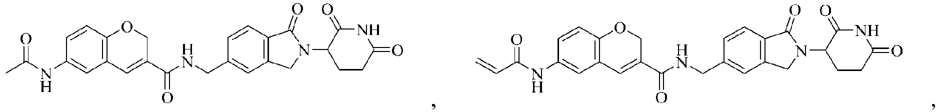 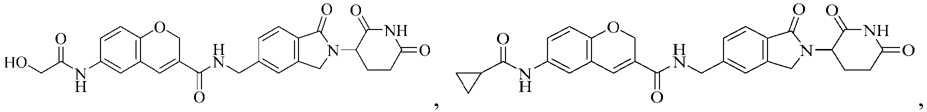 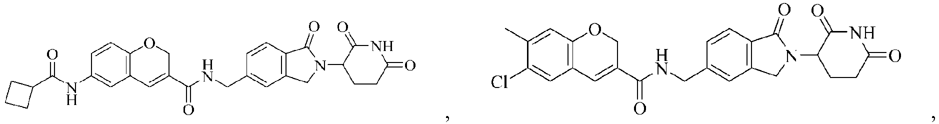 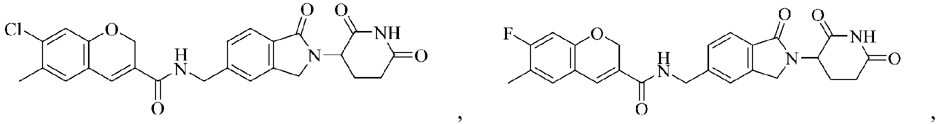 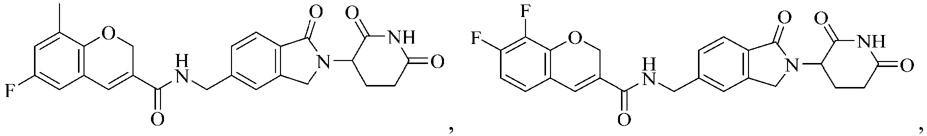 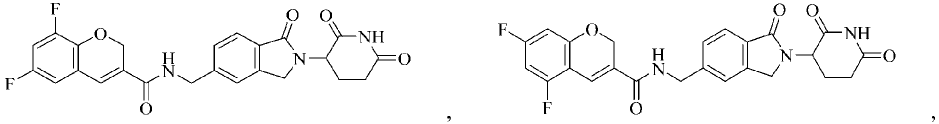 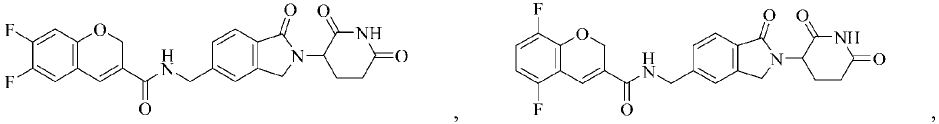 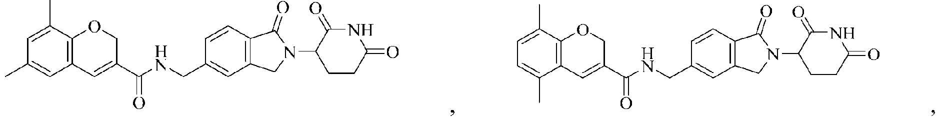 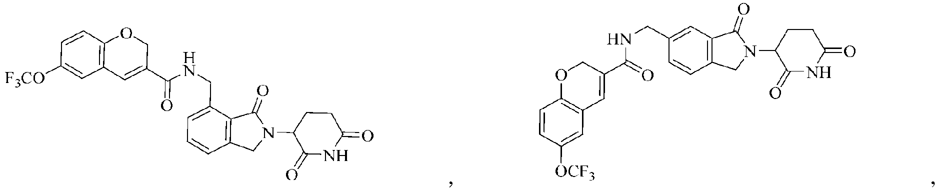 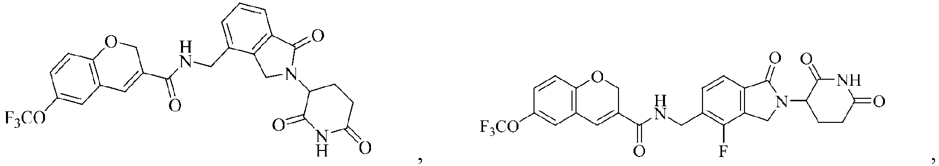 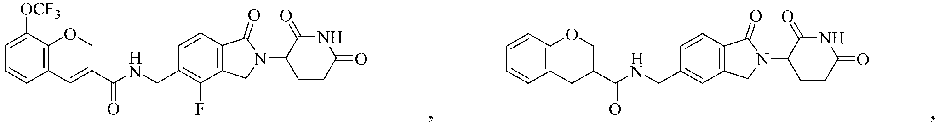 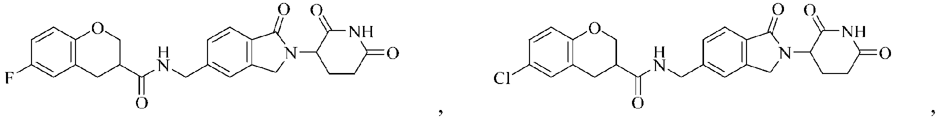 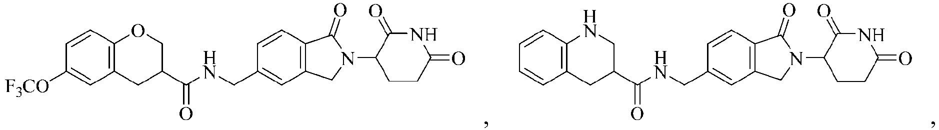 and

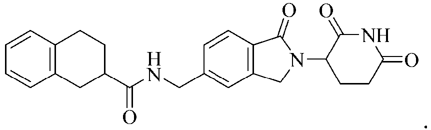

8. A pharmaceutical composition, comprising a therapeutically effective amount of the compound or the pharmaceutically acceptable salt, tautomer, mesomer, racemate, enantiomer, or diastereomer thereof according to any one of claims 1-7 and a pharmaceutically acceptable carrier or excipient.

9. Use of the compound according to any one of claims 1-7 in the preparation of a medicament for treating or preventing a disease related to mutation, unbalanced expression, allosterism, and dysfunction of GSPT1, IKZF1, IKZF2, IKZF3, CK1α, N-MYC, or C-MYC protein.

10. The use according to claim 9, wherein the related disease is cancer, aging, an immune disease, or a neurological disease, wherein the cancer is selected from acute myeloid leukemia, liver cancer, acute lymphocytic leukemia, bladder cancer, bone cancer, brain cancer, breast cancer, cervical cancer, choriocarcinoma, chronic lymphocytic leukemia (CLL), chronic granulocytic leukemia (CML), colon cancer, esophageal cancer, gallbladder cancer, gastric cancer, gastrointestinal stromal tumor, head and neck cancer, Hodgkin lymphoma, laryngeal cancer, leukemia, lung cancer, melanoma, mesothelioma, multiple myeloma, ovarian cancer, pancreatic cancer, prostate cancer, rectal cancer, renal cancer, sarcoma, skin cancer, small cell lung cancer, testicular cancer, throat cancer, thyroid cancer, and uterine cancer.

11. Use of the compound according to any one of claims 1-7 in the preparation of a GSPT1 degrader.
